# EUROPÄISCHE PATENTSCHRIFT

(11) **EP 1 244 641 B1**
(45) Veröffentlichungstag und Bekanntmachung des Hinweises auf die Patenterteilung: **13.04.2005**
(21) Anmeldenummer: 00991169.4
(22) Anmeldetag: 12.12.2000
(51) Int. Cl.: C07D 277/54, C07D 417/12, C07F 9/6539, C07F 7/18, A61K 31/426, A61P 31/22

(54) **THIAZOLYLAMID-DERIVATE**
THIAZOLYL AMIDE DERIVATIVES
DERIVES DU TYPE THIAZOLYLAMIDE

(30) Priorität: 23.12.1999 DE 19962532; 11.08.2000 DE 10039265
(43) Veröffentlichungstag der Anmeldung: 02.10.2002
(73) Patentinhaber: Bayer HealthCare AG, 51368 Leverkusen (DE)
(72) Erfinder: FISCHER, Rüdiger, 50259 Pulheim (DE); KLEYMANN, Gerald, 32107 Bad Salzuflen (DE); BETZ, Ulrich, 42119 Wuppertal (DE); BAUMEISTER, Judith, 42277 Wuppertal (DE); BENDER, Wolfgang, 42113 Wuppertal (DE); ECKENBERG, Peter, 42115 Wuppertal (DE); HANDKE, Gabriele, 42489 Wülfrath (DE); HENDRIX, Martin, 51519 Odenthal (DE); HENNINGER, Kerstin, 42115 Wuppertal (DE); JENSEN, Axel, 42553 Velbert (DE); KELDENICH, Jörg, 42113 Wuppertal (DE); SCHNEIDER, Udo, 51373 Leverkusen (DE); WEBER, Olaf, West Haven, CT 06516-4175 (US)
(86) Internationale Anmeldenummer: PCT/EP2000/012564
(87) Internationale Veröffentlichungsnummer: WO 2001/047904

(56) Entgegenhaltungen:
- WO-A-00/53591
- WO-A-97/24343
- WO-A-99/37291
- GB-A- 1 323 045
- ZIEGLER C ET AL: "2-AMINOTHIAZOLESULFONAMIDES" JOURNAL OF ORGANIC CHEMISTRY,AMERICAN CHEMICAL SOCIETY. EASTON,US, Bd. 25, August 1960 (1960-08), Seiten 1454-1455, XP000942449 ISSN: 0022-3263 in der Anmeldung erwähnt

## Beschreibung

Die vorliegende Erfindung betrifft neue Verbindungen, nämlich Thiazolylamid-Derivate, Verfahren zu ihrer Herstellung sowie ihre Verwendung als Arzneimittel, insbesondere als antivirale Arzneimittel.

Aus der Publikation C. Ziegler et al., J. Org. Chem. 25, 1960, 1454-1455 sind 2-Aminothiazol-5-sulfonamide bekannt. Außerdem werden in der deutschen Offenlegungsschrift 2101640 N-Thiazol-2-yl-amide und -harnstoffe mit einer herbiziden Wirkung beschrieben.

Die WO97/24343 betrifft Phenylthiazolderivate mit Anti-Herpes Virus-Eigenschaften.

Die WO99/42455 betrifft ebenfalls Phenylthiazolderivate mit Anti-Herpes Virus-Eigenschaften.

Die WO99/47507 betrifft 1,3,4-Thiadiazolderivate mit Anti-Herpes Virus-Eigenschaften.

Die vorliegende Erfindung betrifft neue Verbindungen, bei denen es sich Thiazolylamid-Derivate der allgemeinen Formel (I) handelt: 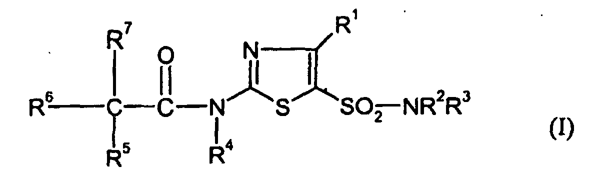 in welcher
- R¹: für Wasserstoff, Halogen, (C₁-C₆)-Alkyl, (C₁-C₆)-Alkoxy, Amino-(C₁-C₆)-alkyl oder Halogen-(C₁-C₆)-alkyl steht,
- R² und R³: gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Tri-(C₁-C₆)-alkylsilyloxy, Resten der Formel 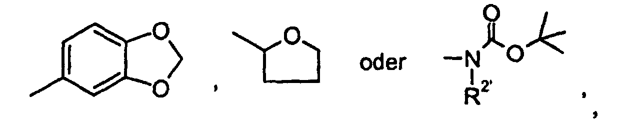 worin R^{2'} für Wasserstoff oder (C₁-C₄)-Alkyl steht,
einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8-gliedrigen gesättigten oder ungesättigten, nicht aromatischen, Heterocyclus mit bis zu 3 Heteroatomen aus der Reihe S, N und/oder O, und (C₆-C₁₀)-Aryl, das seinerseits durch Hydroxy oder (C₁-C₆)-Alkoxy substituiert sein kann, besteht, oder
für eine Gruppe der Formel 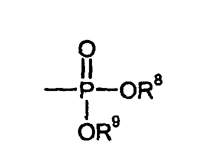 stehen, worin R⁸ und R⁹ gleich oder verschieden voneinander sind und für Wasserstoff und (C₁-C₄)-Alkyl stehen, oder
für eine Gruppe der Formel 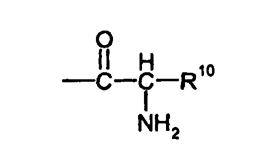 steht, worin R¹⁰ die Seitengruppe einer natürlich vorkommenden α-Aminosäure darstellt, oder
für eine Gruppe der Formel 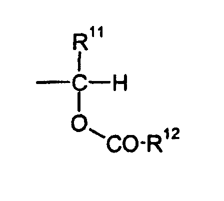 steht, worin R¹¹ für (C₁-C₄)-Alkyl steht, und R¹² für Wasserstoff, (C₁-C₄)-Alkyl oder für eine Gruppe der Formel 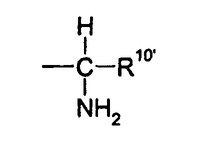 steht, worin R^{10'} die Seitengruppe einer natürlich vorkommenden α-Aminosäure darstellt, oder
- R² und R³: gemeinsam mit dem Stickstoffatom einen 5- bis 6-gliedrigen gesättigten Heterocyclus bildet, der gegebenenfalls noch ein Sauerstoffatom aufweisen kann,
- R⁴: für Wasserstoff, (C₁-C₆)-Acyl, (C₂-C₆)-Alkenyl, (C₃-C₈)-Cycloalkyl steht, oder
- R⁴: für (C₁-C₆)-Alkyl steht, das gegebenfalls substituiert sein kann durch 1 bis 3 Substituenten, die aus der Gruppe ausgewählt werden, die aus Halogen, Hydroxy, (C₃-C₈)-Cycloalkyl, (C₁-C₆)Acyl, (C₁-C₆)-Alkoxy, Carboxyl, 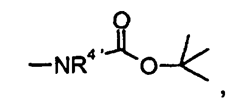 worin R^{4'} für Wasserstoff steht, -(OCH₂CH₂)ₙOCH₂CH₃, worin n 0 oder 1 ist, Phenoxy, (C₆-C₁₀)-Aryl und -NR¹³R¹⁴ besteht,
worin R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder R¹³ und R¹⁴ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR¹⁵ enthalten kann, und durch Oxo substituiert sein kann,
worin
R¹⁵ Wasserstoff oder (C₁-C₄)-Alkyl bedeutet, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 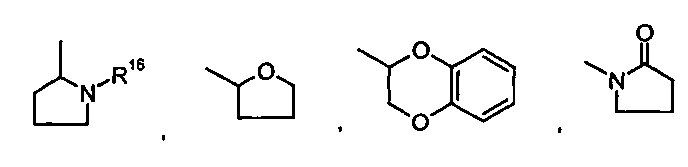 oder 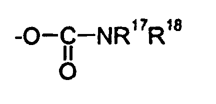 substituiert ist, worin
R¹⁶ Wasserstoff oder (C₁-C₆)-Alkyl bedeutet,
R¹⁷ und R¹⁸ gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
- R⁵: für Wasserstoff, (C₁-C₆)-Alkyl, Halogen, Amino, Mono- oder Di(C₁-C₆)-Alkylamino oder für (C₁-C₆)-Alkanoylamino steht,
- R⁶: für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-lkyl, Halogen-(C₁-C₆)-lkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, Tri-(C₁-C₆)-alkylsilyloxy, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkylthio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formel 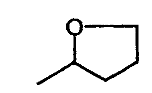 substituiert ist,
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-(C₁-C₆)-alkoxy, Amino, (C₁-C₆)-Alkylthio, Hydroxy, Carboxyl, Carbamoyl, Aminocarbonyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen-(C₁-C₆)-alkyl und Hydroxy-(C₁-C₆)-alkyl substituiert sein kann,
- (C₂-C₆)-Alkenyl
   und Gruppen der Formeln
   - -OR¹⁹,
   - -NR²⁰R²¹ oder -CO-NR²²R²³,
   - Carbazol, Dibenzofuran oder Dibenzothiophen,
   - Xanthen oder 9,10-Dihydroacridin
   besteht,
worin R¹⁹ Phenyl bedeutet, das seinerseits gegebenenfalls durch eine Gruppe der Formel -NR²⁴R²⁵ substituiert ist,
worin
R²⁴ und R²⁵ gleich oder verschieden sind und Wasserstoff, (C₁-C₆)-Alkyl oder (C₁-C₆)-Acyl bedeuten,
oder
R¹⁹ (C₁-C₆)-Alkyl bedeutet, das gegebenenfalls ein- bis dreifach durch Hydroxy und/oder Halogen substituiert ist,
R²⁰ und R²¹ gleich oder verschieden sind und Wasserstoff, Carbamoyl, Mono- oder Di-(C₁-C₆)-alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R²² und R²³ gleich oder verschieden sind und Wasserstoff oder (C₁-C₆)-Alkyl bedeuten, und R⁷ die Bedeutung von R⁵ aufweisen kann und mit dieser gleich oder verschieden sein kann,
und deren Salze.

Physiologisch unbedenkliche Salze der erfindungsgemäßen Verbindungen können beispielsweise Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können weiterhin Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Die Erfindung schließt in ihrem Umfang auch solche Verbindungen ein, die erst im Körper zu den eigentlichen Wirkstoffen der Formel (I) umgewandelt werden (sogenannte Prodrugs).

(C₁-C₆)-Alkyl steht zweckmäßig für einen geradkettigen oder verzweigten Alkylrest mit 1 bis 6 Kohlenstoffatomen. Bevorzugt ist ein geradkettiger oder verzweigter Alkylrest mit 1 bis 4 Kohlenstoffatomen-(C₁-C₄). Beispielsweise seien genannt:

Methyl, Ethyl, n-Propyl, Isopropyl, n-Butyl, sek.-Butyl, tert.-Butyl, n-Pentyl und n-Hexyl. Besonders bevorzugt ist ein geradkettiger oder verzweigter Alkylrest mit 1 bis 3 Kohlenstoffatomen ((C₁-C₃)-Alkyl).

Halogen(C₁-C₆)-alkyl steht zweckmäßig für eine (C₁-C₆)-Alkylgruppe, die wie oben definiert sein kann, und die 1 bis 3 Halogenatome, nämlich F, Cl, Br und/oder I, bevorzugt Chlor oder Fluor, als Substituenten aufweist, beispielsweise seien erwähnt Trifluormethyl, Fluormethyl etc.

Hydroxy(C₁-C₆)-alkyl steht zweckmäßig für eine (C₁-C₆)-Alkylgruppe, die wie oben definiert sein kann, und die 1 bis 3 Hydroxygruppen als Substituenten aufweist, beispielsweise seien erwähnt Hydroxymethyl etc.

(C₂-C₆)-Alkenyl steht im Rahmen der Erfindung zweckmäßig für einen geradkettigen oder verzweigten Alkenylrest mit 2 bis 6 Kohlenstoffatomen. Beispielsweise seien genannt: Ethenyl, n-Prop-2-en-1-yl und n-But-2-en-1-yl. Bevorzugt ist ein geradkettiger oder verzweigter Alkenylrest mit 2 bis 4 Kohlenstoffatomen.

(C₁-C₆)-Alkoxy steht zweckmäßig für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen-(C₁-C₄). Beispielsweise seien genannt: Methoxy, Ethoxy, n-Propoxy, Isopropoxy, tert.-Butoxy, n-Pentoxy und n-Hexoxy. Besonders bevorzugt ist ein geradkettiger oder verzweigter Alkoxyrest mit 1 bis 3 Kohlenstoffatomen-(C₁-C₃).

Halogen-(C₁-C₆)-alkoxy steht zweckmäßig für einfach oder mehrfach halogensubstituiertes (C₁-C₆)-Alkoxy. Bezüglich des (C₁-C₆)-Alkoxy-Anteils sowie der Definition von Halogen sei auf die obige Definition verwiesen. Beispielsweise schließt Halogen-(C₁-C₆)-alkoxy ein oder mehrfach partiell chloriertes und/oder fluoriertes oder perfluoriertes (C₁-C₆)Alkoxy ein wie Trifluormethoxy, Fluormethoxy, Chlormethoxy, Pentafluorethoxy, Trifluormethylmethoxy etc. ein.

Partiell fluoriertes (C₁-C₆)-Alkoxy mit bis zu 6 Fluoratomen steht zweckmäßig für einen geradkettigen oder verzweigten Alkoxyrest mit 1 bis 6 Kohlenstoffatomen, der mit 1 bis 6, bevorzugt 1 bis 4, noch bevorzugter 1 bis 3 Fluoratomen substituiert sein kann. Bevorzugt ist ein geradkettiger oder verzweigter Alkoxyrest mit 1 bis 4 Kohlenstoffatomen und 1 bis 4 Fluoratomen. Beispielsweise seien genannt: Methoxy, Ethoxy, n-Propoxy, Isopropoxy, tert.-Butoxy, n-Pentoxy und n-Hexoxy, die jeweils ein bis 4 Fluoratome aufweisen. Besonders bevorzugt sind (1,3-Difluorprop-2-yl)-oxy und 1,1,2,2-Tetrafluorethoxy.

(C₁-C₆)-Alkylthio steht zweckmäßig für einen geradkettigen oder verzweigten Alkythiorest mit 1 bis 6 Kohlenstoffatomen. Bevorzugt ist ein geradkettiger oder verzweigter Alkylthiorest mit 1 bis 4 Kohlenstoffatomen-(C₁-C₄). Beispielsweise seien genannt: Methylthio, Ethylthio, n-Propylthio, Isopropylthio, tert.-Butylthio, n-Pentylthio und n-Hexylthio. Besonders bevorzugt ist ein geradkettiger oder verzweigter Alkylthiorest mit 1 bis 3 Kohlenstoffatomen (C₁-C₃)-Alkylthio.

(C₁-C₆)-Alkoxycarbonyl steht zweckmäßig für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen-(C₁-C₄). Beispielsweise seien genannt: Methoxycarbonyl, Ethoxycarbonyl, n-Propoxycarbonyl, Isopropoxycarbonyl und tert.-Butoxycarbonyl. Besonders bevorzugt ist ein geradkettiger oder verzweigter Alkoxycarbonylrest mit 1 bis 4 Kohlenstoffatomen-(C₁-C₄).

Mono- oder Di-(C₁-C₆)-alkylaminocarbonyl zweckmäßig steht im Rahmen der Erfindung zweckmäßig für eine Carbamoylgruppe (H₂N-CO-), in der ein oder beide Wasserstoffatome durch eine (C₁-C₆)-Alkylgruppe ersetzt sind. Bezüglich der Definition der (C₁-C₆)-Alkylgruppe sei auf die obigen Erläuterung von (C₁-C₆)-Alkyl verwiesen. Beispielsweise seien erwähnt Methylaminocarbonyl, Dimethylaminocarbonyl etc.

Mono- oder Di- (C₁-C₆)-acylamino steht im Rahmen der Erfindung zweckmäßig für eine Aminogruppe (H₂N-), in der ein oder beide Wasserstoffatome durch eine (C₁-C₆)-Acylgruppe ersetzt sind. Bezüglich der Definition der (C₁-C₆)-Acylgruppe sei auf die obigen Erläuterung von (C₁-C₆)Acyl verwiesen. Beispielsweise seien erwähnt (C₁-C₆)Alkanoyl, wie in der Definition von (C₁-C₆)Acyl erwähnt.

(C₁-C₆)-Alkylsulfoxy stellt zweckmäßig eine (C₁-C₆)-Alkyl-S(=O)-Gruppe dar, wobei bezüglich der (C₁-C₆)-Alkylgruppe auf die diesbezügliche obige Definition verwiesen werden kann.

(C₁-C₆)-Alkylsulfonyl stellt zweckmäßig eine (C₁-C₆)-Alkyl-SO₂-Gruppe dar, wobei bezüglich der (C₁-C₆)-Alkylgruppe auf die diesbezügliche obige Definition verwiesen werden kann.

(C₆-C₁₀)-Aryl steht im allgemeinen für einen aromatischen Rest mit 6 bis 10 Kohlenstoffatomen. Bevorzugte Arylreste sind Phenyl und Naphthyl.

(C₁-C₆)-Acyl steht im Rahmen der Erfindung zweckmäßig für einen geradkettigen oder verzweigten Acylrest mit 1 bis 6 Kohlenstoffatomen. Beispielsweise seien genannt: Formyl, Acetyl, Ethanoyl, Propanoyl, Isopropanoyl, Butanoyl, Isobutanoyl und Pentanoyl. Bevorzugt ist ein geradkettiger oder verzweigter Acylrest mit 1 bis 4 Kohlenstoffatomen. Besonders bevorzugt sind Acetyl und Ethanoyl.

(C₃-C₈)-Cycloalkyl steht im Rahmen der Erfindung für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl. Die Bedeutung von (C₃-C₆)-Cycloalkyl steht entsprechend zweckmäßig für Cyclopropyl, Cyclopentyl, Cyclobutyl, Cyclohexyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

(C₁-C₆)-Alkanoyl steht im Rahmen der Erfindung für Formyl sowie (C₁-C₅)-Alkylcarbonylgruppen, (C₁-C₅)-Alkyl eine geradkettige oder verzweigtkettige Alkylgruppe mit 1 bis 5 Kohlenstoffatomen sein kann, beispielsweise Acetyl, Propionyl, Butyryl, Pentanoyl.

Ein 5- bis 6-gliedriger aromatischer Heterocyclus mit bis zu 3 Heteroatomen aus der Reihe S, O und/oder N steht beispielsweise für Pyridyl, Pyrimidyl, Thienyl, Furyl, Pyrrolyl, Thiazolyl, N-Triazolyl, Oxazolyl oder Imidazolyl. Bevorzugt sind Pyridyl, Furyl, Thiazolyl und N-Triazolyl.

Ein 5- bis 6-gliedriger aromatischer benzokondensierter Heterocyclus mit bis zu 3 Heteroatomen aus der Reihe S, O und/oder N steht beispielsweise für Benzimidazolyl.

Ein 5- bis 6-gliedriger über ein Stickstoffatom gebundener gesättigter Heterocyclus, der aus zwei Substituentengruppen zusammen mit dem Stickstoffatom, an das sie gebunden sind, gebildet werden kann, und der gegebenenfalls ein weiteres Heteroatom aus der Reihe S oder O oder einem Rest der Formel -NR¹⁵, worin R¹⁵ wie oben definiert ist, enthalten kann, steht im Rahmen der Erfindung im allgemeinen für Morpholinyl, Piperidinyl, Piperazinyl, Methylpiperazinyl, Thiomorpholinyl oder Pyrrolidinyl Besonders bevorzugt sind Morpholinyl, Piperidinyl, Pyrrolidinyl und Thiomorpholinyl.

Ein gegebenenfalls über ein Stickstoffatom gebundener 3- bis 8- gliedriger gesättigter oder ungesättigter, nicht aromatischen Heterocyclus mit bis zu 3 Heteroatomen aus der Reihe S, N und/oder O schließt z.B. die oben genannten 5- bis 6-gliedrigen über ein Stickstoffatom gebundenen gesättigten Heterocyclen ein sowie 3-, 7- und 8-gliedrige Heterocyclen, wie z.B. Aziridine (z.B. 1-Azacyclopropan-1-yl), Azetidine (z.B. 1-Azacyclobutan-1-yl) und Azepine (z.B. 1-Azepan-1-yl) ein. Die ungesättigten Vertreter können 1 bis 2 Doppelbindungen im Ring enthalten.

Die Seitengruppe einer natürlich vorkommenden α-Aminosäure in der Bedeutung von R¹⁰ schließt beispielsweise ein: Wasserstoff (Glycin), Methyl (Alanin), Propan-2-yl (Valin), 2-Methyl-propan-1-yl (Leucin), 1-Methyl-propan-t-yl (Isoleucin), eine Propan-1,3-diyl-Gruppe, die mit dem Stickstoffatom der Aminogruppe verbunden ist (Prolin), eine 2-Hydroxypropan-1,3-diyl-Gruppe, die mit dem Stickstoffatom der Aminogruppe verbunden ist (Hydroxyprolin), eine Gruppe der Formel 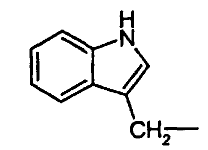 (Tryptophan), eine Benzylgruppe (Phenylalanin), eine Methylthioethylgruppe (Methionin), Hydroxymethyl (Serin), p-Hydroxybenzyl (Tyrosin), 1-Hydroxy-ethan-1-yl (Threonin), Mercaptomethyl (Cystein), Carbamoylmethyl (Asparagin), Carbamoylethyl (Glutamin), Carboxymethyl (Asparaginsäure), Carboxyethyl (Glutaminsäure), 4-Aminobutan-1-yl (Lysin), 3-Guanidinopropan-1-yl (Arginin), Imidazol-4-ylmethyl (Histidin), 3-Ureidopropan-1-yl (Citrullin), Mercaptoethyl (Homocystein), Hydroxyethyl (Homoserin), 4-Amino-3-hydroxybutan-1-yl (Hydroxylysin), 3-Amino-propan-1-yl (Omithin), etc.

In einer weiteren Ausführungsform betrifft die Erfindung Verbindungen der allgemeinen Formel (I) nach Anspruch 1: 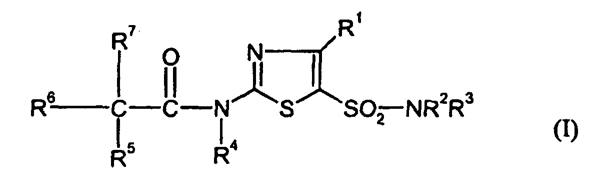 in welcher
- R¹: für Wasserstoff, Halogen, (C₁-C₆)-Alkyl, (C₁-C₆)-Alkoxy, Amino(C₁-C₆)alkyl oder Halogen(C₁-C₆)alkyl steht,
- R² und R³: gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Resten der Formel 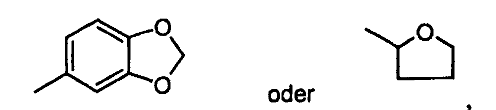 einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8- gliedrigen gesättigten oder ungesättigten, nicht aromatischen, Heterocyclus mit bis zu 3 Heteroatomen aus der Reihe S, N und/oder O, und
(C₆-C₁₀)-Aryl, das seinerseits durch Hydroxy oder (C₁-C₆)-Alkoxy substituiert sein kann, besteht, oder
für eine Gruppe der Formel 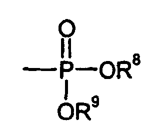 stehen, worin R⁸ und R⁹ gleich oder verschieden voneinander sind und für Wasserstoff und (C₁-C₄)-Alkyl stehen, oder
für eine Gruppe der Formel 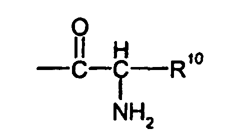 steht, worin R¹⁰ die Seitengruppe einer natürlich vorkommenden α-Aminosäure darstellt, oder
für eine Gruppe der Formel 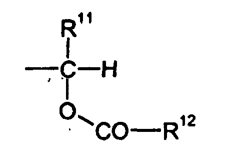 steht, worin R¹¹ für (C₁-C₄)-Alkyl steht, und R¹² für Wasserstoff, (C₁-C₄)-Alkyl oder für eine Gruppe der Formel 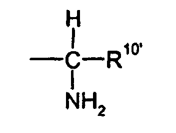 steht, worin R^{10'} die Seitengruppe einer natürlich vorkommenden α-Aminosäure darstellt, oder
- R² und R³: gemeinsam mit dem Stickstoffatom einen 5- bis 6-gliedrigen gesättigten Heterocyclus bildet, der gegebenenfalls noch ein Sauerstoffatom aufweisen kann,
- R⁴: für Wasserstoff, (C₁-C₆)-Acyl, (C₂-C₆)-Alkenyl, (C₃-C₈)-Cycloalkyl steht, oder
- R⁴: für (C₁-C₆)-Alkyl steht, das gegebenfalls substituiert sein kann durch 1 bis 3 Substituenten, die aus der Gruppe ausgewählt werden, die aus Halogen, Hydroxy, (C₁-C₆)Acyl, (C₁-C₆)Alkoxy,
-(OCH₂CH₂)ₙOCH₂CH₃, worin n 0 oder 1 ist, Phenoxy, (C₆-C₁₀)-Aryl und -NR¹³R¹⁴ besteht,
worin R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder R¹³ und R¹⁴ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel ―NR¹⁵ enthalten kann, und durch Oxo substituiert sein kann, worin R¹⁵ Wasserstoff oder (C₁-C₄)-Alkyl bedeutet, oder
- R⁴: für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 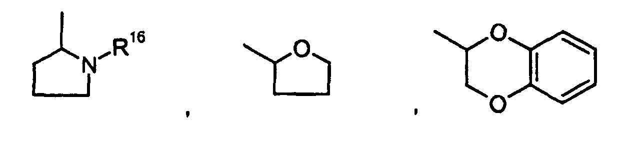 oder 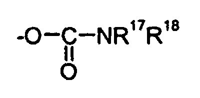 substituiert ist,
worin
R¹⁶ Wasserstoff oder (C₁-C₆)-Alkyl bedeutet,
R¹⁷ und R¹⁸ gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
- R⁵: für Wasserstoff, (C₁-C₆)-Alkyl, Halogen, Amino, Mono- oder Di(C₁-C₆)-Alkylamino oder für (C₁-C₆)-Alkanoylamino steht,
- R⁶: für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)Alkoxycarbonylamino, (C₁-C₆)Alkylsulfoxy, (C₁-C₆)Alkylsulfonyl, Tri(C₁-C₆)alkylsilyloxy, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkyithio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formel 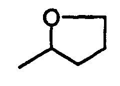 substituiert ist,
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)Alkoxycarbonylamino, (C₁-C₆)Alkylsulfoxy, (C₁-C₆)Alkylsulfonyl, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycalbonylamino, (C₁-C₆)-Alkyl, Halogen(C₁-C₆)alkyl und Hydroxy(C₁-C₆)-alkyl substituiert sein kann,
   und Gruppen der Formeln
   - ―OR¹⁹,
   - -NR²⁰R²¹ oder -CO-NR²²R²³
besteht,
worin R¹⁹ Phenyl bedeutet, das seinerseits gegebenenfalls durch eine Gruppe der Formel -NR²⁴R²⁵ substituiert ist, worin
R²⁴ und R²⁵ gleich oder verschieden sind und Wasserstoff, (C₁-C₆)-Alkyl oder (C₁-C₆)-Acyl bedeuten,
oder
R¹⁹ (C₁-C₆)-Alkyl bedeutet, das gegebenenfalls ein- bis dreifach durch Hydroxy und/oder Halogen substituiert ist,
- R²⁰ und R²¹: gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
- R²² und R²³: gleich oder verschieden sind und Wasserstoff oder (C₁-C₆)-Alkyl bedeuten,
und R⁷ die Bedeutung von R⁵ aufweisen kann und mit dieser gleich oder verschieden sein kann,
und deren Salze.

In einer bevorzugten Ausführungsform betrifft die Erfindung Verbindungen der allgemeinen Formel (I) 1, worin R¹ für Wasserstoff oder (C₁-C₆)-Alkyl steht.

In einer weiteren bevorzugten Ausführungsform betrifft die Erfindung Verbindungen der allgemeinen Formel (I), worin R² und R³ jeweils unabhängig für Wasserstoff oder (C₁-C₆)-Alkyl stehen.

In einer weiteren bevorzugten Ausführungsform betrifft die Erfindung Verbindungen der allgemeinen Formel (I), worin R⁴ für Wasserstoff oder (C₁-C₆)-Alkyl steht.

In einer weiteren bevorzugten Ausführungsform betrifft die Erfindung Verbindungen der allgemeinen Formel (I) nach Anspruch, worin R⁵ für Wasserstoff steht.

In einer weiteren bevorzugten Ausführungsform betrifft die Erfindung Verbindungen der allgemeinen Formel (I), worin
- R⁶: für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxy-carbonyl, Nitro, Halogen(C₁-C₆)alkyl, Halogen(C₁-C₆)alkoxy, Amino, Hydroxy, Mono- oder Di(C₁-C₆)alkylamino, Mono- oder Di(C₁-C₆)-Alkanoylamino, (C₁-C₆)Alkoxycarbonylamino, und/oder Cyano substituiert sein kann, und
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 2 Halogenatome substituiert sein kann,
besteht.

In einer weiteren bevorzugten Ausführungsform betrifft die Erfindung Verbindungen, die die folgende Formel aufweisen: 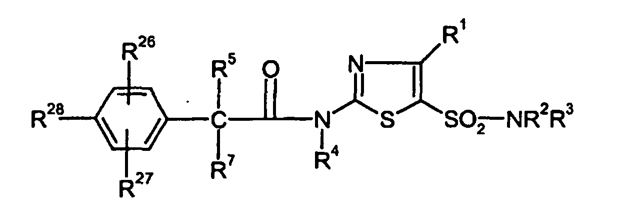 worin
R¹, R², R³, R⁴, R⁵ und R⁷ wie im Anspruch 1 definiert sind,
- R²⁶ und R²⁷: gleich oder verschieden sind und für Wasserstoff, Halogen, (C₁-C₆)-Alkoxy, (C₁-C₆)-Alkoxycarbonyl, (C₁-C₆)-Alkylthio, Hydroxy, Carboxyl, partiell fluoriertes (C₁-C₆)-Alkoxy mit bis zu 6 Fluoratomen, (C₁-C₆)-Alkyl, eine Gruppe der Formeln -OR¹⁹, -NR²⁰R²¹ oder -CO-NR²²R²³ stehen, worin
- R¹⁹: Phenyl bedeutet, das seinerseits gegebenenfalls durch eine Gruppe der Formel -NR²⁴R²⁵ substituiert ist,
worin
- R²⁴ und R²⁵: gleich oder verschieden sind und Wasserstoff, (C₁-C₆)-Alkyl oder (C₁-C₆)-Acyl bedeuten, oder
- R¹⁹: (C₁-C₆)-Alkyl bedeutet, das gegebenenfalls ein- bis dreifach durch Hydroxy und/oder Halogen substituiert ist,
- R²⁰ und R²¹.: gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
- R²² und R²³: gleich oder verschieden sind und Wasserstoff oder (C₁-C₆)-Alkyl bedeuten,
- R²⁸: für (C₆-C₁₀)-Aryl steht, das gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-(C₁-C₆)-alkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, Tri-(C₁-C₆)-alkylsilyloxy, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann, oder
- R²⁸: für einen gegebenenfalls über ein Stickstoffatom gebundenen 5- bis 6-gliedrigen aromatischen Heterocyclus mit bis zu 3 Heteroatomen aus der Reihe S, N und/oder O steht, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-C₁-C₆)-alkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
und deren Salze.

Besonders bevorzugt sind z.B. die Verbindung N-[5-(Aminosulfonyl)-4-methyl-1,3-thiazol-2-yl]-2-[1,1'-biphenyl]-4-yl-N-methylacetamid der Formel: 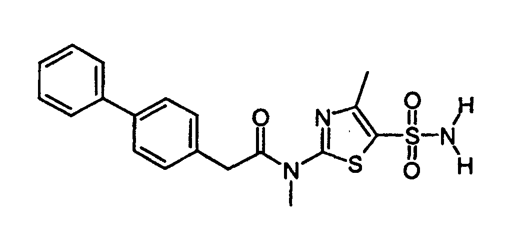 die Verbindung N-[5-(Aminosulfonyl)-4-methyl-1,3-thiazol-2-yl]-2-(2'-fluor[1,1'-biphenyl]-4-yl)-N-methylacetamid der Formel: 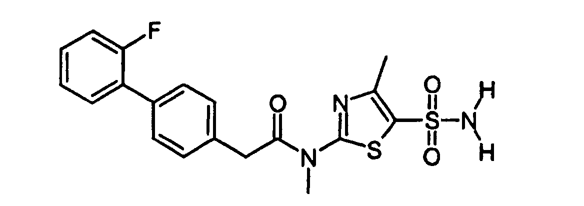 sowie die Verbindung N-[5-(aminosulfonyl)-4-methyl-1,3-thiazol-2-yl]-N-methyl-2-[4-(2-pyridinyl)phenyl]acetamid der Formel: 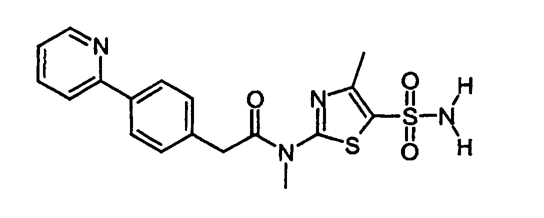 und pharmazeutisch verträgliche Salze davon.

Die Erfindung betrifft ferner Verbindungen der allgemeinen Formel (IV) 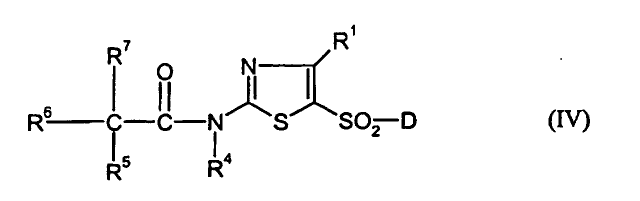 worin
- R¹, R⁴, R⁵, R⁶ und R⁷: die für die Formel (I) angegebene Bedeutung haben, und D ein Halogenatom ist.

Die Erfindung betrifft weiterhin Verfahren zur Herstellung der Verbindungen der allgemeinen Formel (I), dadurch gekennzeichnet, dass man
[A] Verbindungen der allgemeinen Formel (II) 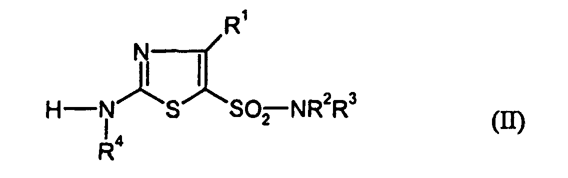 in welcher
   R¹, R², R³ und R⁴ die oben angegebene Bedeutung haben,
   mit Verbindungen der allgemeinen Formel (III) 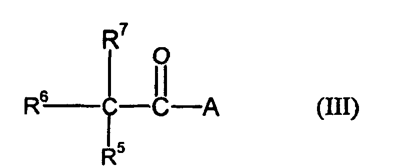 in welcher
   - A: für eine Abgangsgruppe, wie z.B. Halogen, vorzugsweise Chlor, oder Hydroxy steht, und R⁵, R⁶ und R⁷ die oben angegebene Bedeutung haben, in inerten Lösemitteln, gegebenenfalls in Anwesenheit einer Base und/oder eines Hilfsmittels zu Verbindungen der Formel (I) umsetzt,
[B] Verbindungen der allgemeinen Formel (IV) 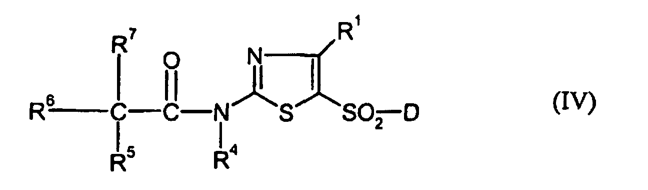 worin
   - R¹, R⁴, R⁵, R⁶ und R⁷: die oben angegebene Bedeutung haben, und D ein Halogenatom, vorzugsweise Chlor ist, mit Aminen der allgemeinen Formel (V):

   HNR²R³ (V)

   worin
   - R² und R³: die oben angegebene Bedeutung haben, in inerten Lösemitteln zu Verbindungen der Formel (I) umsetzt,
[C] Verbindungen der allgemeinen Formel (X) 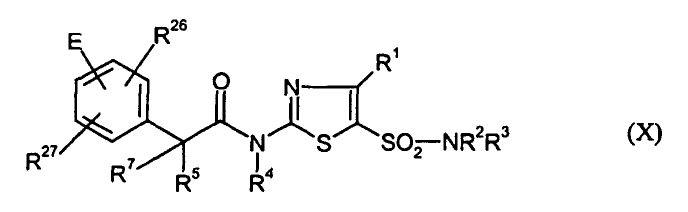 worin
   - R¹, R², R³, R⁴, R⁵, R⁷, R²⁶ und R²⁷: die oben angegebene Bedeutung haben, und E Trifluormethansulfonat oder Halogen, vorzugsweise Brom oder Iod ist, mit Boronsäuren oder Stannanen der allgemeinen Formel (XI):

   R²⁸M (XI)

   worin
   - R²⁸: die oben angegebene Bedeutung hat und M beispielsweise eine Tri(C₁-C₆)alkylstannylgruppe, wie eine Trimethylstannylgruppe oder eine Boronsäuregruppe sein kann, in inerten Lösemitteln in Gegenwart von Palladiumkatalysatoren, z.B. Tetrakis(triphenylphosphan)palladium (0), ggf. in Anwesenheit von Base, z.B. Kaliumphosphat bei Temperaturen von 50 - 140 °C zu Verbindungen der Formel (XIV) 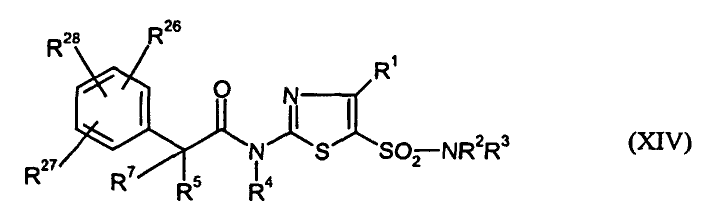
   umsetzt, und
[D] Verbindungen der allgemeinen Formel (XII) 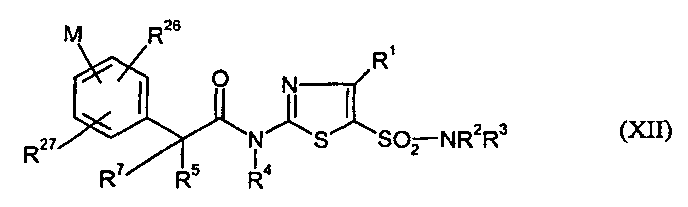
worin
- R¹, R², R³, R⁴, R⁵, R⁷, R²⁶ und R²⁷: die oben angegebene Bedeutung haben, und M die oben angegebene Bedeutung besitzt, mit Trifluormethansulfonaten oder Halogeniden der allgemeinen Formel (XIII):

R²⁸E (XIII)

worin
- R²⁸: die oben angegebene Bedeutung hat und E die oben angegebene Bedeutung besitzt, in inerten Lösemitteln in Gegenwart von Palladiumkatalysatoren, z.B. Tetrakis(triphenylphosphan)palladium (0), ggf. in Anwesenheit von Base, z.B. Kaliumphosphat bei Temperaturen von 50 - 140 °C zu Verbindungen der Formel (XIV) umsetzt.

Das erfindungsgemäße Verfahren [A] kann durch folgendes Formelschemata beispielhaft erläutert werden:

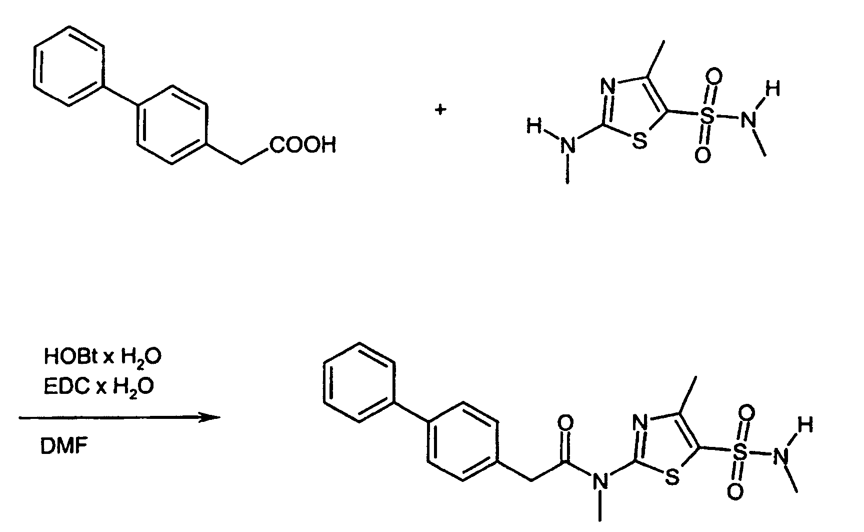

Hierin bedeuten:
HOBt: 1-Hydroxy-1H-benzotriazol
EDC: *N'*-(3-Dimethylaminopropyl)-*N*-ethylcarbodiimid x HCl
DMF: *N*,*N*-Dimethylformamid

Das erfindungsgemäße Verfahren [C] kann durch folgendes Formelschemata beispielhaft erläutert werden:

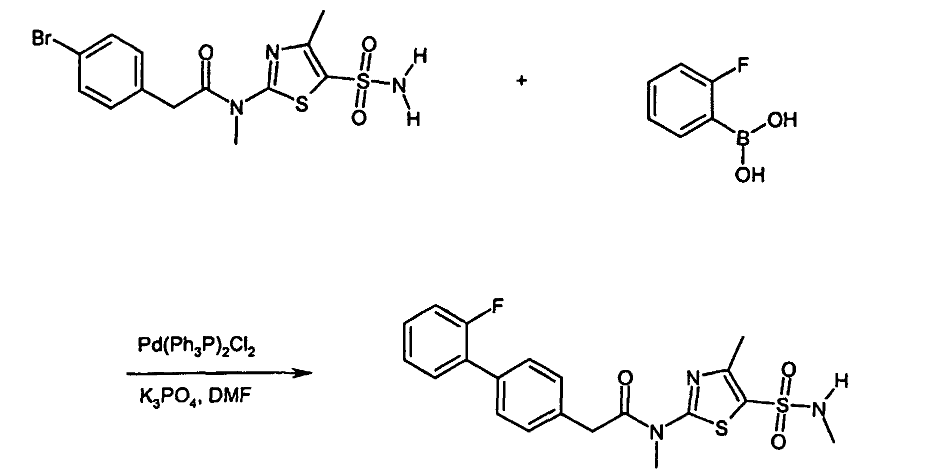

Hierin bedeutet:
DMF: *N*,*N-*Dimethylformamid

Das erfindungsgemäße Verfahren [D] kann durch folgendes Formelschemata beispielhaft erläutert werden:

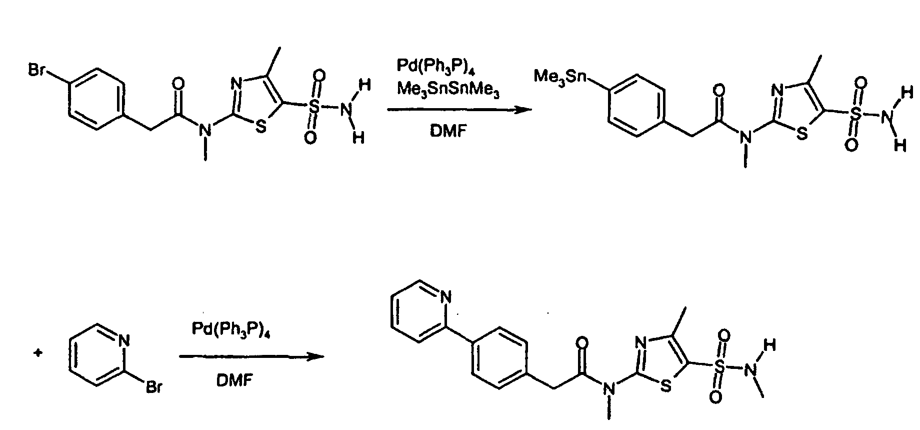

Hierin bedeutet:
DMF: *N,N*-Dimethylformamid

Als Lösemittel für die Verfahren [A], [B], [C] und [D] eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid (DMF) oder Acetonitril. Ebenso ist es möglich, Gemische der genannten Lösemittel zu verwenden. Bevorzugt ist DMF.

Als Basen für das erfindungsgemäße Verfahren [A] können im allgemeinen anorganische oder organische Basen eingesetzt werden. Hierzu gehören vorzugsweise organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, N-Methylmorpholin oder N-Methylpiperidin oder Morpholin. Bevorzugt ist Triethylamin.

Als Hilfsmittel eignen sich an sich bekannte Dehydratisierungs- bzw. Kupplungsreagenzien, wie beispielsweise Carbodiimide, wie Diisopropylcarbodiimid, Dicyclohexylcarbodiimid (DCC) oder *N*-(3-Dimethylaminopropyl)-*N*'-ethylcarbodiimid (EDC), oder Carbonylverbindungen wie Carbonyldiimidazol (CDI) oder Isobutyl-chloroformiat, oder 1,2-Oxazoliumverbindungen wie 2-Ethyl-5-phenyl-1,2-oxazolium-3-sulfonat, oder Phosphorverbindungen wie Propanphosphonsäureanhydrid, Phosphorsäurediphenylesterazid, Benzotriazolyl-*N*-oxy-tris(dimethylamino)phosphonium-Hexafluorophosphat (BOP), oder Uronium-Verbindungen wie *O*-Benzotriazol-1-yl-*N*,*N*,*N*',*N*'-tetramethyluronium-Hexafluoro-phosphat (HBTU), oder Methansulfonsäurechlorid, gegebenenfalls in Gegenwart von Hilfsstoffen wie N-Hydroxysuccinimid oder N-Hydroxybenzotriazol.

Im allgemeinen setzt man die Base in einer Menge von 0,05 Mol bis 10 Mol, bevorzugt von 1 Mol bis 2 Mol bezogen auf 1 Mol der Verbindung der Formel (III) ein.

Die erfindungsgemäßen Verfahren werden im allgemeinen in einem Temperaturbereich von -50°C bis +100°C, bevorzugt von -30°C bis +60°C, durchgeführt.

Die erfindungsgemäßen Verfahren werden im allgemeinen bei Normaldruck durchgerührt. Es ist aber auch möglich, das Verfahren bei Überdruck oder bei Unterdruck durchzuführen (z.B. in einem Bereich von 0,5 bis 5 bar).

Die Verbindungen der allgemeinen Formel (II) können beispielsweise hergestellt werden, indem man Verbindungen der allgemeinen Formel (VI) 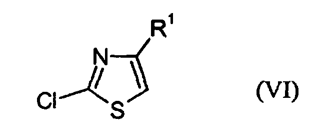 in welcher
- R¹: die oben angegebene Bedeutung hat,
durch Umsetzung mit dem System Chlorsulfonsäure/SOCl₂ in die Verbindungen der allgemeinen Formel (VII) 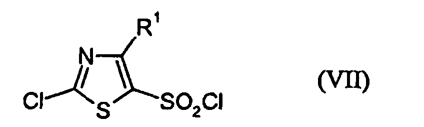 in welcher
- R¹: die oben angegebene Bedeutung hat,
überführt, anschließend mit Aminen der allgemeinen Formel (V)

HNR²R³ (V)

in welcher
R² und R³ die oben angegebene Bedeutung haben,
in inerten Lösemitteln die Verbindungen der allgemeinen Formel (VIII) 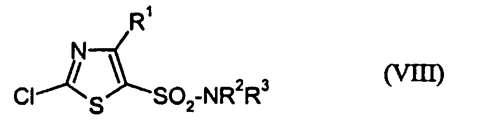 in welcher
R¹, R² und R³ die oben angegebene Bedeutung haben,
herstellt, und in einem letzten Schritt eine Umsetzung mit Aminen der allgemeinen Formel (IX)

H₂N-R^{4'} (IX)

in welcher
- R^{4'}: die oben angegebene Bedeutung von R⁴ hat und mit dieser gleich oder verschieden ist, aber nicht Wasserstoff ist,
in inerten Lösemitteln und in Anwesenheit einer Base durchführt.

Die Reaktion mit Chlorsulfonsäure/SO₂Cl erfolgt zunächst bei Raumtemperatur und anschließend unter der Rückflußtemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Lösemittel für die Umsetzung mit den Aminen der allgemeinen Formel (V) eignen sich Alkohole wie beispielsweise Methanol, Ethanol, Propanol und Isopropanol. Bevorzugt ist Methanol.

Die Umsetzung mit den Aminen der allgemeinen Formel (V) erfolgt zunächst bei Raumtemperatur und anschließend unter der Rückflußtemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Umsetzung mit den Verbindungen der allgemeinen Formel (IX) erfolgt in Ethern wie beispielsweise Diethylether, Dioxan, Tetrahydrofuran oder Glykoldimethylether. Bevorzugt ist Methanol.

Als Basen können im allgemeinen anorganische oder organische Basen eingesetzt werden. Hierzu gehören vorzugsweise organische Amine (Tri(C₁-C₆)alkylamine, wie Triethylamin), oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Bevorzugt ist Triethylamin.

Im allgemeinen setzt man die Base in einer Menge von 0,05 Mol bis 10 Mol, bevorzugt von 1 Mol bis 2 Mol bezogen auf 1 Mol der Verbindung der Formel (VIII) ein.

Die Verbindungen der allgemeinen Formel (VI) sind teilweise bekannt oder nach üblichen Methoden herstellbar [vgl. Hantzsch, Chem. Ber. 1927, 60, 2544].

Die Verbindungen der allgemeinen Formel (VII) und (VIII) sind neu und können wie oben beschrieben hergestellt werden.

Amine der allgemeinen Formeln (V) und (IX) sind bekannt.

Verbindungen der allgemeinen Formeln (III) sind bekannt oder lassen sich nach literaturbekannten Verfahren herstellen.

Biphenylmethylcarbonsäure- bzw. Biphenylessigsäurederivate der Formel (III) lassen sich in an sich bekannter Weise durch übergangsmetallkatalysierte, beispielsweise palladiumkatalysierte Kupplungsreaktionen, wie z.B. der Suzuki- oder Stille-Kupplung herstellen. Die Pyridylphenylmethylcarbonsäurederivate der Formel (III) sind literaturbekannt (siehe z.B. M. Artico et al. in *Eur. J*. *Med*. *Chem.* (1992) 27, 219-228) oder lassen sich nach an sich bekannten Verfahren herstellen. Die folgenden Reaktionsschemata A, B, C und D illustrieren beispielhaft die Synthese von Biphenylessigsäurederivaten aus den entsprechenden Boronsäuren sowie die Synthese von Pyridylphenylessigsäurederivaten aus den entsprechenden Stannylverbindungen:

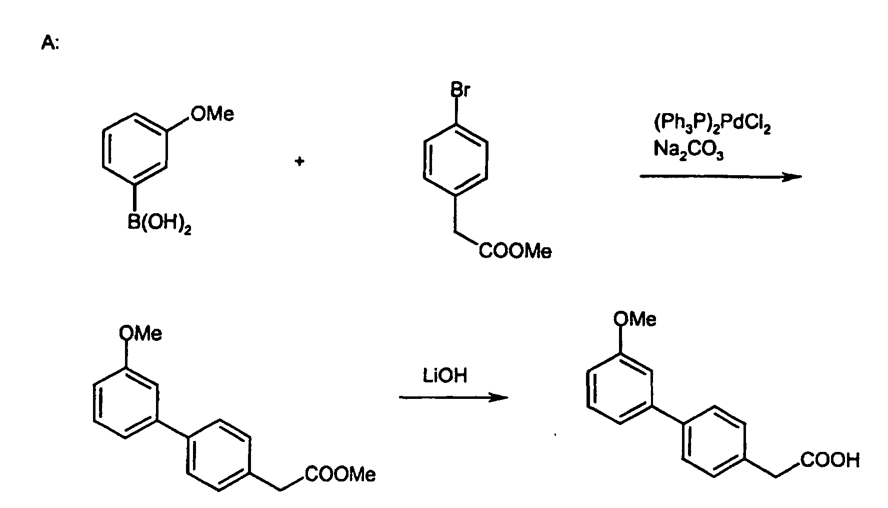

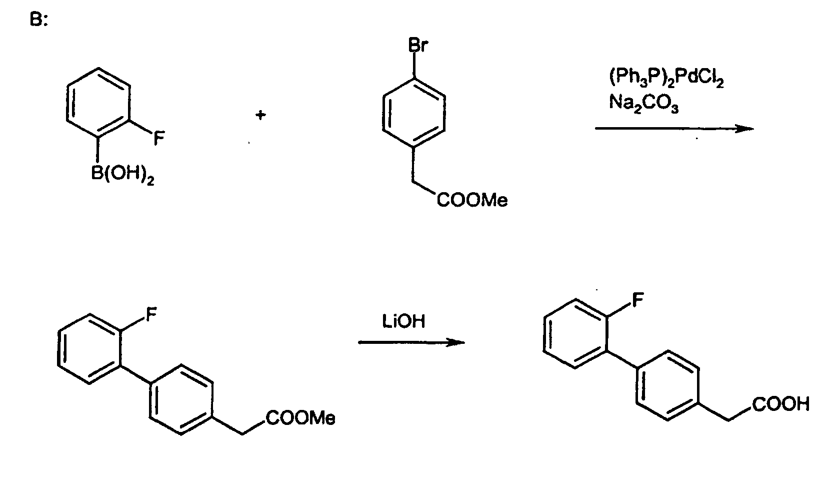

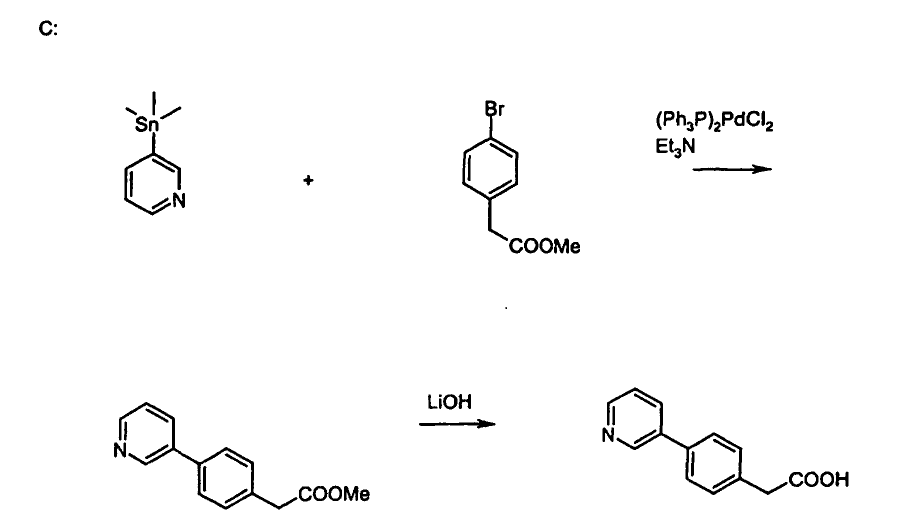

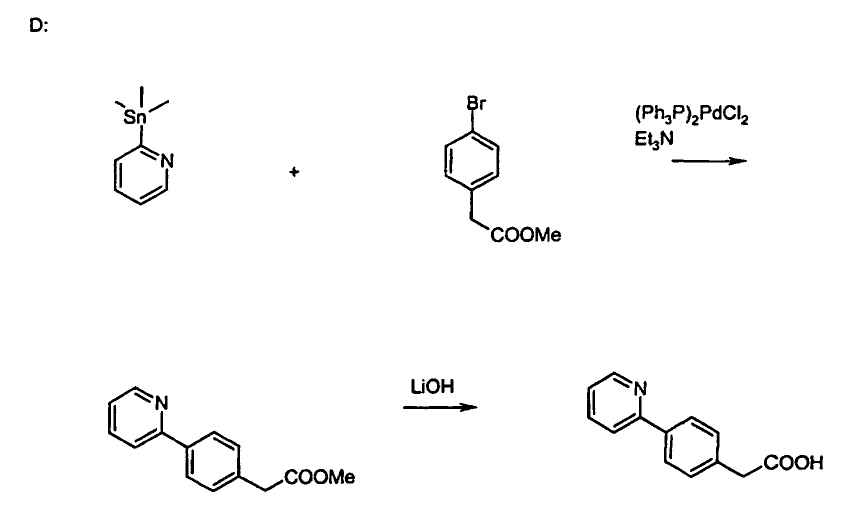

Verbindungen der Formel (III), in denen R⁵ und R⁷ zum Beispiel Fluor ist, lassen sich nach dem im folgenden Reaktionsschema gezeigten Verfahren herstellen:

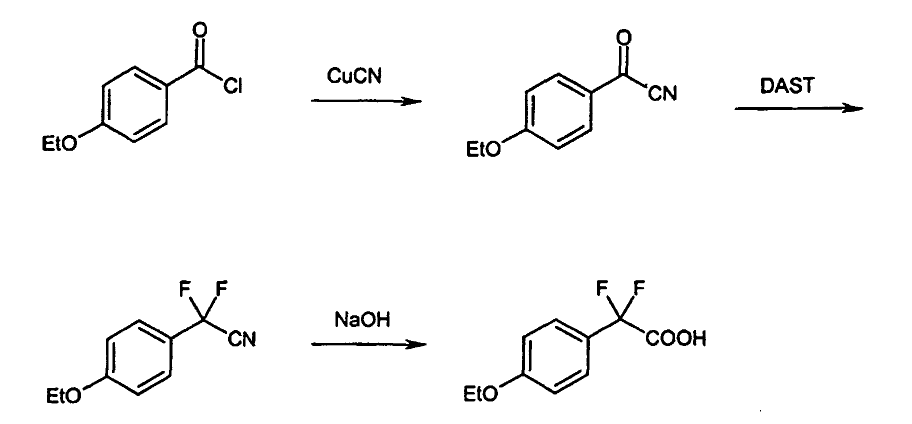

Die Fluorierung mit DAST (N,N-Diethylaminoschwefeltrifluorid) erfolgt dabei gemäß J. Fluor. Chem. 61, 1993, 117.

Die Erfindung betrifft ferner die Verbindungen der Formel (I) Anspruch 1 zur Verwendung als Arzneimittel.

Die Erfindung betritt ferner eine pharmazeutische Zusammensetzung, die eine Verbindung der allgemeinen Formel (I) in Mischung mit mindestens einem pharmazeutisch verträglichen Träger oder Exzipienten umfaßt.

Die Erfindung betrifft ferner die Verwendung einer Verbindung der allgemeinen Formel (I) 1 zur Herstellung eines Arzneimittels, insbesondere eines Arzneimittels zur Behandlung und/oder Prävention viraler Infektionen, wie Herpes Viren, insbesondere Herpes Simplex-Viren.

Die Erfindung betrifft ferner die Verwendung von N-[5-(aminosulfonyl)-1,3-thiazol-2-yl]acetamid-Derivaten, bevorzugt von N-[5-(aminosulfonyl)-1,3-thiazol-2-yl]-2-phenylacetamid-Derivaten, noch bevorzugter von N-[5-(aminosulfonyl)-1,3-thiazol-2-yl]-2-[1,1'-biphenyl)-4-ylacetamid-Derivaten zur Herstellung von Arzneimitteln, insbesondere die Verwendung der genannten Derivate zur Herstellung von Mitteln zur Behandlung und/oder Prävention von viralen Infektionen bei Menschen oder Tieren, wie durch Herpes-Viren, insbesondere durch Herpes Simplex-Viren. N-[5-(Aminosulfonyl)-1,3-thiazol-2-yl]acetamid-Derivate, N-[5-(Aminosulfonyl)-1,3-thiazol-2-yl]-2-phenylacetamid-Derivate bzw. N-[5-(Aminosulfonyl)-1,3-thiazol-2-yl]-2-[1,1'-biphenyl]-4-ylacetamid-Derivate meint hier solche Verbindungen, die aus der Substitution eines oder mehrerer Wasserstoffatome aus N-[5-(Aminosulfonyl)-1,3-thiazol-2-yl]acetamid, N-[5-(Arninosulfonyl)-1,3-thiazol-2-yl]-2-phenylacetamid bzw. N-[5-(Aminosulfonyl)-1,3-thiazol-2-yl]-2-[1,1'-biphenyl]-4-ylacetamid ableiten.

Die erfindungsgemäßen Verbindungen der allgemeinen Formeln (I) zeigen ein nicht vorhersehbares überraschendes Wirkspektrum. Sie zeigen eine antivirale Wirkung gegenüber Vertretern der Gruppe Herpes viridae, besonders gegenüber den Herpes Simplex Viren (HSV). Sie sind somit zur Behandlung und Prophylaxe von Erkrankungen, die durch Herpes-Viren, insbesondere Erkrankungen, die durch Herpes Simplex Viren hervorgerufen werden.

### In vitro-Aktivität

### Viren und Zellen:

HSV (HSV-1 Walki, HSV-1F oder HSV-2G) wurde auf Vero-Zellen (ATCC CCL-81) unter folgenden Bedingungen vermehrt: Die Zellen wurden in M199 Medium (5 % fötales Kälberserum, 2 mM Glutamin, 100 IU/ml Penicillin, 100 µg/ml streptomycin) in Zellkulturflaschen bei 37°C und 5 % CO₂ gezüchtet. Die Zellen wurden zweimal pro Woche jeweils 1:4 gesplittet. Für die Infektion wurde das Medium abgenommen, die Zellen mit "Hank's solution" gewaschen, mit 0.05 %Trypsin, 0.02 %EDTA (Seromed L2143) abgelöst und mit einer Dichte von 4x10⁵ Zellen pro ml unter den oben genannten Bedingungen für 24 Stunden inkubiert. Dann wurde das Medium abgenommen und die Viruslösung mit einer m.o.i von < 0.05 in einem Volumen von 2 ml pro 175 cm² Oberfläche dazugegeben. Nach einstündiger Inkubation unter den genannten Bedingungen wurde das Medium auf ein Volumen von 50 ml pro 175 cm² -Flasche aufgefüllt. 3 Tage nach Infektion zeigten die Kulturen deutliche Zeichen eines zytopathischen Effektes. Das Virus wurde durch zweimaliges Frieren (-80°C) und Tauen (37°C) freigesetzt. Der Zelldebris wurde durch Zentrifugation (300g, 10min, 4°C) abgetrennt und der Überstand in Aliquots bei -80°C weggefroren.

Der Virustiter wurde über einen Plaque-Assay bestimmt. Dafür wurden Verozellen in einer Dichte von 4x10⁵ Zellen pro Vertiefung in 24 well Platten ausgesät und nach 24 Stunden Inkubation (37°C, 5 % CO₂) mit Verdünnungen des Virusstocks von 10⁻² bis 10⁻¹² (100µl Inokulum) infiziert. 1 Stunde nach Infektion wurde das Medium abgenommen und die Zellen mit 1 ml Overlay-Medium (0.5% Methylcellulose, 0.225 Natriumbikarbonat, 2 mM Glutamin, 100 IU/ml Penicillin, 100 µg/ml Streptomycin, 5 % fötales Kälberserum in MEM-Eagle Medium mit Earl's Salz) überschichtet und für 3 Tage inkubiert. Im Anschluß wurden die Zellen mit 4 % Formalin für 1 Stunde fixiert, mit Wasser gewaschen, mit Giemsa (Merck) für 30 min gefärbt und im Anschluß gewaschen und getrocknet. Mit einem Plaqueviewer wurde der Virustiter bestimmt. Die für die Experimente verwendeten Virusstocks hatten einen Titer von 1 x 10⁶/ml - 1 x 10⁸/ml.

Die Anti-HSV-Wirkung wurde in einem Screening-Testsystem in 96-Well-Mikrotiterplatten unter Zuhilfenahme von diversen Zellinien neuronalen, lymphoiden und epithelialen Ursprungs wie zum Beispiel Vero (Nierenzellinie der grünen Meerkatze), MEF (murine embryonale Fibroblasten), HELF (humane embryonale Fibroblasten), NT2 (humane neuronale Zellinie) oder Jurkat (humane lymphoide T-Zellinie) bestimmt. Der Einfluß der Substanzen auf die Ausbreitung des cytopathogenen Effektes wurde im Vergleich zu der Referenzsubstanz Acyclovir-Natrium (Zovirax^{R}), einem klinisch zugelassenen anti-Herpes-Chemotherapeutikum, bestimmt.

Die in DMSO (Dimethylsulfoxid) gelösten Substanzen (50 mM) werden auf Mikrotiterplatten (z.B. 96-Well MTP) in Endkonzentrationen von 250 - 0,5 µM (mikromolar) in Doppelbestimmungen (4 Substanzen/Platte) untersucht. Bei potenten Substanzen werden die Verdünnungen über mehrere Platten bis 0,5 pM (picomolar) weitergeführt. Toxische und cytostatische Substanzwirkungen werden dabei miterfaßt. Nach den entsprechenden Substanzverdünnungen (1:2) auf der Mikrotiterplatte in Medium wird eine Suspension von Zellen (1x10⁴ Zellen pro Vertiefung) wie zum Beispiel von Vero-Zellen in M199 (Medium 199) mit 5 % fötalem Kälberserum, 2 mM Glutamin und optional 100 IU/ml Penicillin und 100 µg/ml Streptomycin oder MEF-Zellen in EMEM (Eagle's Minimum Essential Medium) mit 10 % fötalem Kälberserum, 2 mM Glutamin und optional 100 IU/ml Penicillin und 100 µg/ml Streptomycin, oder HELF-Zellen in EMEM mit 10 % fötalem Kälberserum, 2 mM Glutamin und optional 100 IU/ml Penicillin und 100 µg/ml Streptomycin, oder NT2- und Jurkat-Zellen in DMEM (4,5 mg/l Glukose plus Pyridoxin) mit 10 % fötalem Kälberserum 2 mM Glutamin, 1 mM Natrium Pyruvat, nicht essentiellen Aminosäuren und optional 100 IU/ml Penicillin und 100 µg/ml Streptomycin in jedes Näpfchen gegeben und die Zellen in den relevanten Vertiefungen mit einer entsprechenden Virusmenge infiziert (HSV-1 F oder HSV-2 G mit einer m.o.i (multiplicity of infection) von 0,0025 für HELF, Vero und MEF Zellen sowie einer m.o.i von 0,1 für NT2- und Jurkat-Zellen). Die Platten werden anschließend bei 37°C in einem CO₂-Brutschrank (5 % CO₂) über mehrere Tage inkubiert. Nach dieser Zeit ist der Zellrasen von z.B. Vero-Zellen in den substanzfreien Viruskontrollen, ausgehend von 25 infektiösen Zentren, durch den cytophatogenen Effekt der HSV-Viren völlig lysiert bzw. zerstört (100 % CPE). Die Platten werden zunächst optisch mit Hilfe eines Mikroskopes ausgewertet und dann mit einem Fluoreszenzfarbstoff analysiert. Hierzu wird der Zellkulturüberstand aller Näpfchen der MTP abgesaugt und mit 200 µl PBS-Waschlösung befüllt. Das PBS wird abermals abgesaugt und alle Wells mit 200 µl Fluoreszenzfarbstofflösung (Fluorescein-diacetate, 10 µg/ml in PBS) befüllt. Nach einer Inkubationszeit von 30-90 min werden die Testplatten in einem Fluoreszenzmessgerät bei einer Anregungswellenlänge von 485 nm und einer Emissionswellenlänge von 538 nm vermessen.

Die Ergebnisse sind für einige Verbindungen in der folgenden Tabelle zusammengefaßt.

**Tabelle**

| **Beispiel** | **IC50 HSV-1 F/Vero** | **IC50 HSV-2 G/Vero** |
|---|---|---|
| 14 | 0,1 µM | 0,75 µM |
| 57 | <0,01 µM | <0,01 µM |
| 8 | 0,1 µM | 0,1 µM |
| 23 | 0,03 µM | 0,1 µM |
| 38 | 0,05 µM | 0,016 µM |
| 87 | < 0,01 µM | < 0,01 µM |
| 126 | 0,01 µM | 0,1 µM |
| Zovirax (Aciclovir-Natrium) | 1 µM | 3 µM |

IC₅₀ bedeutet hier die halbmaximale Fluoreszenzintensität mit Bezug zur nicht infizierten Zellkontrolle (100 % Wert). Man kann den IC₅₀-Wert auch auf eine geeignete Wirkstoffkontrolle (siehe Assaybeschreibung: infizierte Zellen in Gegenwart von einer Substanz mit anti-herpes Wirkung geeigneter Konzentration, wie z.B. Zovirax 20 µM) beziehen. Diese Wirkstoffkontrolle erreicht etwa Fluoreszenzintensitäten von 85 bis 95 % mit Bezug zur Zellkontrolle.

Bevorzugt sind erfindungsgemäße N-[5-(aminosulfonyl)-1,3-thiazol-2-yl]acetamid-Derivate, deren IC₅₀ (HSV-1 F/Vero) im oben beschriebenen in-vitro Screening-Testsystem bevorzugt weniger als 50 µM, bevorzugter weniger als 25 µM und ganz besonders bevorzugt weniger als 10 µM beträgt.

Die erfindungsgemäßen Verbindungen stellen somit wertvolle Wirkstoffe zur Behandlung und Prophylaxe von Erkrankungen dar, die durch Herpes-Viren, insbesondere Herpes Simplex-Viren ausgelöst werden. Als Indikationsgebiete können beispielsweise genannt werden:
1) Behandlung und Prophylaxe von Herpes-Infektionen, insbesondere Herpes Simplex-Infektionen bei Patienten mit Krankheitsbildern wie Herpes labialis, Herpes genitalis, und HSV bedingter Keratitis, Enzephalitis, Pneumonie, Hepatitis etc.
2) Behandlung und Prophylaxe von Herpes-Infektionen, insbesondere Herpes Simplex-Infektionen bei immunsupprimierten Patienten (z.B. AIDS-Patienten, Krebspatienten, Patienten mit genetisch bedingter Immundefiziens, Transplantationspatienten)
3) Behandlung und Prophylaxe von Herpes-Infektionen, insbesondere Herpes Simplex-Infektionen bei Neugeborenen und Kleinkindern
4) Behandlung und Prophylaxe von Herpes-Infektionen, insbesondere Herpes Simplex-Infektionen und Herpes-, insbesondere Herpes Simplex-positiven Patienten zur Unterdrückung der Rekurrenz (Suppressionstherapie)

### In vivo-Wirkung

### Tiere:

6 Wochen alte weibliche Mäuse, Stamm BALB/cABom, wurden von einem kommerziellen Züchter (Bomholtgard Breeding and Research Centre Ltd.) bezogen.

### Infektion:

Die Tiere wurden in einem dichten Glasgefäß mit Diethylether (Merck) anästhesiert. 50 µl einer Verdünnung des Virusstocks (Infektionsdosis 5x10⁴ Pfu) wurden mit einer Eppendorfpipette in die Nase der anästhesierten Tiere eingebracht. Diese Infektionsdosis führt bei 90-100 % der Tiere durch eine generalisierte Infektion mit prominenten respiratorischen und zentralnervösen Symptomen im Mittel zwischen 5 und 8 Tagen zum Tode.

### Behandlung und Auswertung:

6 Stunden nach Infektion wurden die Tiere mit Dosen von 0,1-100 mg/kg Körpermasse 3 mal täglich 7.00 Uhr, 14.00 Uhr und 19 Uhr über einen Zeitraum von 5 Tagen behandelt. Die Substanzen wurden in DMSO vorgelöst und in Tylose/PBS(Hoechst) resuspendiert (Endkonzentration 1,5 % DMSO, 0,5 % Tylose in PBS).

Nach der letzten Applikation wurden die Tiere weiter beobachtet und die Todeszeitpunkte festgestellt.

Ein Vergleich der Überlebenskurven erbrachte für die Verbindung des Beispiels 57 z.B eine ED₅₀ von etwa 0,7 mg/kg für HSV-2, wobei ED₅₀ bedeutet, das bei dieser Dosis 50 % der Tiere überleben.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe und Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend, sind um den angegebenen Dosierungsspielraum zu erreichen.

Die Formulierungen werden beispielsweise hergestellt durch Verstrecken der Wirkstoffe mit Lösemitteln und/oder Trägerstoffe, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch, insbesondere perlingual oder intravenös.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 20 mg/kg, vorzugsweise etwa 0,01 bis 10 mg/kg Körpergewicht zur Erzielung wirksamer Ergebnisse zu verabreichen, und bei oraler Applikation beträgt die Dosierung etwa 0,01 bis 30 mg/kg, vorzugsweise 0,1 bis 20 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Gegebenenfalls kann es sinnvoll sein, die erfindungsgemäßen Verbindungen mit anderen Wirkstoffen insbesondere antiviralen Wirkstoffen zu kombinieren.

### Ausgangsverbindungen

### Beispiel I

### 2-Chlor-4-methyl-1,3-thiazol-5-sulfonylchlorid

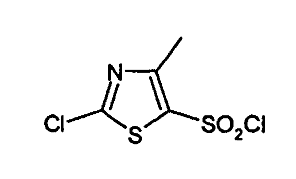

150 g (1,12 mol) 2-Chlor-4-methyl-1,3-thiazol werden bei Raumtemperatur zu einer Lösung von 331 g (2.81 mmol) Thionylchlorid in 653 g (5,61 mmol) Chlorsulfonsäure zugetropft. Die Lösung wird 48 h zum Rückfluß erhitzt. Anschließend wird die Mischung auf 3 l Eiswasser gegeben und mit 4 x 400 ml Dichlormethan extrahiert. Die vereinigten organischen Phasen werden mit 2,.5 l Wasser gewaschen, über Natriumsulfat getrocknet und eingeengt. Nach Destillation des Rohproduktes werden 233,7 g Produkt in Form eines Öls erhalten. (Sdp 87-96°C, 0,7 mbar, GC 98,1%, Ausbeute 89,6%).

### Beispiel II

### 2-Chlor-4-methyl-1,3-thiazol-5-sulfonamid

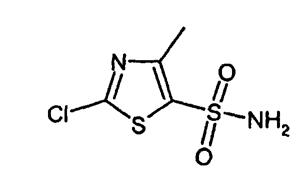

Zu einer Lösung aus 208 g (95 %ig, 0.9 mol) 2-Chlor-4-methyl-1,3-thiazol-5-sulfonylchlorid in 1000 ml Tetrahydrofuran werden 117,7 g (1,8 mol) einer 26 %igen wässrigen Ammoniaklösung bei -10C° zugetropft. Man läßt 2 h ohne weitere Kühlung nachrühren und engt anschließend den Reaktionsansatz am Rotationverdampfer ein. Das Rohprodukt wird ohne weitere Reinigung in die nächste Stufe eingesetzt.

### Beispiel III

### 4-Methyl-2-(methylamino)-1,3-thiazol-5-sulfonamid

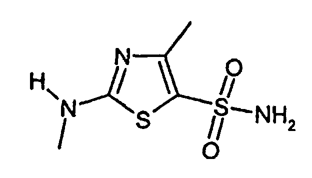

144 g (0,576 mol) 2-Chlor-4-methyl-1,3-thiazol-5-sulfonamid werden bei Raumtemperatur in 600 ml Acetonitril vorgelegt und 147 g (1,9 mol) einer 40 %igen wässrigen Methylamin-Lösung bei Raumtemperatur zudosiert. Der Reaktionsansatz wird 6 h bei 50°C nachgerührt und anschließend am Rotationsverdampfer eingeengt. Der Rückstand wird mit Wasser versetzt, abgesaugt und getrocknet.
Ausbeute: 78 g (66%)
Fp.: 194°C

### Beispiel IV

### 2-Fluorphenylboronsäure

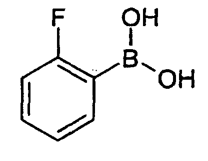

155 g (0,86 mol) 2-Fluorbrombenzol werden unter Argon in 732 ml absolutem Tetrahydrofuran vorgelegt und bei -78 °C langsam mit 600 ml 1,6 M *n*-Butyllithium in Hexan versetzt. Dann wird 2 h bei -78 °C nachgerührt. Anschließend werden bei -78°C 298 ml (1.28 mol) Borsäuretrimethylester zugetropft. Nach 1 h wird die Kühlung entfernt und das Reaktionsgemisch über Nacht gerührt und auf Raumtemperatur erwärmt. Zur Aufarbeitung wird der Ansatz mit 346 ml gesättigter Ammoniumchloridlösung bei 0 °C versetzt, der pH mit 1N HCl auf 6 eingestellt und die wässrige Phase 3 mal mit je 250 ml Methylenchlorid extrahiert. Die vereinigten organischen Phasen werden mit gesättigter Natriumchloridlösung gewaschen und mit Magnesiumsulfat getrocknet. Man erhält Beispiel IV in Form eines beigen Feststoffs.
Ausbeute: 60.0 g (48 %)
MS (EI, m/z): 140 (80%, [M]⁺), 96 (100 %, [C₆H₅F]⁺)

### Beispiel V

### (2'-Fluor[1,1'-biphenyl]-4-yl)essigsäuremethylester

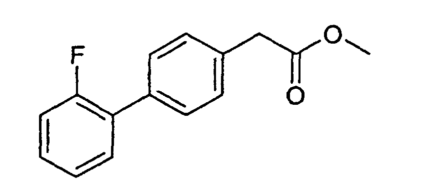

47,6 g (0,21 mol) 4-Bromphenylessigsäuremethylester werden unter Argon in 400 ml absolutem Tetrahydrofuran vorgelegt und bei Raumtemperatur mit 320 ml 1M Sodalösung und 40 g (0,28 mol) 2-Fluorphenylboronsäure versetzt. Nach Zugabe von 7,0 g (0,01 mol) Bis(triphenylphosphan)palladium (II) chlorid wird 18 h unter Rückfluß erhitzt. Nach dem Abkühlen wird mit 500 ml Wasser verdünnt und dreimal mit je 300 ml Essigsäureethylester extrahiert. Die vereinigten organischen Phasen werden mit je 400 ml gesättigter Ammoniumchloridlösung, Wasser und gesättigter Natriumchloridlösung gewaschen, über Magnesiumsulfat getrocknet und am Vakuum vom Solvenz befreit. Man erhält Beispiel V nach Kieselgelfiltration (Petrolether/Essigsäureethylester 10:1) als farbloses Öl.
Ausbeute: 46.0 g (94 %)
¹H-NMR (500 MHz, CDCl₃, δ /ppm): 3.71 (s, 2H), 3.76 (s, 3H), 7.18 - 7.46 (m, 4H), 7.40 (d, J = 8.3 Hz; 2H), 7.56 (dd, J₁ = 8.3 Hz, J₂ = 1.7 Hz; 2H).

### Beispiel VI

### (2'-Fluor[1,1'-biphenyl]-4-yl)essigsäure

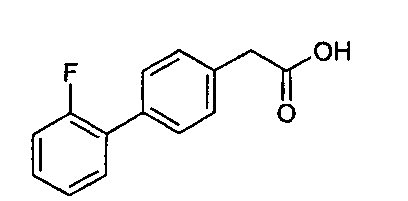

26.5 g (0,11 mol) (2'-Fluoro[1,1'-biphenyl]-4-yl)essigsäuremethylester werden in 50 ml Ethanol vorgelegt und bei Raumtemperatur mit einer Lösung von 12,8 g (0,19 mol) Kaliumhydroxidplätzchen in 25 ml Wasser versetzt. Dann wird 4 h unter Rückfluß erhitzt. Nach dem Abkühlen wird das Rohgemisch am Vakuum eingeengt, der Rückstand in 100 ml Wasser gelöst und mit konz. Salzsäure sauer gestellt. Der Niederschlag wird abfiltriert, mehrfach mit Wasser gewaschen und der Feststoff getrocknet. Man erhält Beispiel VI in Form weißer Kristalle.
Ausbeute: 22,7 g (91 %)
Fp.: 102°C
¹H-NMR (500 MHz, CDCl₃, δ /ppm): 3.74 (s, 2H), 7.18 - 7.47 (m, 4H), 7.41 (d, J = 8.2 Hz; 2H), 7.57 (dd, J₁ = 8.2 Hz, J₂ = 1.6 Hz; 2H).

### Beispiel VII

### [4-(2-Pyridinyl)phenyl]essigsäuremethylester

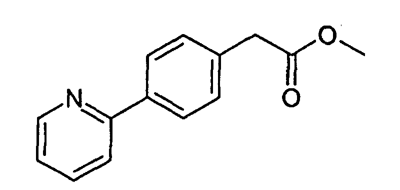

7,85 g (34,3 mmol) 4-Bromphenylessigsäuremethylester werden unter Argon in 95 ml Toluol vorgelegt und bei Raumtemperatur mit 7,97 g (61,7 mmol) Diisopropylethylamin, 9,50 g (37,7 mmol) 2-Trimethylstannylpyridin und 0,4 g (0,3 mmol) Tetrakis(triphenylphosphan)palladium (0) versetzt. Anschließend wird 18 h unter Rückfluß erhitzt. Nach dem Abkühlen wird mit je 100 ml 1N Salzsäure und gesättigter Natriumhydrogencarbonatlösung gewaschen. Die organische Phase wurde verworfen. Die saure und die basische Wasserphase wurden neutral gestellt, mit jeweils 100 ml Dichlormethan extrahiert und die vereinigten organischen Phasen über Magnesiumsulfat getrocknet und am Vakuum vom Solvenz befreit. Man erhält Beispiel VII nach Kieselgelchromatographie (Toluol/Essigsäureethylester Gradient 5:1 - 1:1) als farbloses Öl.
Ausbeute: 1,6 g (19 %)
¹H-NMR (400 MHz, d⁶-DMSO, δ /ppm): 3.64 (s, 3H), 3.76 (s, 2H), 7.33 - 7.40 (m, 1H), 7.39 (d, J = 8.2 Hz; 2 H), 7.86 - 7.90 (m, 1H), 7.96 (d, J = 8.0 Hz; 1H), 8.05 (d, J = 8.2 Hz; 2 H), 8,67 (d, J = 4.2 Hz, breit; 1H).

### Beispiel VIII

### [4-(2-Pyridinyl)phenyl]essigsäure

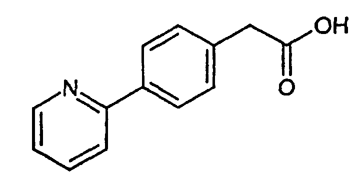

700 mg (3,11 mol) [4-(2-Pyridinyl)phenyl]essigsäuremethylester werden in 5 ml Tetrahydrofuran vorgelegt und bei Raumtemperatur mit 6,2 ml einer 1M Kaliumhydroxidlösung in Wasser versetzt. Dann wird 18 h bei Raumtemperatur gerührt, anschließend das Solvenz am Vakuum weitgehend entfernt, der Rückstand in 10 ml Wasser aufgenommen und mit 2N Salzsäure ein pH-Wert von ca. 5 eingestellt. Zweimalige Extraction der wässrigen Phase mit je 10 ml Dichlormethan lieferte nach Trocknen der vereinigten organischen Phasen über Magnesiumsulfat und Entfernen des Solvenz am Vakuum die Verbindung von Beispiel VIII in Form eines Feststoffs.
Ausbeute: 300 mg (46 %)
¹H-NMR (400 MHz, d⁶-DMSO, δ /ppm): 3.76 (s, 2H), 7.45 - 7.51 (m, 1H), 7.50 (d, J = 8.3 Hz; 2H), 8.00 (td, J₁ = 7.7 Hz, J₂ = 1.9Hz; 1H), 8.07 (d, J = 7.9 Hz; 1 H), 8.15 (d, J = 8.3 Hz; 2H), 8,78 (dt, J₁ = 4.0 Hz, J₂ = 0.9Hz; 1H).

### Herstellungsbeispiele

### Beispiel 15

### N-[5-(aminosulfonyl)-4-methyl-1,3-thiazol-2-yl]-2-[1,1'-biphenyl]-4-yl-N-methylacetamid

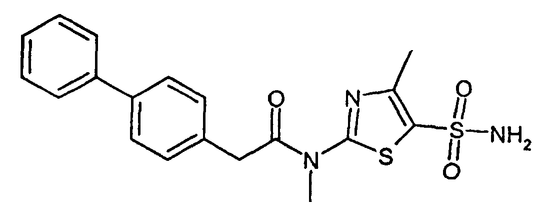

138,2 mg (0,65 mmol) 4-Biphenylessigsäure und 99,7 mg (0,65 mmol) 1-Hydroxy-1Hbenzotriazol Hydrat werden in 5 ml Dimethylformamid bei Raumtemperatur vorgelegt. 150 mg (0,72 mmol) 2-Methylamino-4-methyl-1,3-thiazol-5-sulfonamid und 138,7 mg (0,72 mmol) N'-(3-Dimethylaminopropyl)-N-ethylcarbodiimid Hydrochlorid werden zugegeben und der Ansatz 72 h bei Raumtemperatur gerührt. Anschließend wird der Reaktionsansatz abgesaugt und der Rückstand aus 2-Propanol umkristallisiert. Man erhält einen weißen Feststoff.
Ausbeute: 240 mg (83,0 %)
Fp.: 191°C
¹H-NMR (300 MHz, d⁶-DMSO, δ /ppm): 2.47 (s, 3H; teilweise unter DMSO Signal), 3.71 (s, 3H), 4.20 (s, 2H), 7.32 - 7.70 (m, 11H).

### Beispiel 38

### N-[5-(aminosulfonyl)-4-methyl-1,3-thiazol-2-yl]-N-methyl-2-[4-(2-pyridinyl)phenyl]acetamid

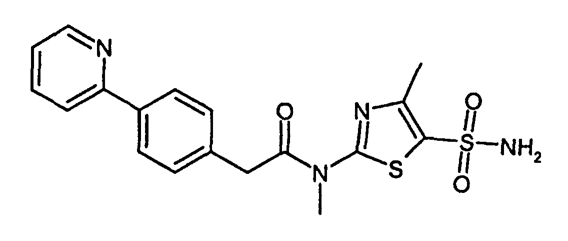

300 mg (1,41 mmol) [4-(2-Pyridinyl)phenyl]essigsäure und 190 mg (1,41 mmol) 1-Hydroxy-1H-benzotriazol Hydrat werden in 4 ml Dimethylformamid bei Raumtemperatur vorgelegt. 307 mg (1,48 mmol) 2-Methylamino-4-methyl-1,3-thiazol-5-sulfonamid und 284 mg (1,48 mmol) N'-(3-Dimethylaminopropyl)-N-ethylcarbodiimid Hydrochlorid werden zugegeben und der Ansatz 18 h bei Raumtemperatur gerührt. Anschließend wird am Vakuum vom Solvenz befreit, der Rückstand in Toluol aufgenommen und das Solvenz erneut am Vakuum entfernt. Der Rückstand wird mit 15 ml Wasser und 3 ml Methanol verrührt, anschließend abfiltriert und das Filtrat mit 20 ml Dichlormethan nachextrahiert. Festoff und Dichlormethanphase werden vereinigt und das Solvenz am Vakuum entfernt. Man erhält die Verbindung von Beispiel 38 in Form eines weißen Feststoffs.
Ausbeute: 440 mg (74,0 %)
Fp.: 188 - 192°C
MS (ESI, m/z): 403 (100%, [M+H]⁺)
¹H-NMR (400 MHz, d⁶-DMSO, δ /ppm): 2.38 (s, 3H; unter DMSO Signal), 3.64 (s, 3H), 4.15 (s, 2H), 7.28 - 7.26 (m, 1H) ,7.32 (d, J = 8 Hz; 2H), 7.58 (s, 2H), 7.82 - 7.96 (m, 2H), 7.98 (d, J = 8.0 Hz; 2H), 8,61 (m; 1H).

### Beispiel 57

### N-[5-(aminosulfonyl)-4-methyl-1,3-thiazol-2-yl]-2-(2'-fluor[1,1'-biphenyl]-4-yl)-N-methylacetamid

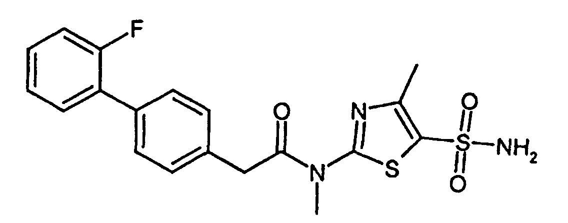

17,33 g (73,3 mmol) (2'-Fluor[1,1'-biphenyl]-4-yl)essigsäure und 9,9 g (73,3 mmol) 1-Hydroxy-1H-benzotriazol Hydrat werden in 600 ml Dimethylformamid bei Raumtemperatur vorgelegt. 16,84 g (81,4 mmol) 2-Methylamino-4-methyl-1,3-thiazol-5-sulfonamid und 15,58 g (81,4 mmol) N'-(3-Dimethylaminopropyl)-N-ethylcarbodiimid Hydrochlorid werden zugegeben und der Ansatz 18 h bei Raumtemperatur gerührt. Das Dimethylformamid wird am Hochvakuum bei 50°C weitgehend entfernt, der Rückstand in 400 ml Dichlormethan aufgenommen und anschließend mit je 350 ml Wasser und 10% Zitronensäurelösung gewaschen. Man erhält nach Trocknen über Magnesiumsulfat und Entfernen des Solvenz am Vakuum die Verbindung von Beispiel 57 in Form eines weißen Feststoffs.
Ausbeute: 23,2 g (76,0 %)
Fp.: 211°C
¹H-NMR (400 MHz, CDCl₃, δ/ppm): 2.58 (s, 3H), 3.73 (s, 3H), 4.07 (s, 2H), 5,91 (s, 2H), 7.13 - 7.46 (m, 4H) ,7.34 (d, J = 8.1 Hz; 2H), 7.56 (d, breit, J = 8.1 Hz; 2H).

### Beispiel 87

### N-[5-(aminosulfonyl)-4-methyl-1,3-thiazol-2-yl]-2-(2',5'-difluor-1,1'-biphenyl-4-yl)-N-methylacetamid

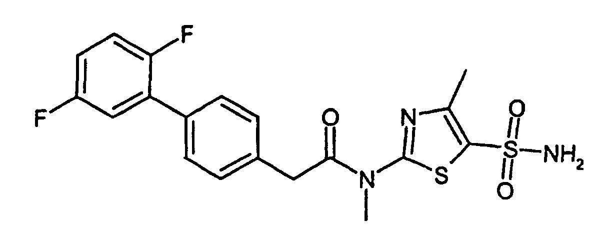

1.00 g (4,0 mmol) (2',5'-Difluor[1,1'-biphenyl]-4-yl)essigsäure und 0,54 g (4.0 mmol) 1-Hydroxy-1H-benzotriazol Hydrat werden in 15 ml Dimethylformamid bei Raumtemperatur vorgelegt. 0,84 g (4.0 mmol) 2-Methylamino-4-methyl-1,3-thiazol-5-sulfonamid und 0,77 g (4.0 mmol) N'-(3-Dimethylaminopropyl)-N-ethylcarbodiimid Hydrochlorid werden zugegeben und der Ansatz 18 h bei Raumtemperatur gerührt. Das Dimethylformamid wird am Hochvakuum bei 50°C weitgehend entfernt, der Rückstand 3 mal mit je 50 ml Wasser ausgerührt, abfiltriert, mit 50 ml Isopropanol verrührt und erneut abfiltriert. Man erhält nach Entfernen des Solvenz am Vakuum die Verbindung von Beispiel 87 in Form eines schwach gelb gefärbten Feststoffs.
Ausbeute: 0,83 g (47,3 %)
Fp.: 184°C
¹H-NMR (400 MHz, DMSO, δ/ppm): 2.49 (s, 3H), 3.71 (s, 3H), 4.24 (s, 2H), 7.22 - 7.46 (m, 3H) ,7.38 (d, J = 8.2 Hz; 2H), 7.56 (d, J = 8.2 Hz; 2H), 7.65 (s, 2H).

### Beispiel 126

### N-[5-(Aminosulfonyl)-4-methyl-1,3-thiazol-2-yl)-N-methyl-2-[4-(1H-pyrazol-1-yl)phenyl]acetamid

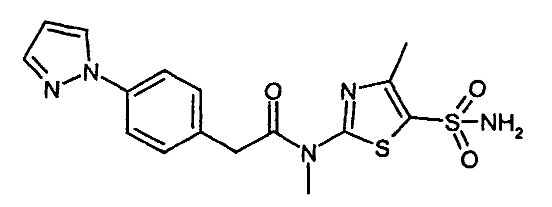

0,100 g (0,48 mmol) 2-Methylamino-4-methyl-1,3-thiazol-5-sulfonamid werden in 10ml N,N-Dimethylformamid gelöst und bei Raumtemperatur mit 0,110 g (0,53 mmol) [4-(1H-Pyrazol-1-yl)phenyl]essigsäure, 0,070 g (0,53 mmol) 1-Hydroxy-1Hbenzotriazol und 0,070 g (0,53 mmol) N,N'-Diisopropylcarbodiimid versetzt. Die Lösung wird über Nacht bei Raumtemperatur gerührt. Anschließend wird die Mischung auf Wasser gegossen und die wässrige Phase dreimal mit Essigsäureethylester extrahiert. Die vereinten organischen Phasen werden mit Natriumsulfat getrocknet und eingeengt. Das Rohprodukt wird an einer präparativen HPLC (RP18-Säule; Laufmittel: Acetonitril-Wasser Gradient) feingereinigt.
Ausbeute: 0,11 g (59 %)
LC-MS (Methode: SMKL-N1-1Low Vol HCl): Retentionszeit: 3,65
MS(ESI): 783 (2Mz+H), 392 (Mz+H).
¹H-NMR (300 MHz, DMSO, δ/ppm): 2.48 (s, 3H), 3.72 (s, 3H), 4.20 (s, 2H), 6.55 (t, J=2Hz; 1H), 7.38 (d, J=7Hz; 2H), 7.65 (s, 2H), 7.75 (d, J=2Hz; 1H), 7.82 (d, J=7Hz; 2H), 8.49 (d, J=2Hz; 1H).

Analog der oben aufgeführten Vorschriften werden die in der folgenden Tabelle aufgeführten Verbindungen hergestellt:

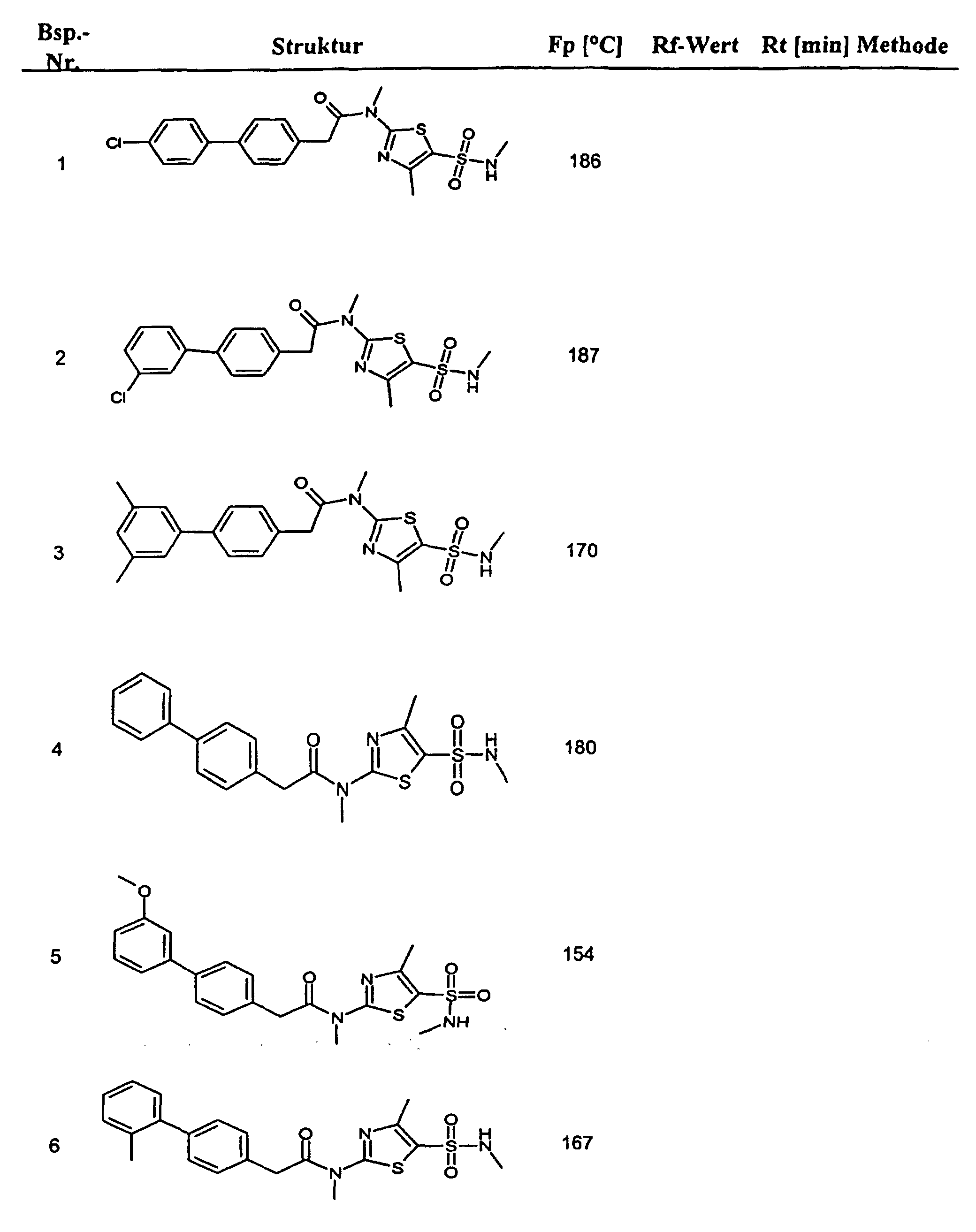

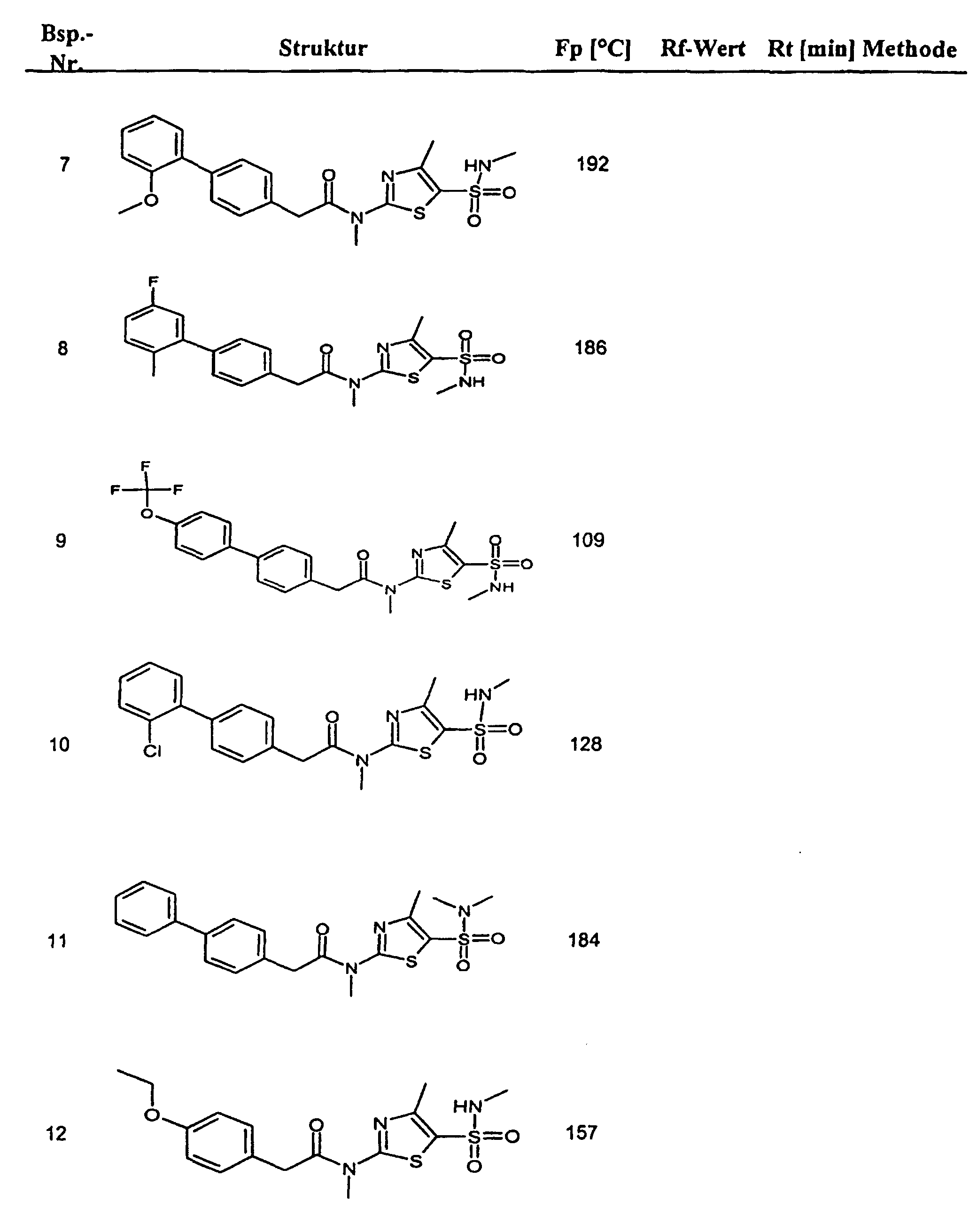

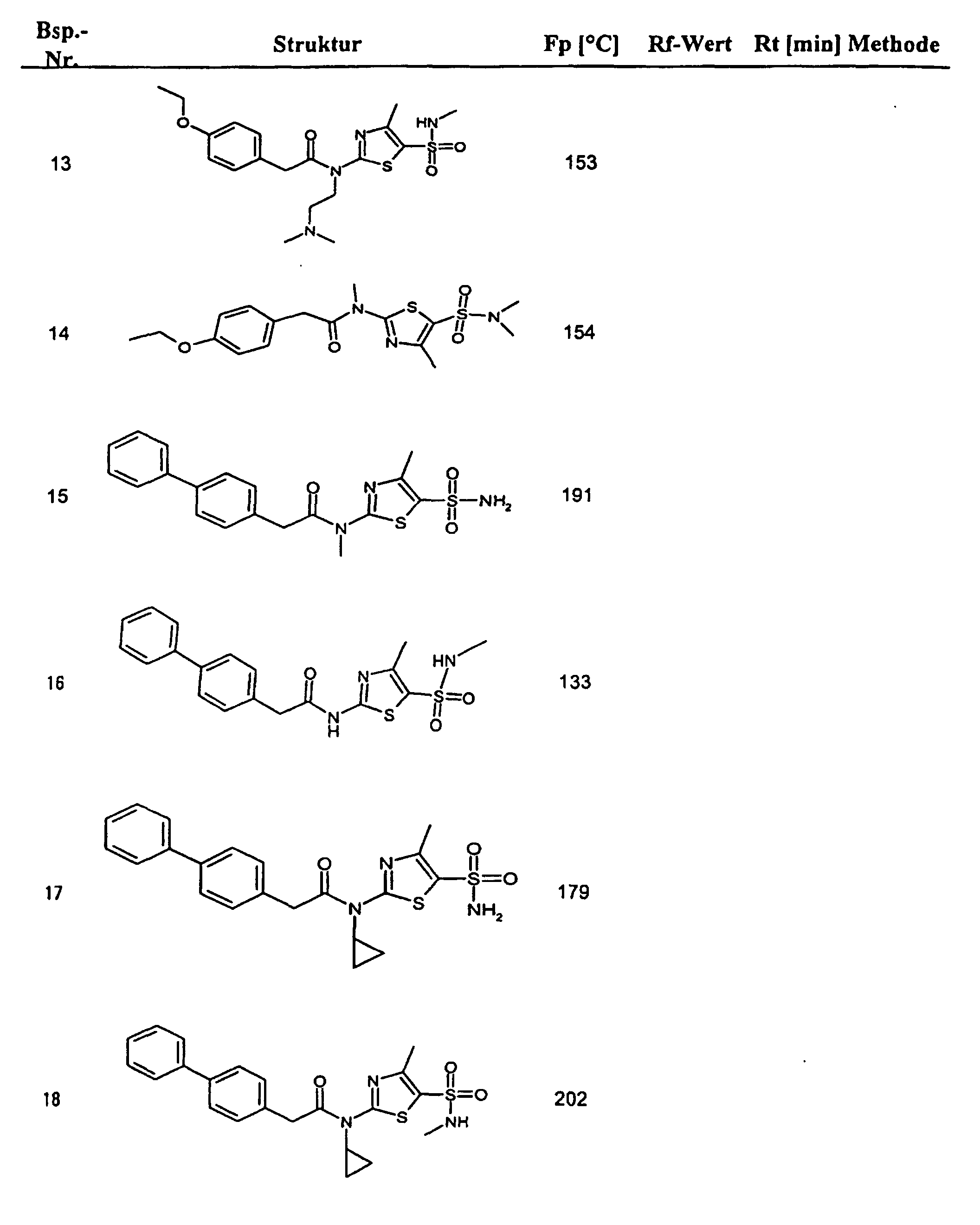

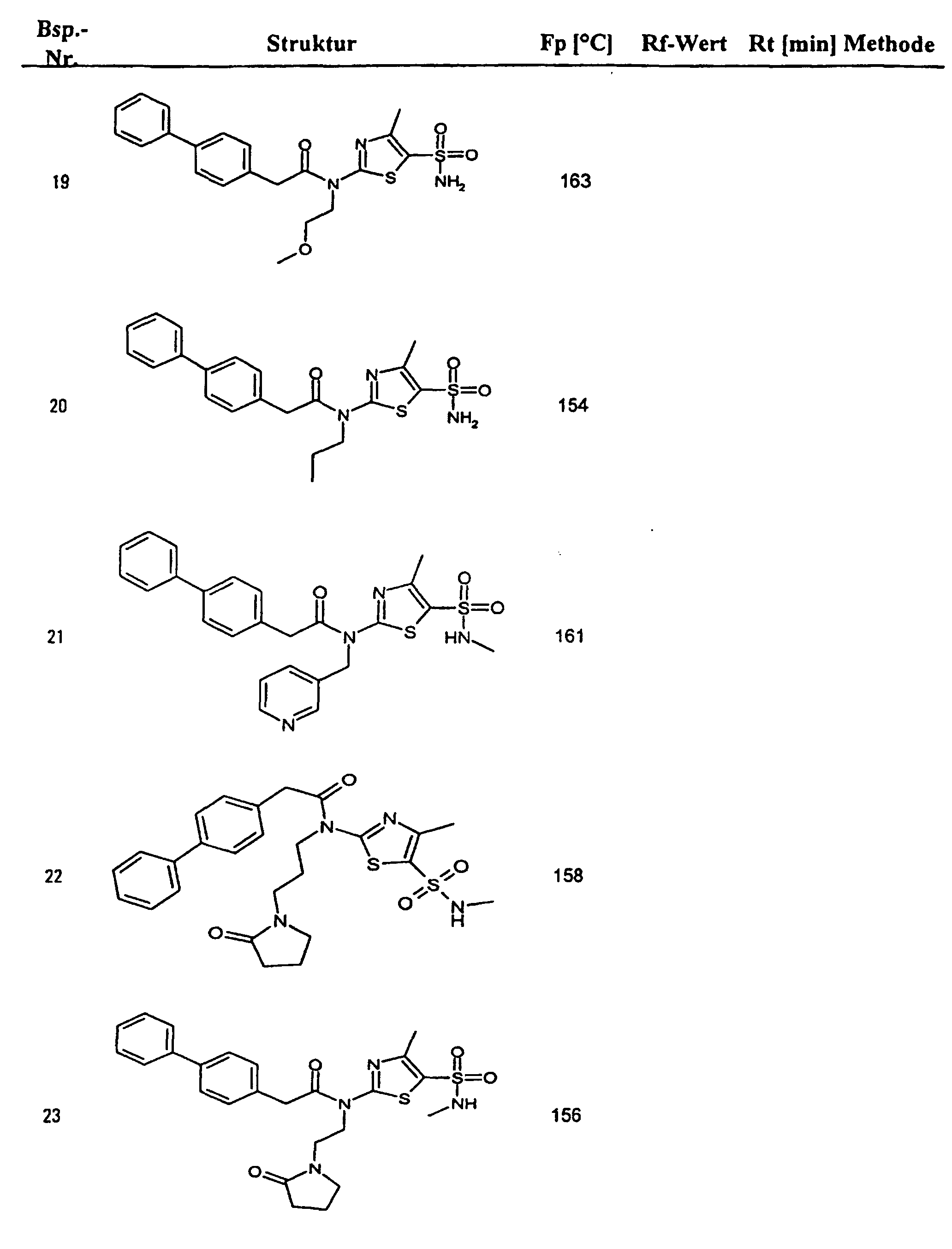

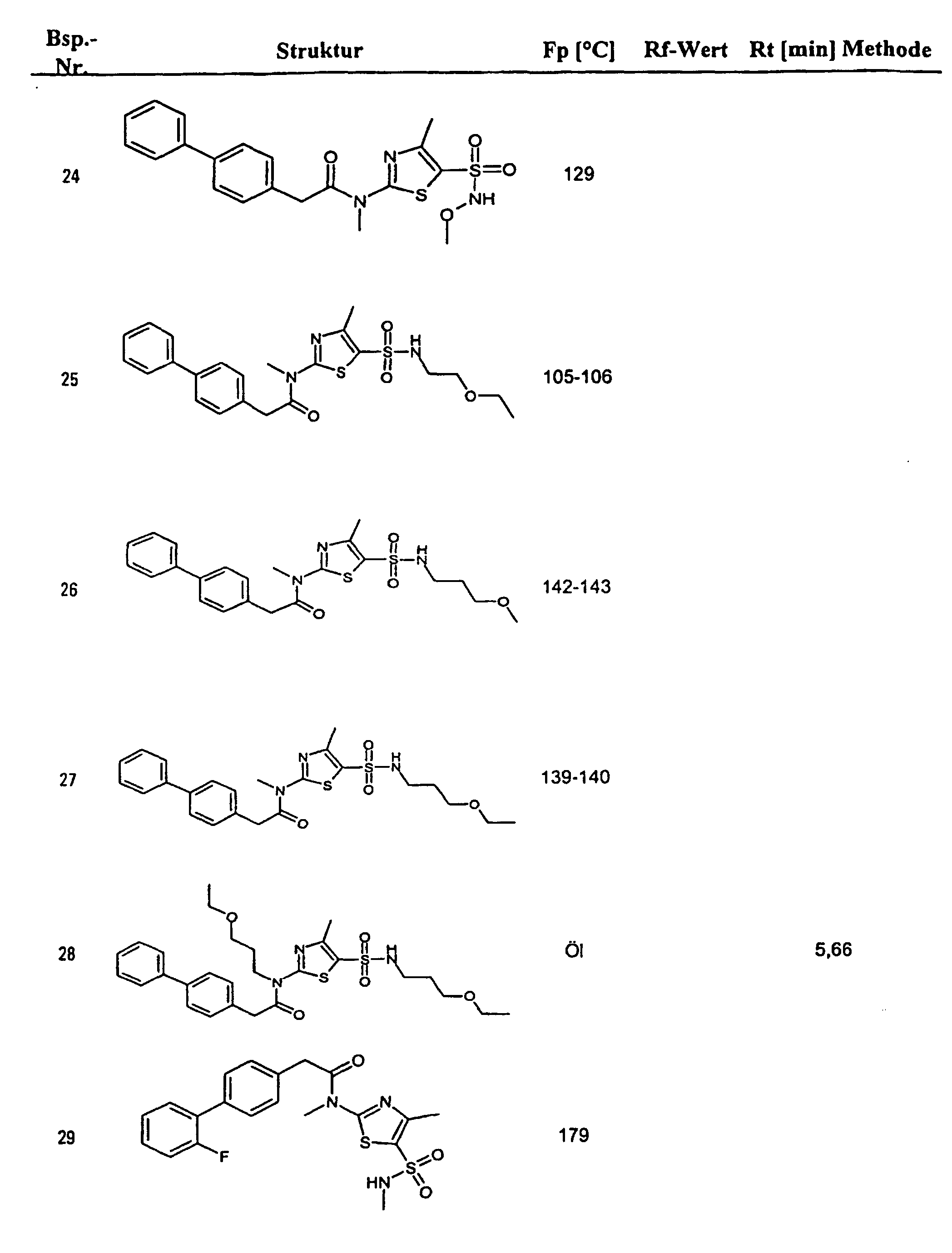

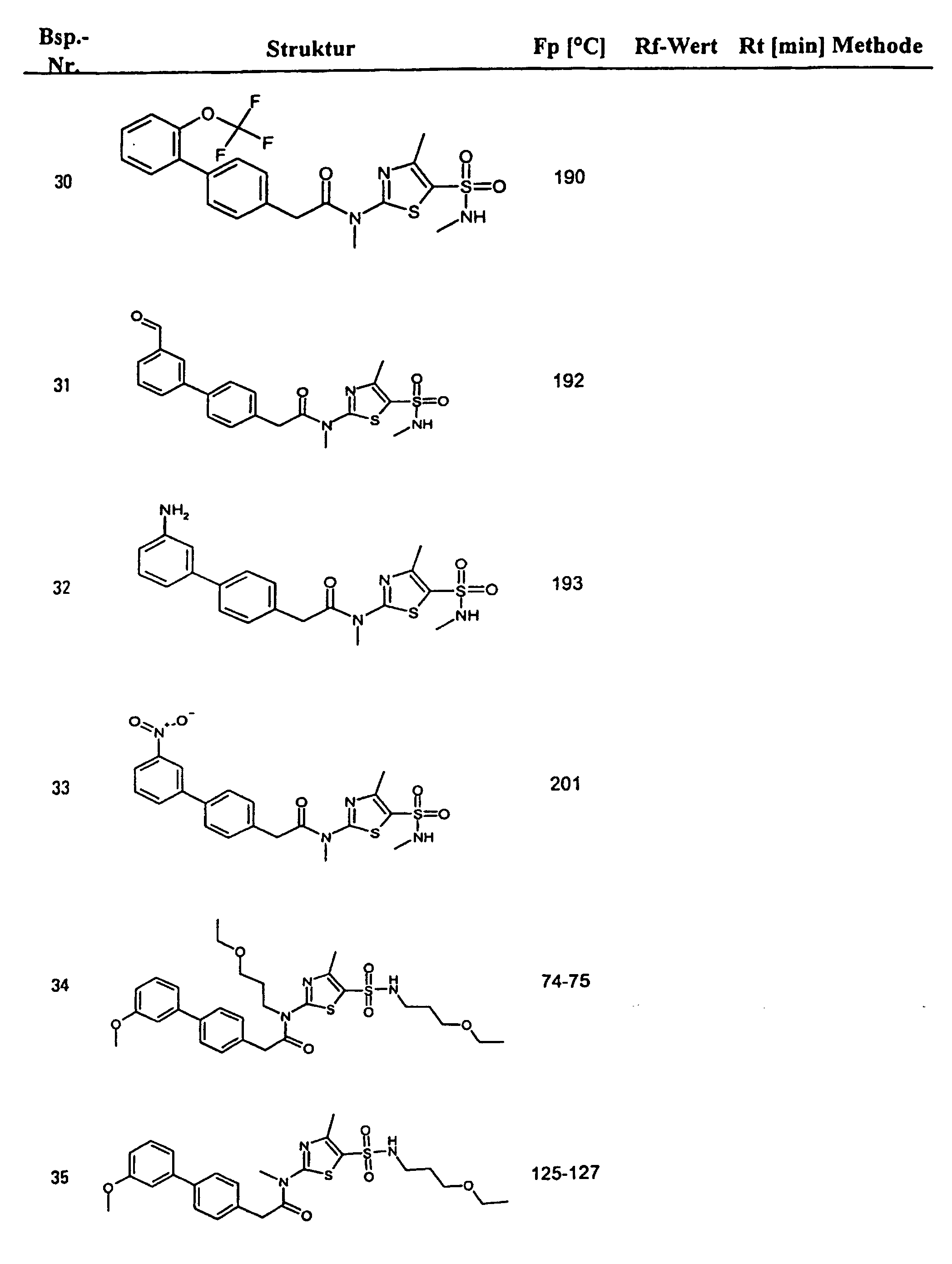

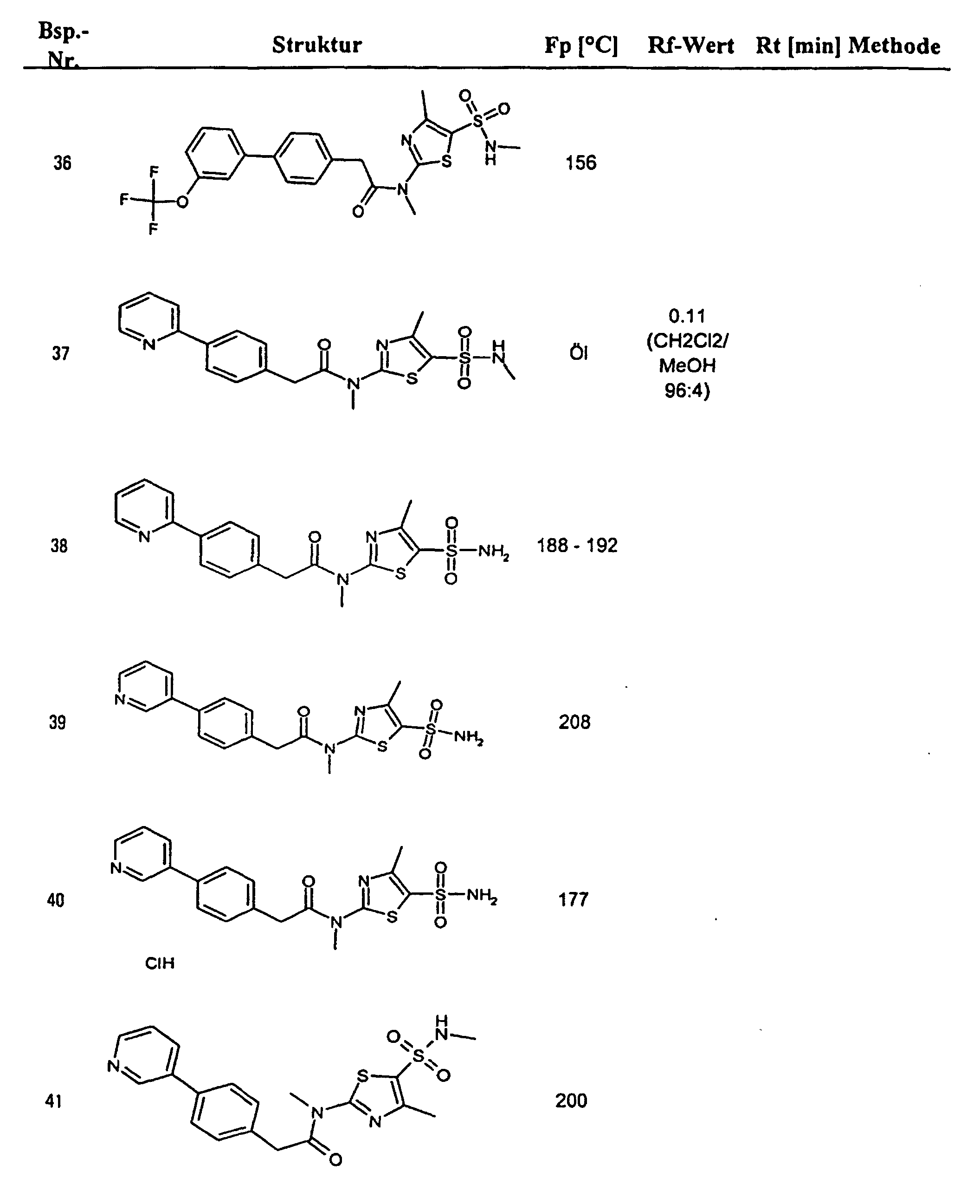

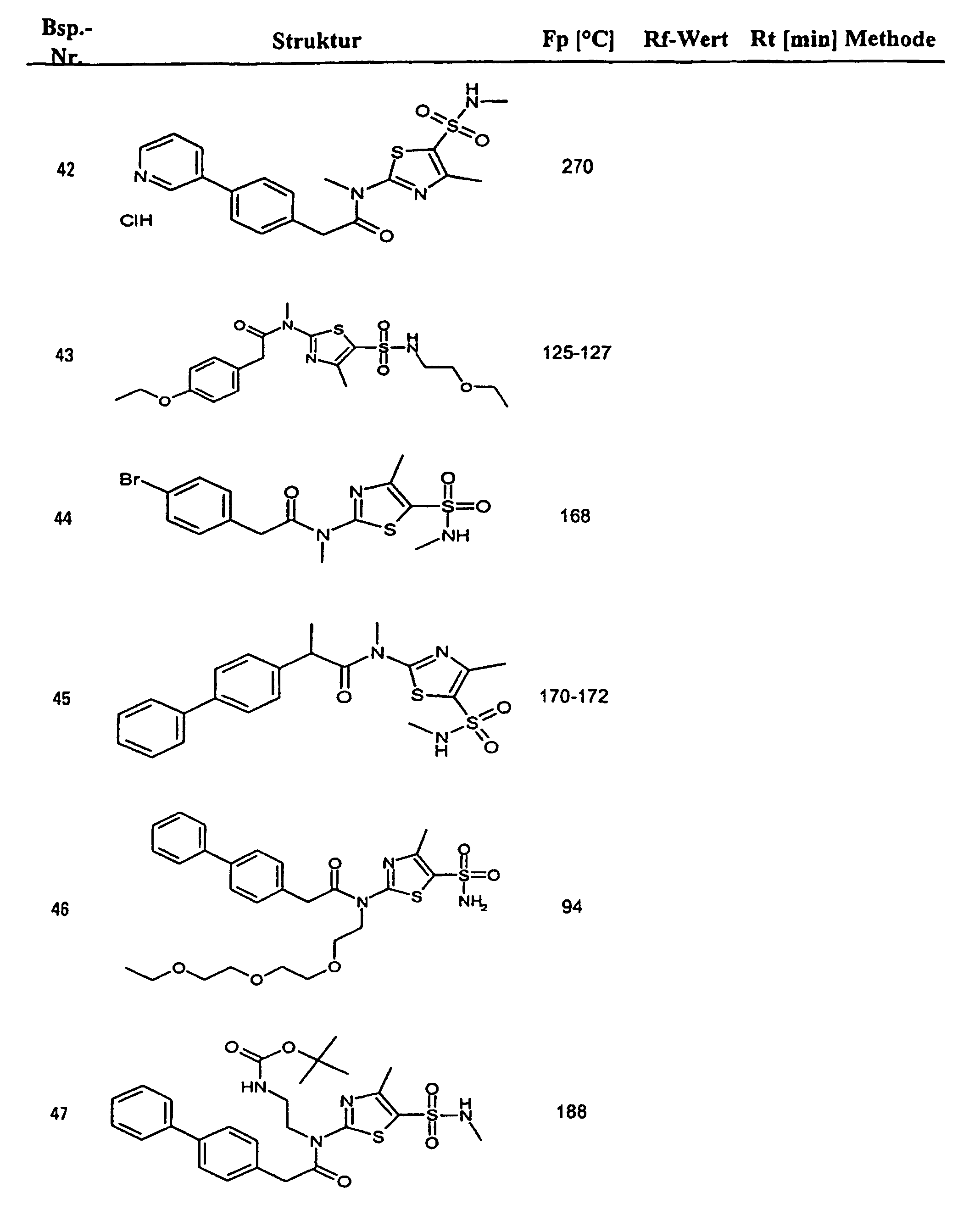

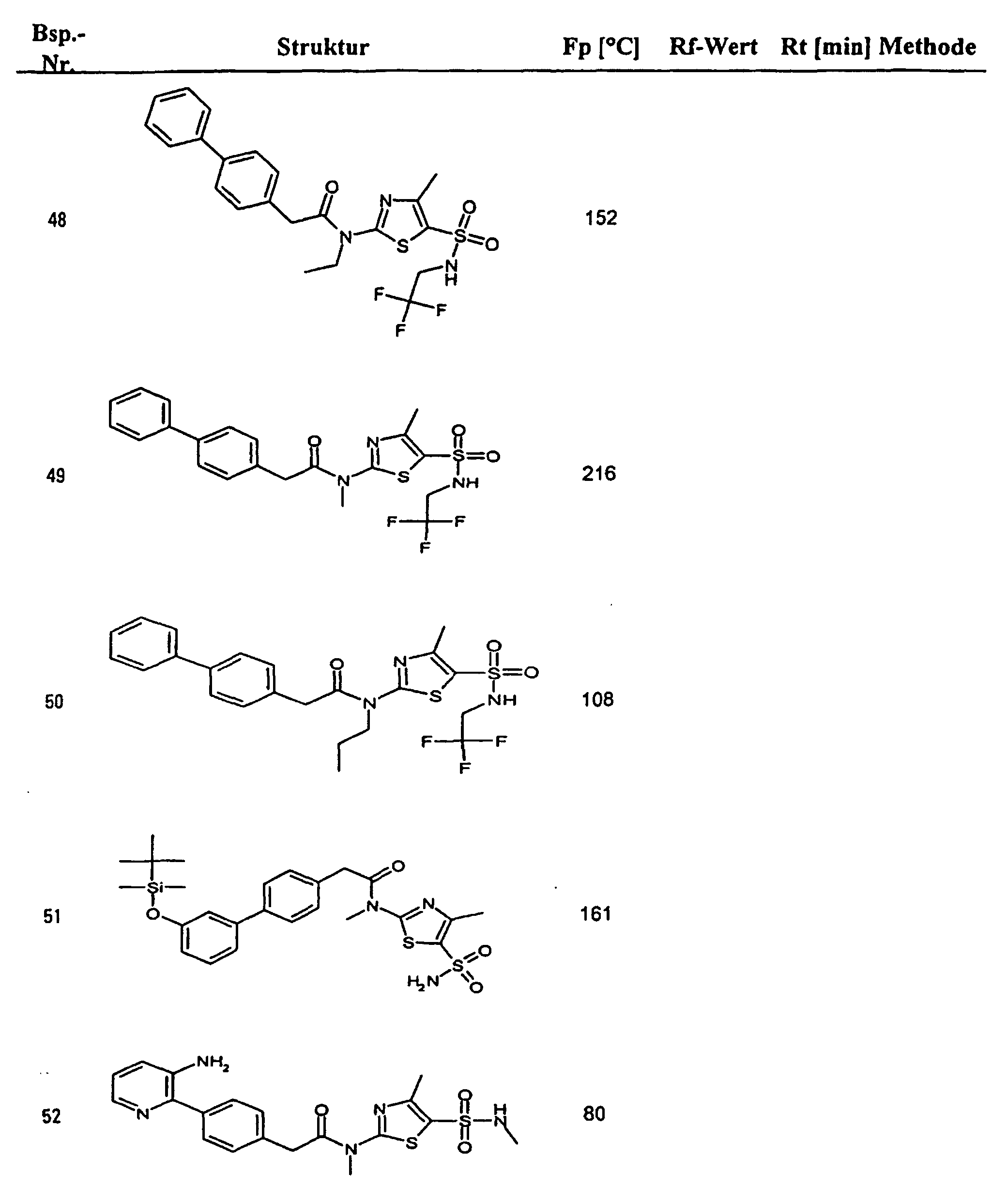

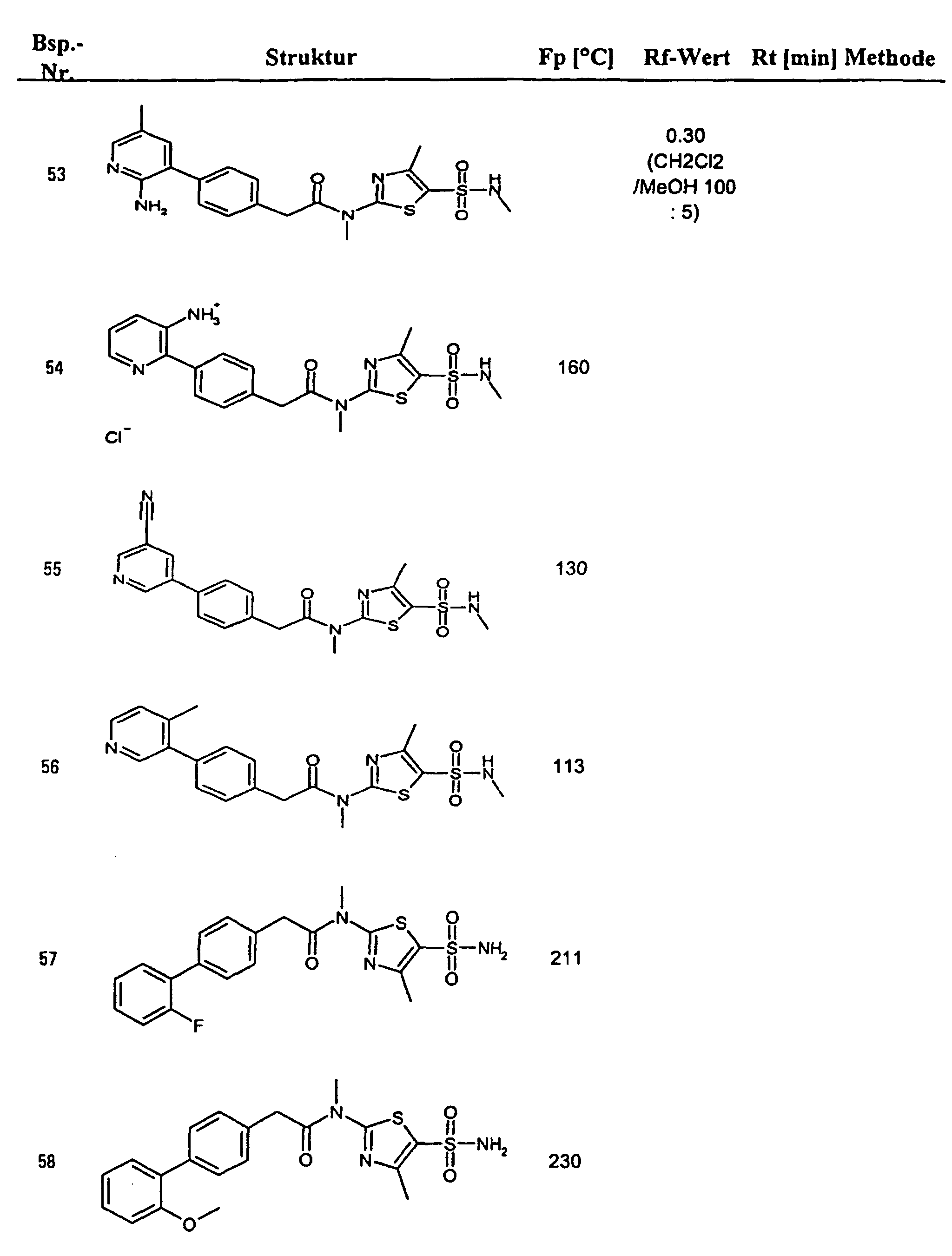

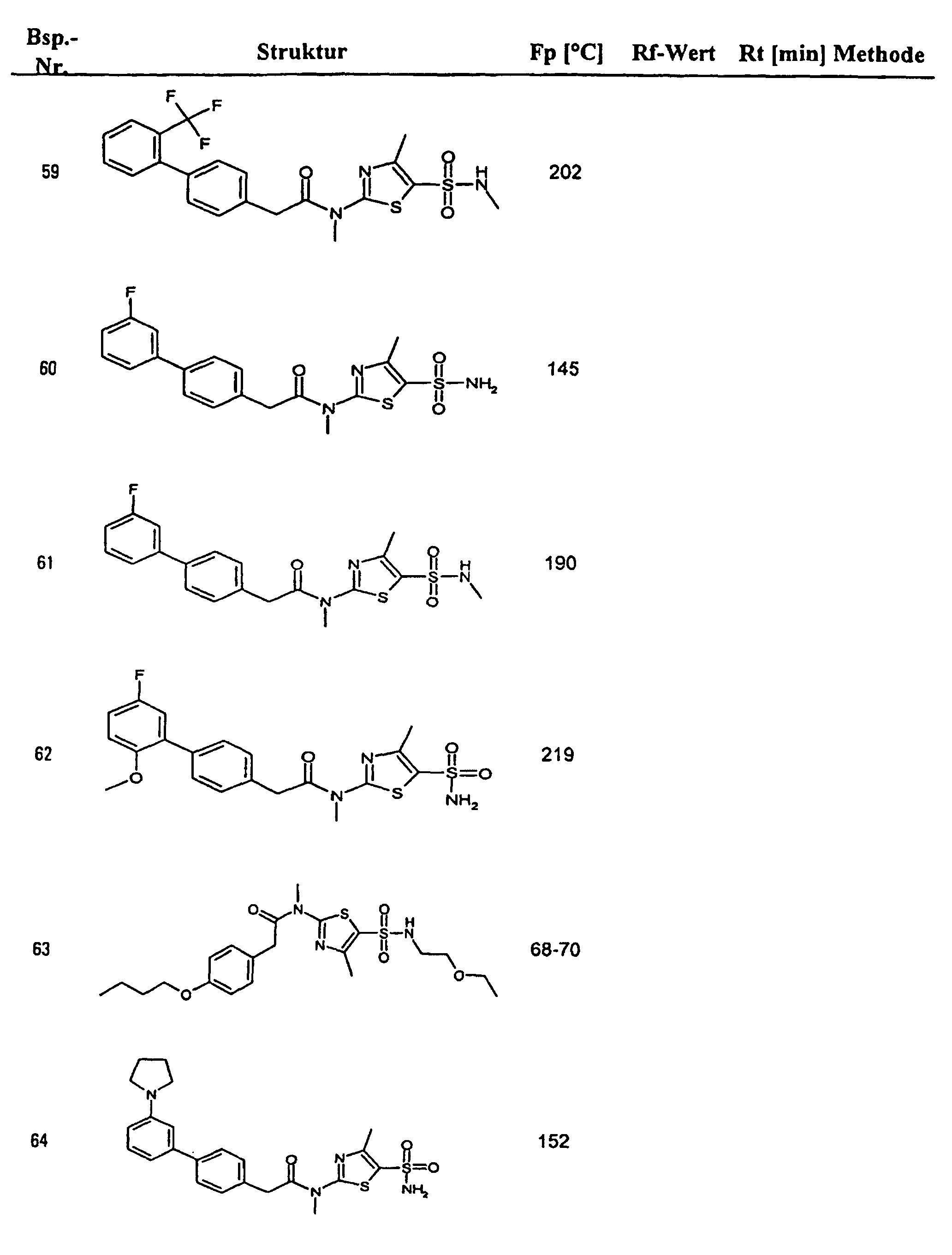

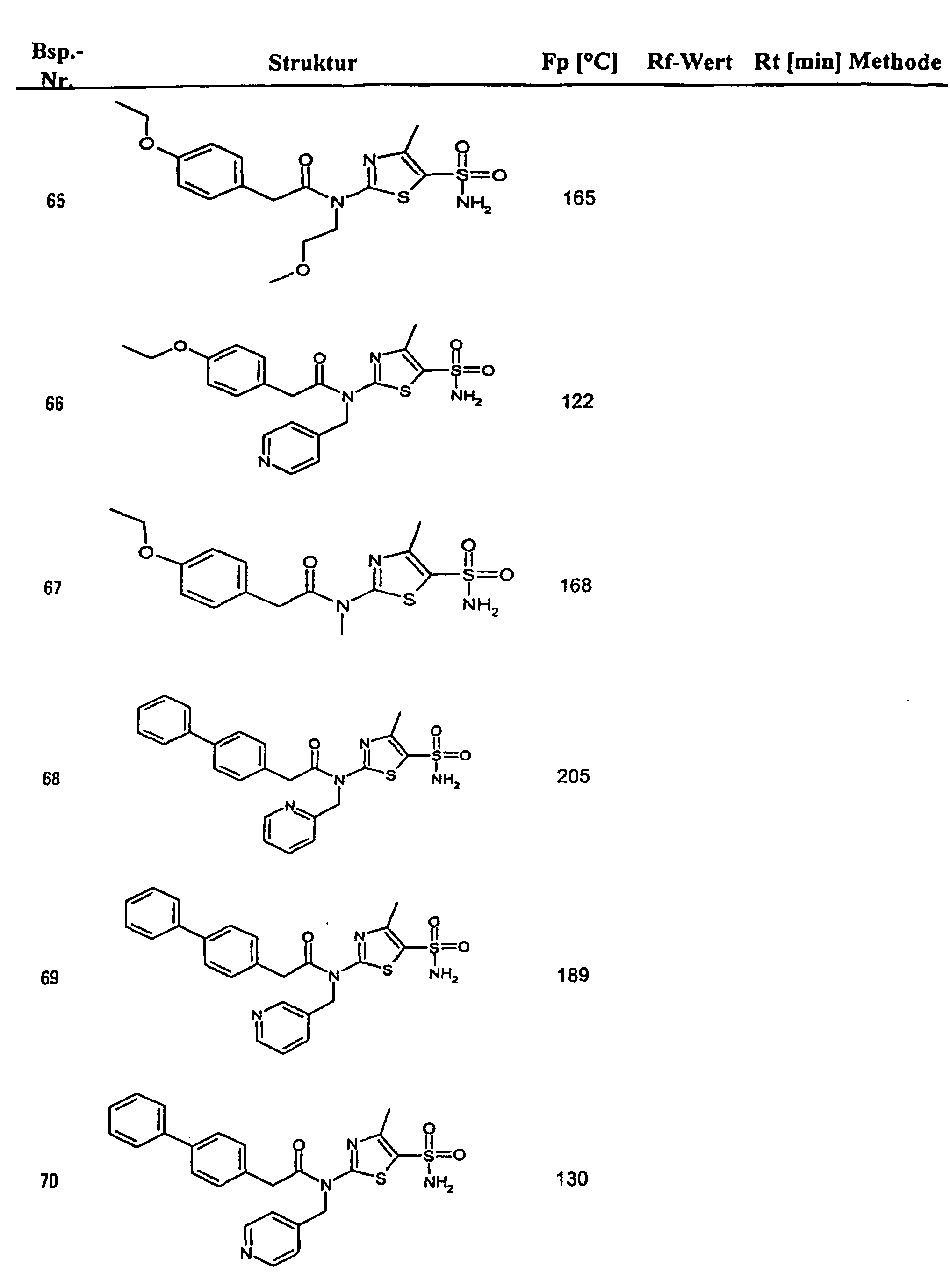

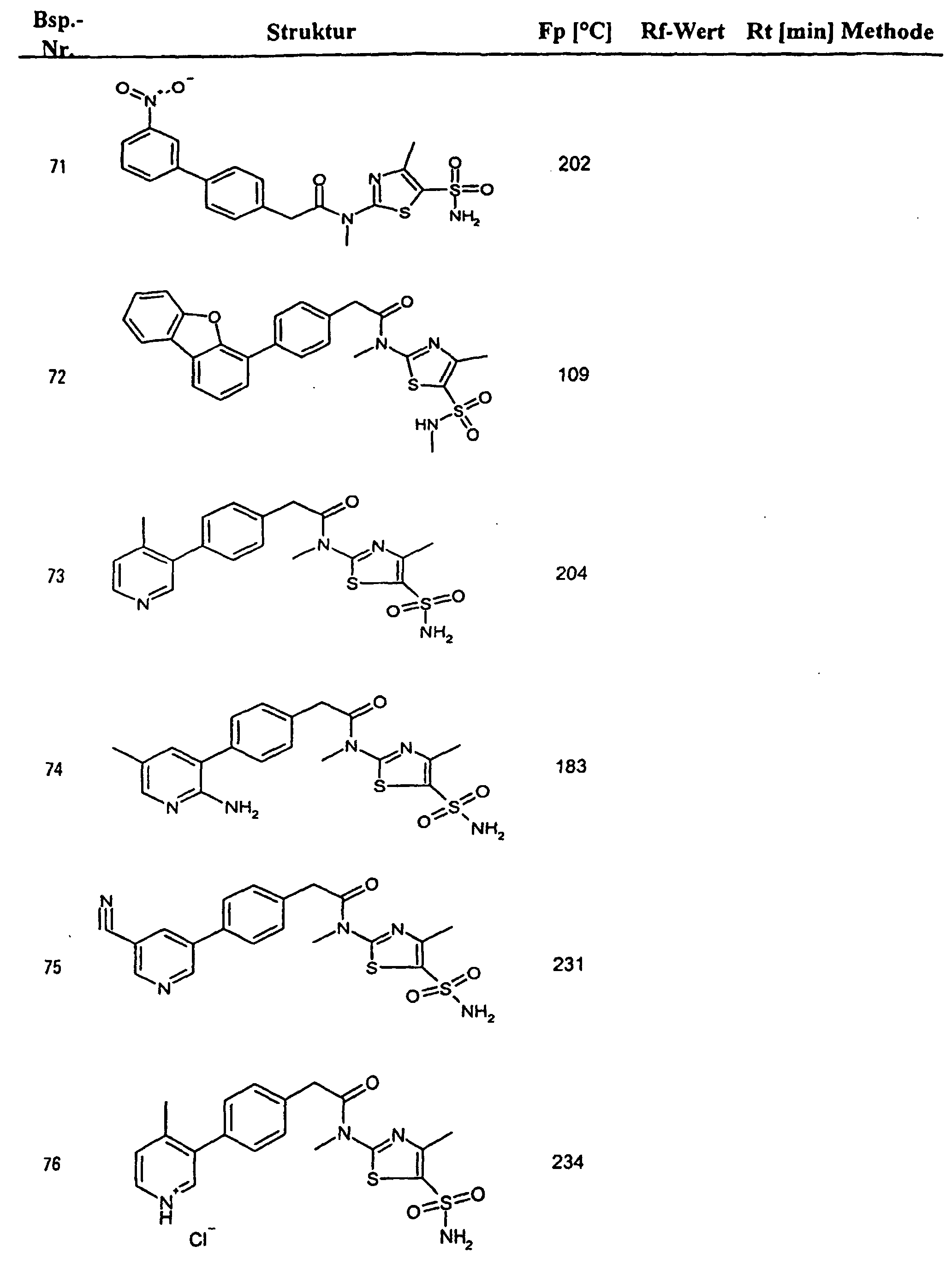

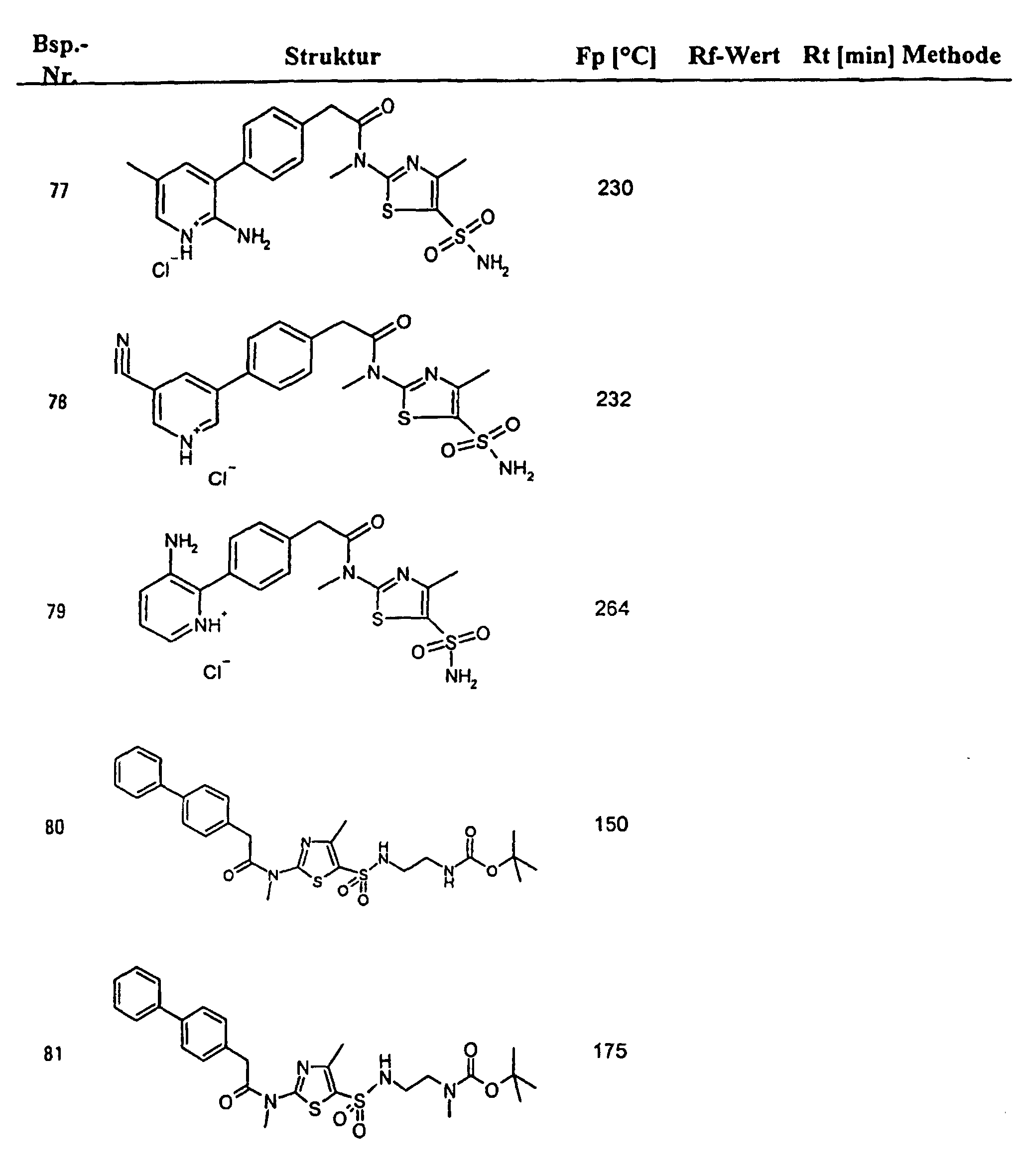

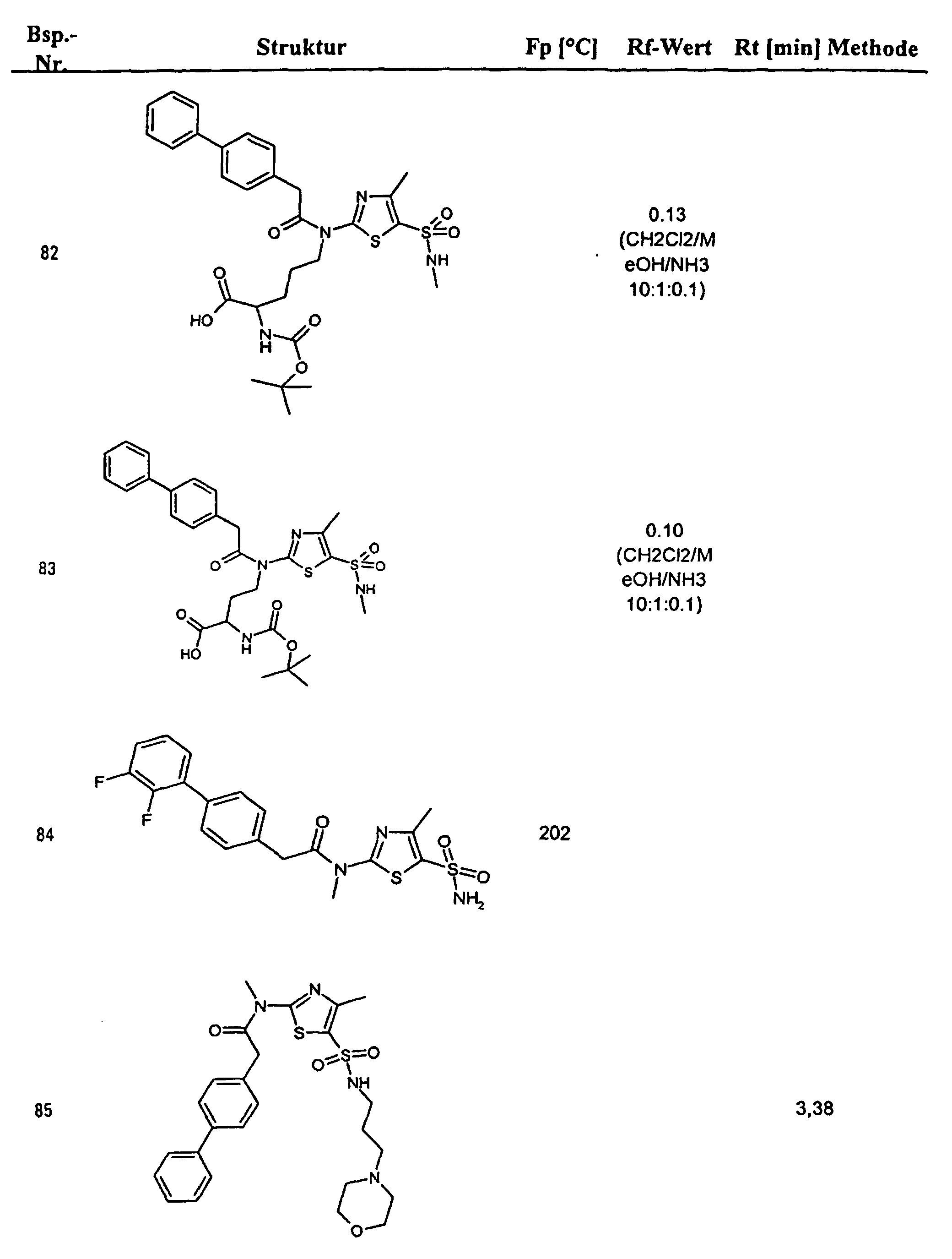

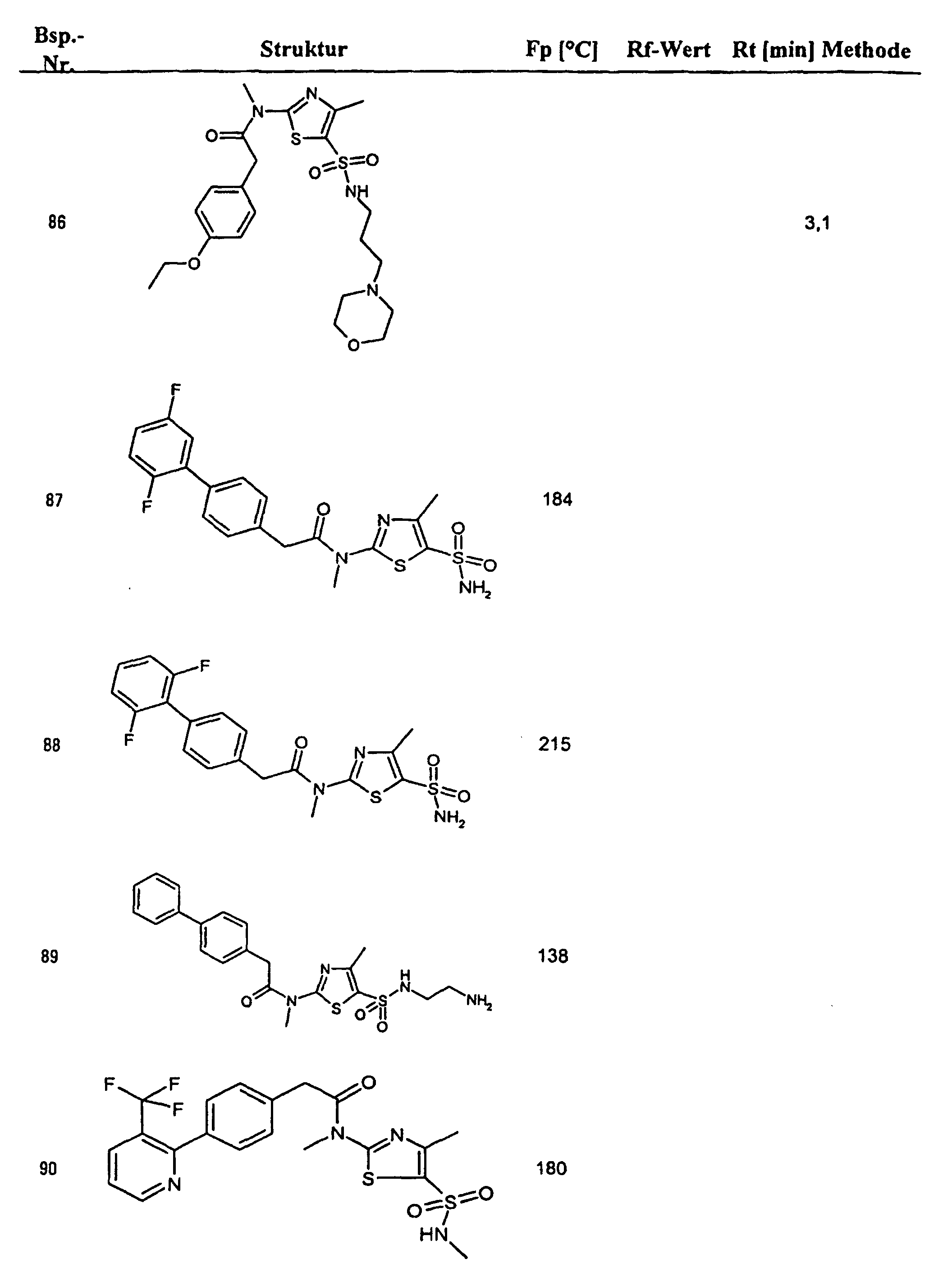

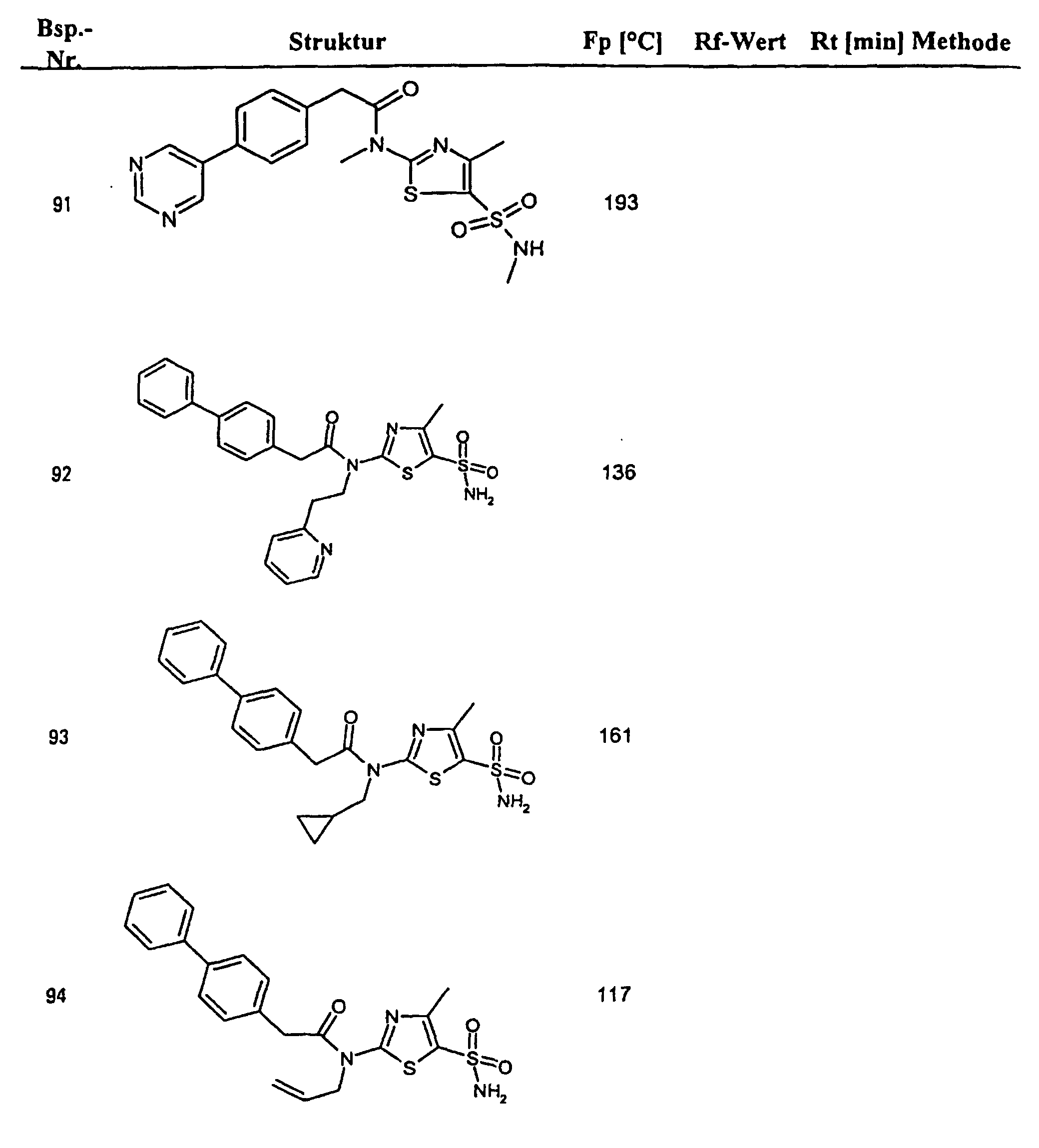

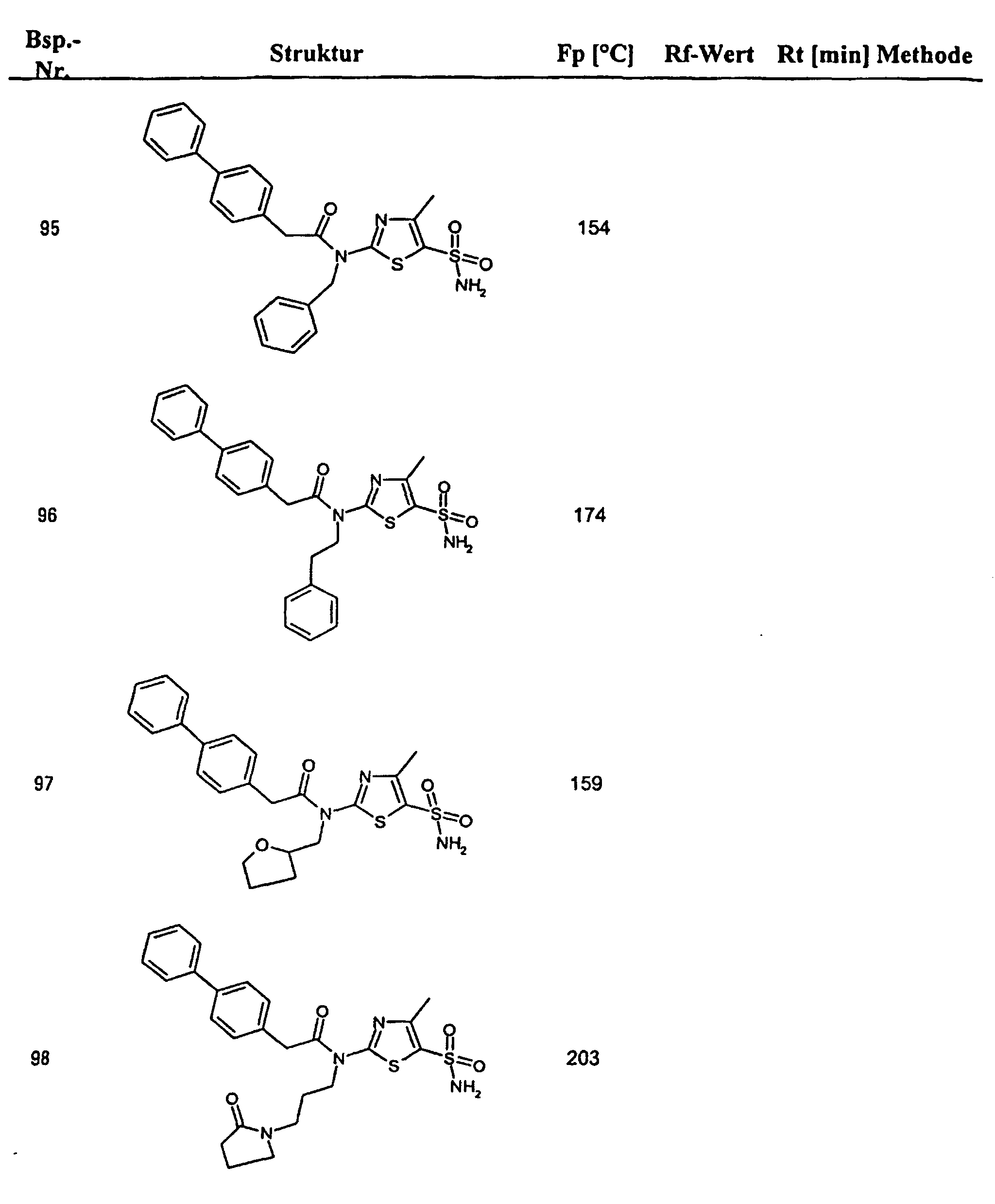

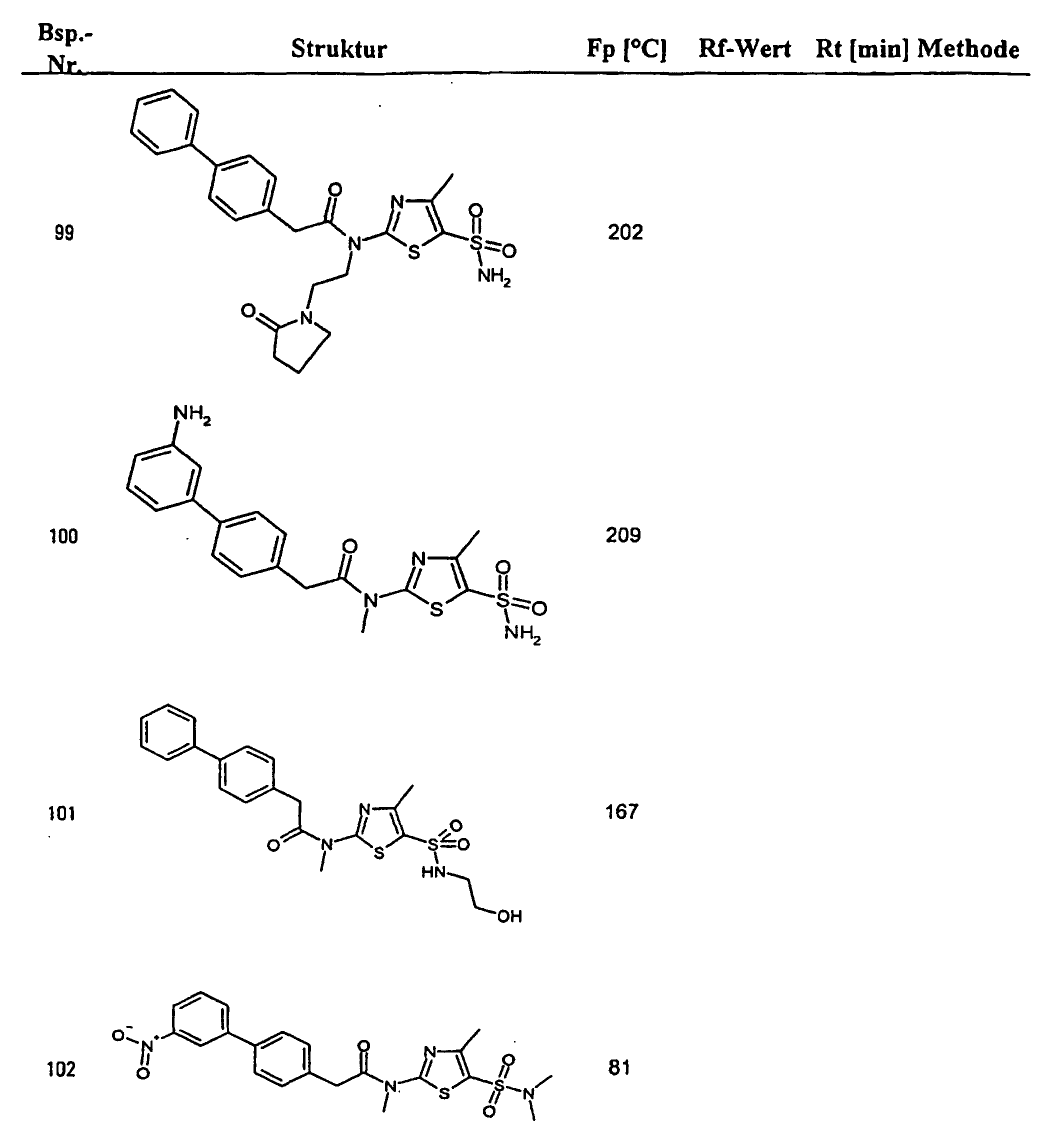

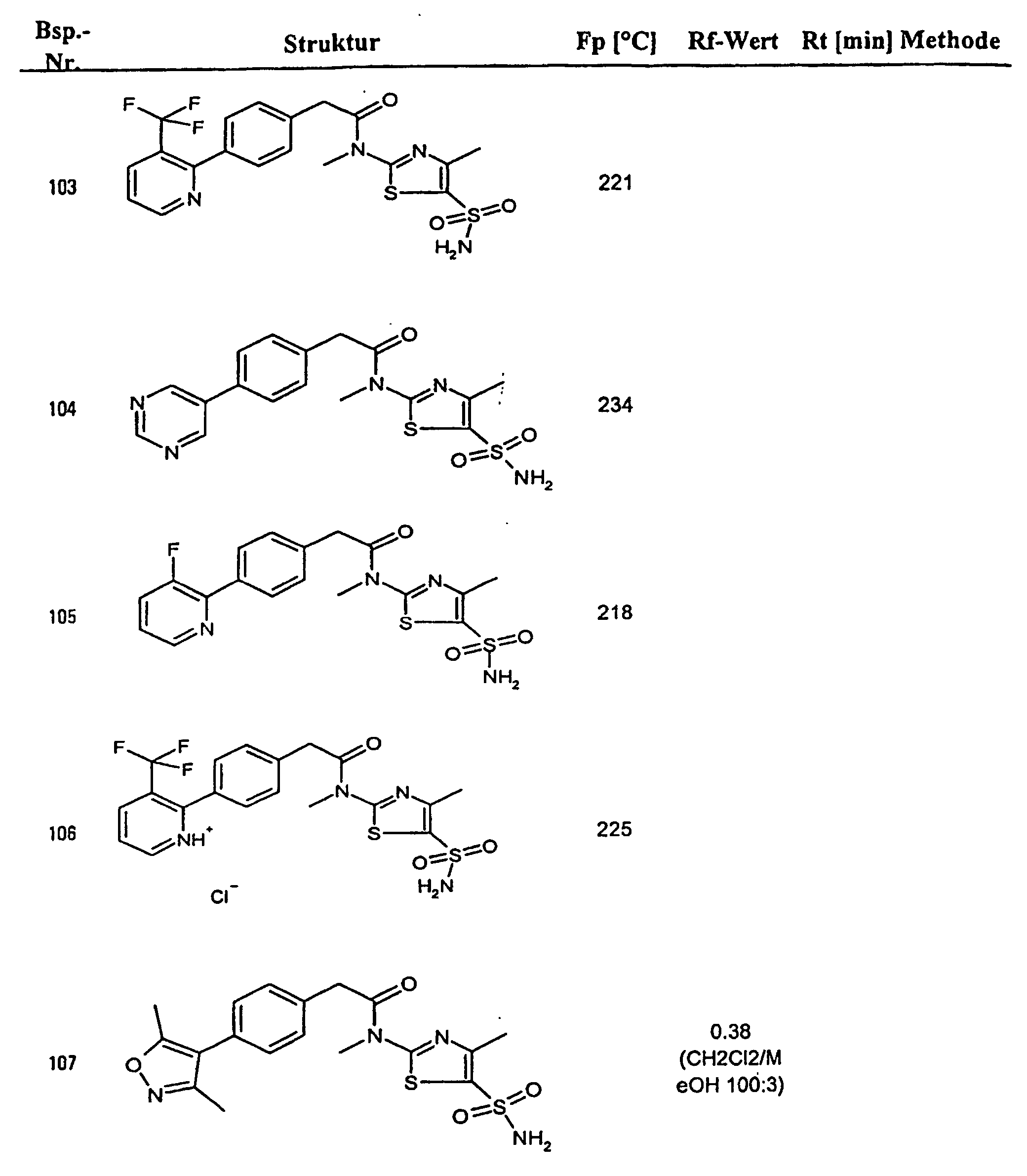

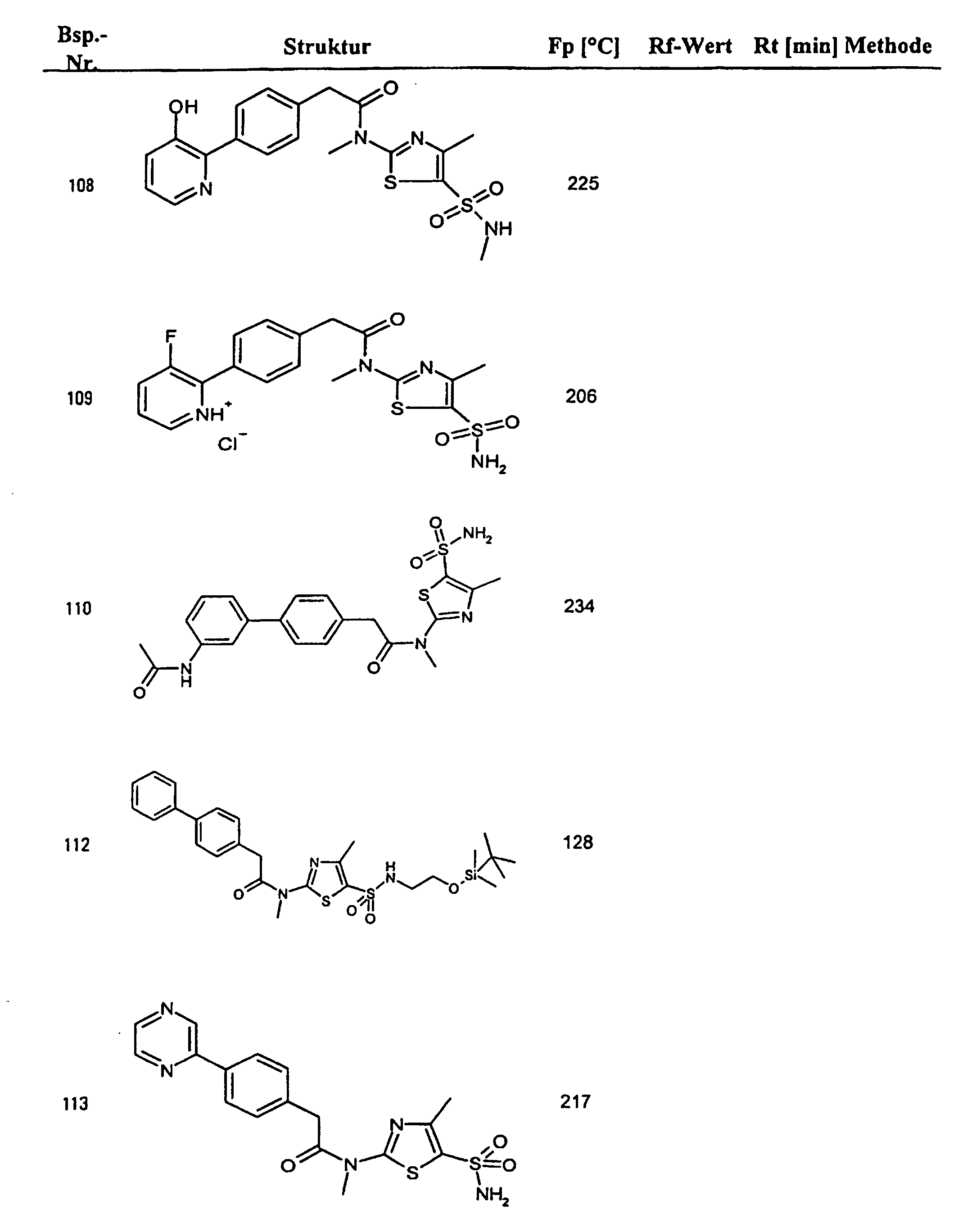

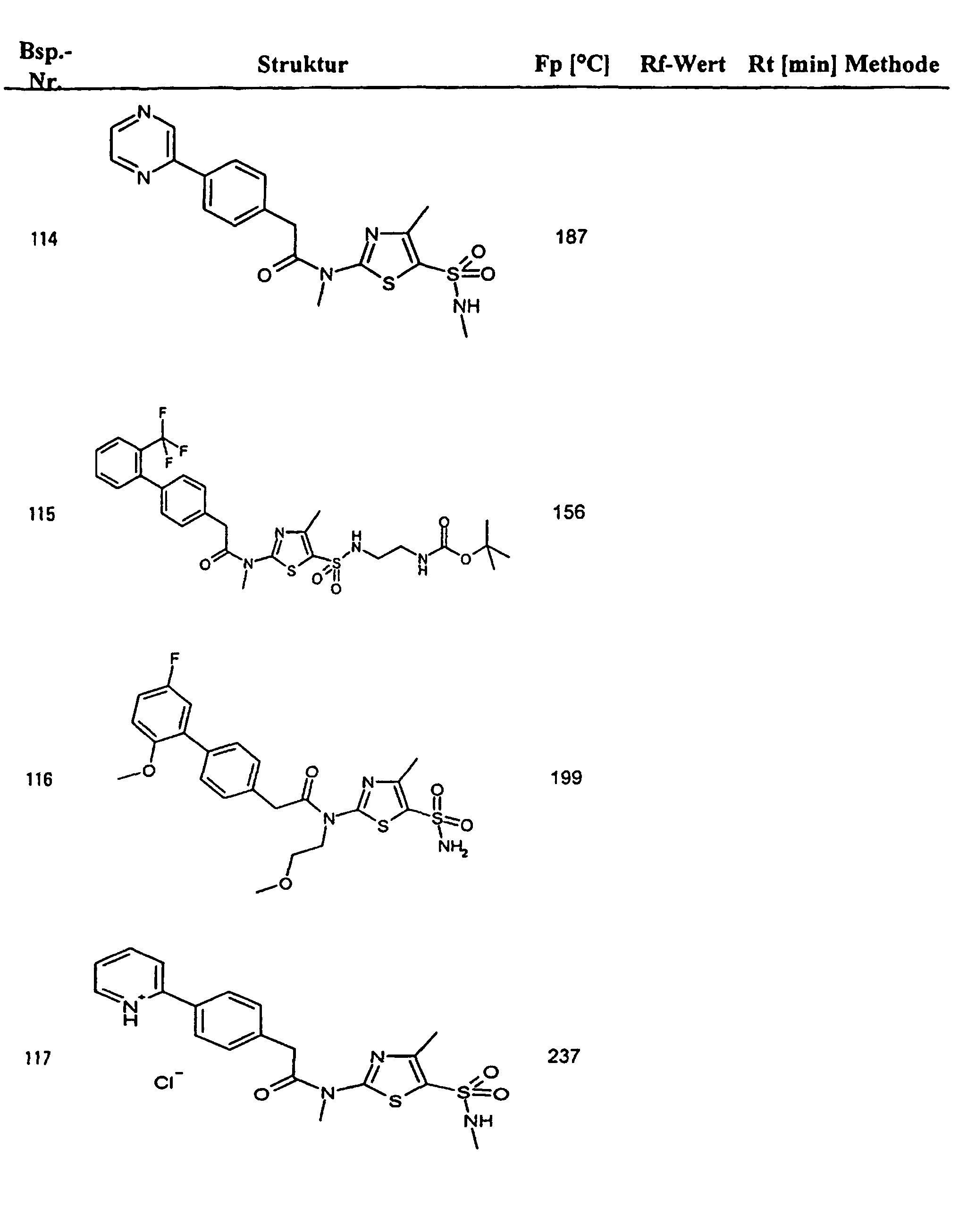

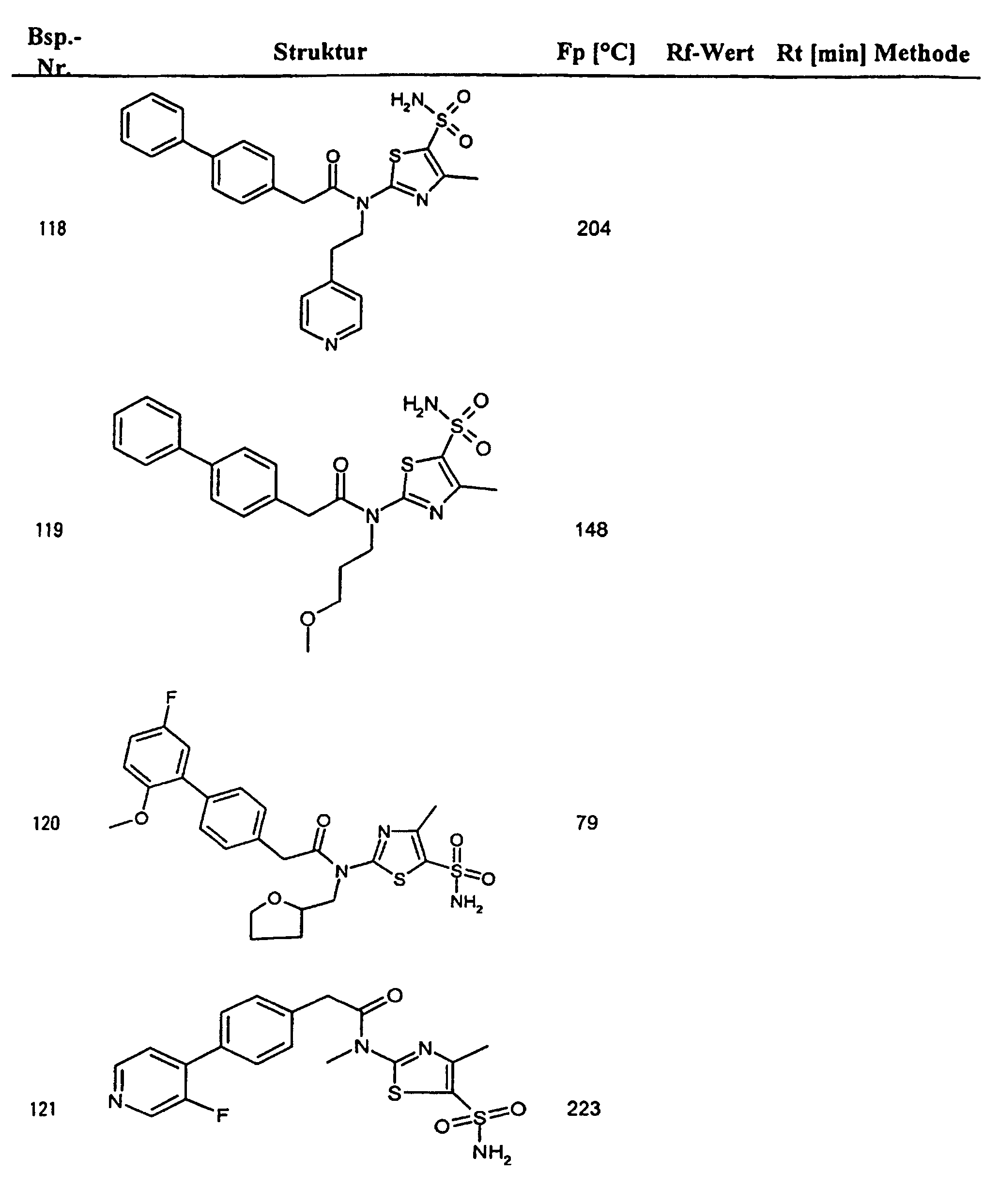

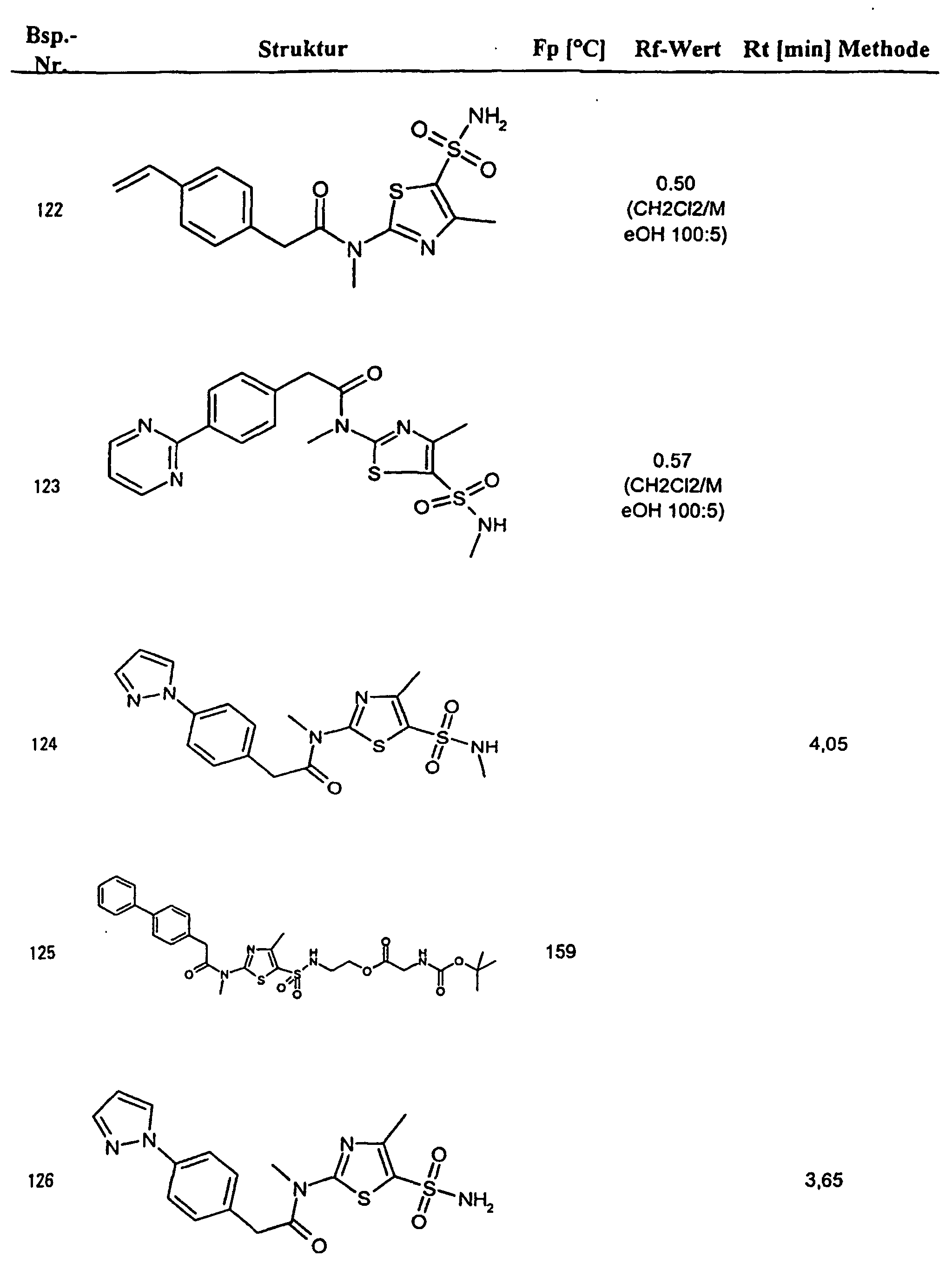

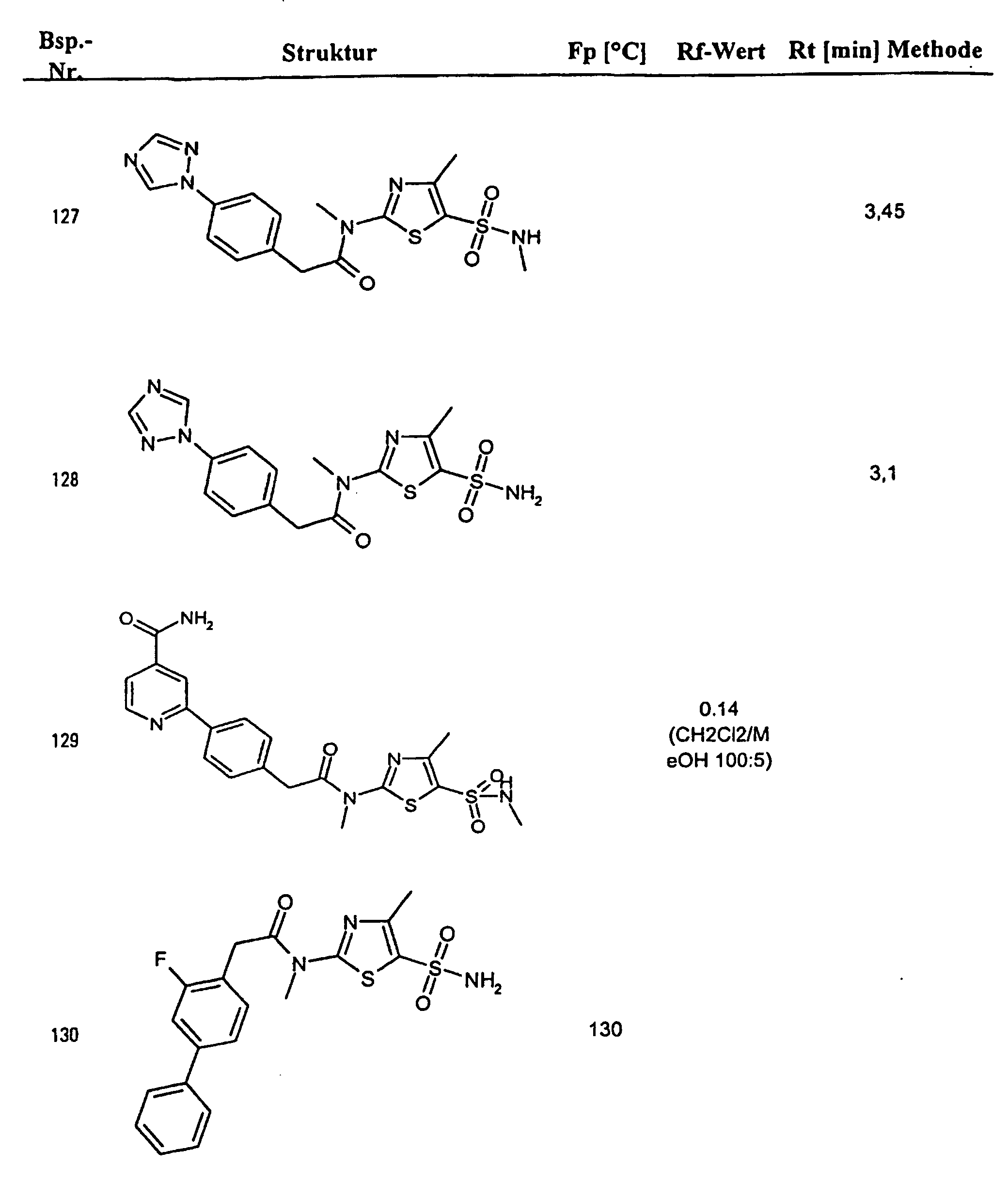

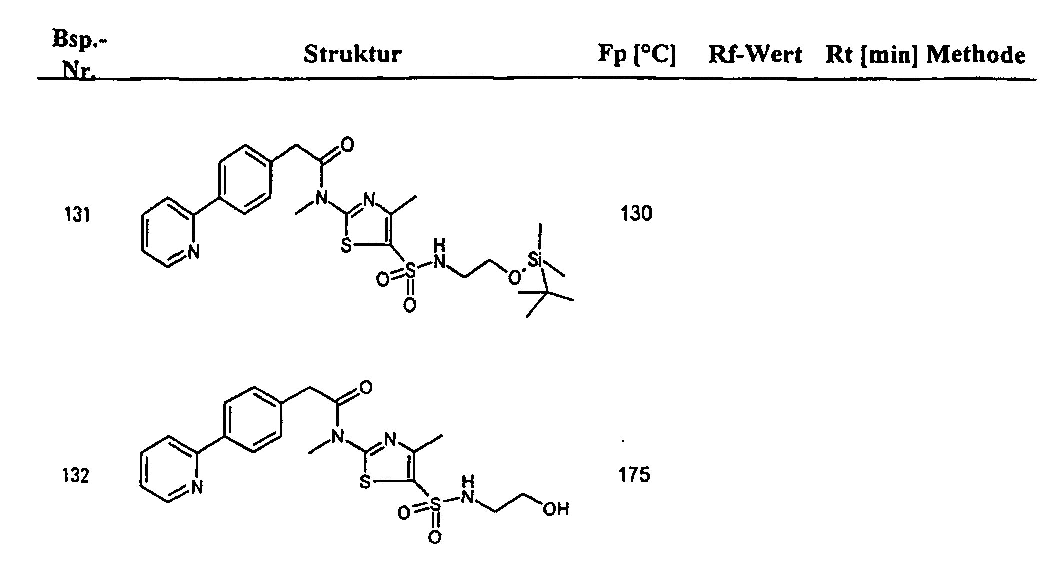

In der vorstehenden Tabelle bedeutet der Rf-Wert den Retentionsindex bei der Dünnschichtchromatographie an Kieselgel. SMKL-N1-1 bezeichnet die folgende LC-MS-Methode

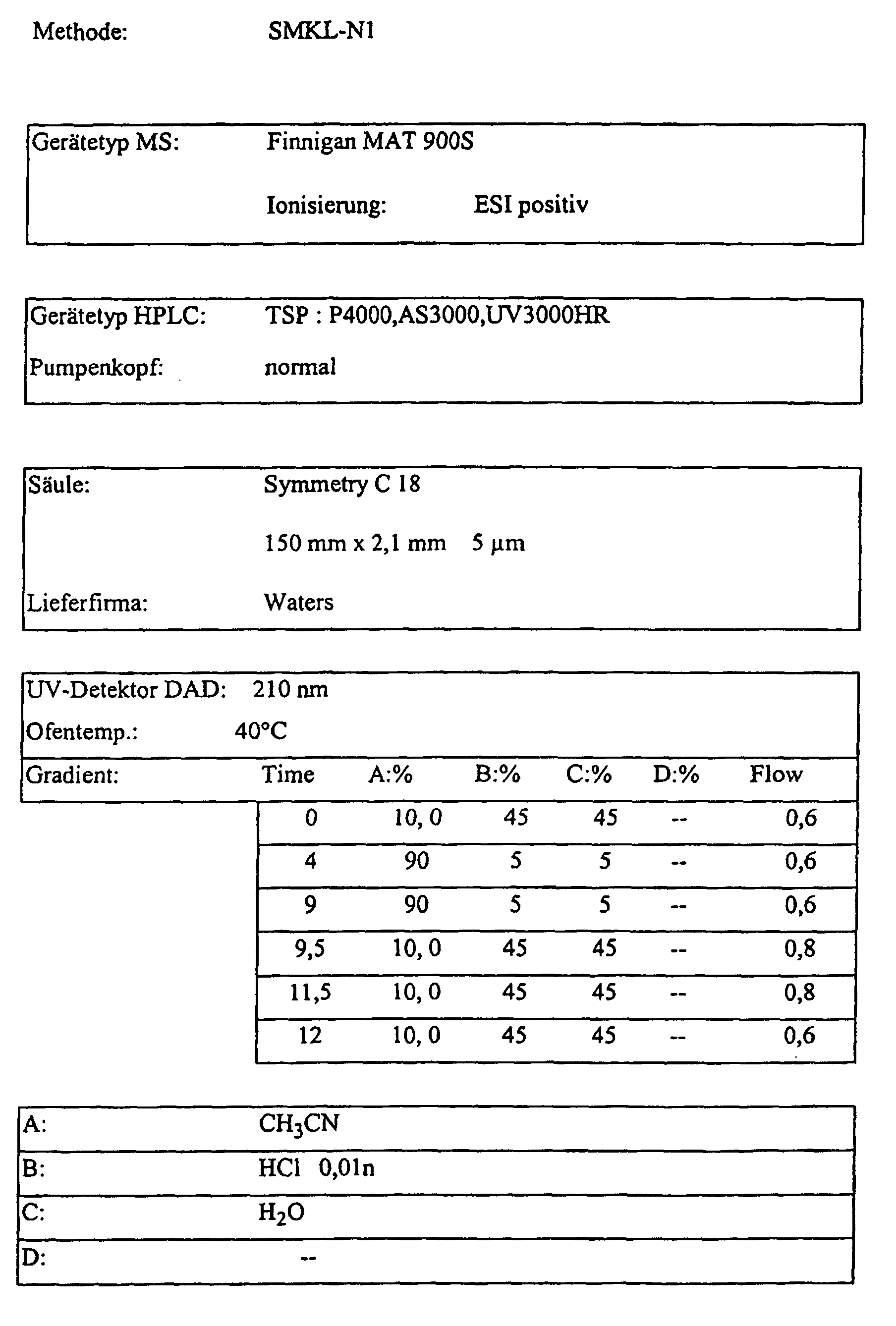

## Patentansprüche

1. Verbindungen der allgemeinen Formel (I): 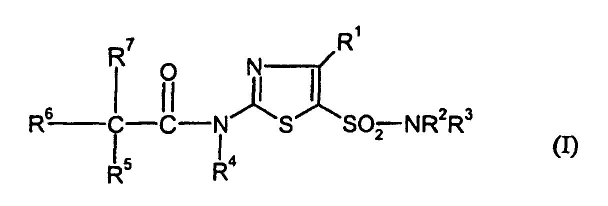 in welcher
R¹ für Wasserstoff, Halogen, (C₁-C₆)-Alkyl, (C₁-C₆)-Alkoxy, Amino-(C₁-C₆)-alkyl oder Halogen-(C₁-C₆)-alkyl steht,
R² und R³ gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Tri-(C₁-C₆)-alkylsilyloxy, Resten der Formel 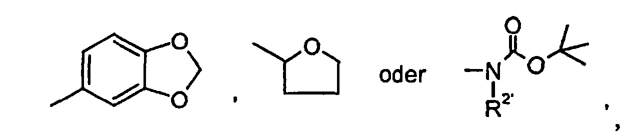 worin R^{2'} für Wasserstoff oder (C₁-C₄)-Alkyl steht,
einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3-bis 8- gliedrigen gesättigten oder ungesättigten, nicht
aromatischen, Heterocyclus mit bis zu 3 Heteroatomen aus der Reihe S, N und/oder O, und
(C₆-C₁₀)-Aryl, das seinerseits durch Hydroxy oder (C₁-C₆)-Alkoxy substituiert sein kann, besteht, oder
für eine Gruppe der Formel 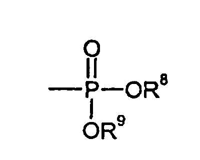 stehen, worin R⁸ und R⁹ gleich oder verschieden voneinander sind und für Wasserstoff und (C₁-C₄)-Alkyl stehen, oder
für eine Gruppe der Formel 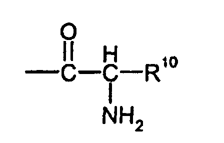 steht, worin R¹⁰ die Seitengruppe einer natürlich vorkommenden α-Aminosäure darstellt, oder
für eine Gruppe der Formel 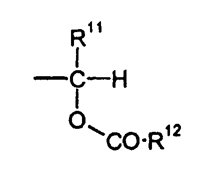 steht, worin R¹¹ für (C₁-C₄)-Alkyl steht, und R¹² für Wasserstoff, (C₁-C₄)-Alkyl oder für eine Gruppe der Formel 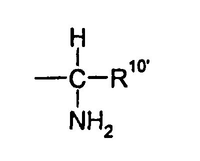 steht, worin R^{10'} die Seitengruppe einer natürlich vorkommenden α-Aminosäure darstellt, oder
R² und R³ gemeinsam mit dem Stickstoffatom einen 5- bis 6-gliedrigen gesättigten Heterocyclus bildet, der gegebenenfalls noch ein Sauerstoffatom aufweisen kann,
R⁴ für Wasserstoff, (C₁-C₆)-Acyl, (C₂-C₆)-Alkenyl, (C₃-C₈)-Cycloalkyl steht, oder
R⁴ für (C₁-C₆)-Alkyl steht, das gegebenfalls substituiert sein kann durch 1 bis 3 Substituenten, die aus der Gruppe ausgewählt werden, die aus Halogen, Hydroxy, (C₃-C₈)-Cycloalkyl, (C₁-C₆)Acyl, (C₁-C₆)-Alkoxy, Carboxyl, 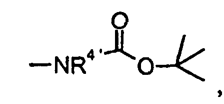 worin R^{4'} für Wasserstoff steht, -(OCH₂CH₂)ₙOCH₂CH₃, worin n 0 oder 1 ist, Phenoxy, (C₆-C₁₀)-Aryl und -NR¹³R¹⁴ besteht,
worin R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxy-carbonyl bedeuten, oder
R¹³ und R¹⁴ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR¹⁵ enthalten kann, und durch Oxo substituiert sein kann,
worin
R¹⁵ Wasserstoff oder (C₁-C₄)-Alkyl bedeutet, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch
Reste der Formeln 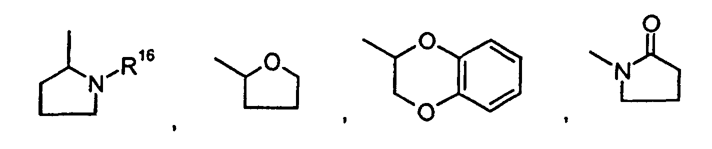 oder 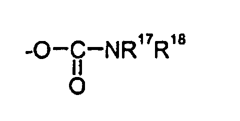 substituiert ist,
worin
R¹⁶ Wasserstoff oder (C₁-C₆)-Alkyl bedeutet,
R¹⁷ und R¹⁸ gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
R⁵ für Wasserstoff, (C₁-C₆)-Alkyl, Halogen, Amino, Mono- oder Di(C₁-C₆)-Alkylamino oder für (C₁-C₆)-Alkanoylamino steht,
R⁶ für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-lkyl, Halogen-(C₁-C₆)-lkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, Tri-(C₁-C₆)-alkylsilyloxy, einem gegebenenfalls über ein Stickstoffatom gebundenen 3-bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkylthio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formel 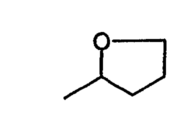 substituiert ist,
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-(C₁-C₆)-alkoxy, Amino, (C₁-C₆)-Alkylthio, Hydroxy, Carboxyl, Carbamoyl, Aminocarbonyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen-(C₁-C₆)-alkyl und Hydroxy-(C₁-C₆)-alkyl substituiert sein kann,
- (C₂-C₆)-Alkenyl
und Gruppen der Formeln
- ―OR¹⁹,
- -NR²⁰R²¹ oder -CO-NR²²R²³,
- Carbazol, Dibenzofuran oder Dibenzothiophen,
- Xanthen oder 9,10-Dihydroacridin
besteht,
worin R¹⁹ Phenyl bedeutet, das seinerseits gegebenenfalls durch eine Gruppe der Formel -NR²⁴R²⁵ substituiert ist,
worin
R²⁴ und R²⁵ gleich oder verschieden sind und Wasserstoff, (C₁-C₆)-Alkyl oder (C₁-C₆)-Acyl bedeuten,
oder
R¹⁹ (C₁-C₆)-Alkyl bedeutet, das gegebenenfalls ein-bis dreifach durch Hydroxy und/oder Halogen substituiert ist,
R²⁰ und R²¹ gleich oder verschieden sind und Wasserstoff, Carbamoyl, Mono- oder Di-(C₁-C₆)-alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R²² und R²³ gleich oder verschieden sind und Wasserstoff oder (C₁-C₆)-Alkyl bedeuten,
und R⁷ die Bedeutung von R⁵ aufweisen kann und mit dieser gleich oder verschieden sein kann,
und deren Salze.

2. Verbindungen der allgemeinen Formel (I) nach Anspruch 1: 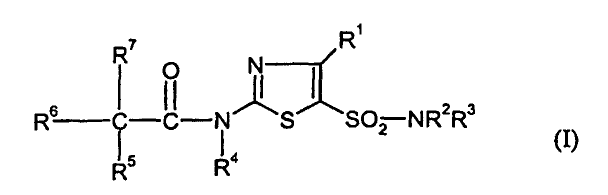 in welcher
R¹ für Wasserstoff, Halogen, (C₁-C₆)-Alkyl, (C₁-C₆)-Alkoxy, Amino(C₁-C₆)alkyl oder Halogen(C₁-C₆)alkyl steht,
R² und R³ gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Resten der Formel 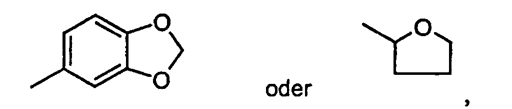 einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8-gliedrigen gesättigten oder ungesättigten, nicht aromatischen, Heterocyclus mit bis zu 3 Heteroatomen aus der Reihe S, N und/oder O, und (C₆-C₁₀)-Aryl, das seinerseits durch Hydroxy oder (C₁-C₆)-Alkoxy substituiert sein kann, besteht, oder
für eine Gruppe der Formel 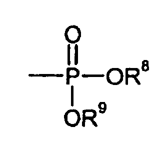 stehen, worin R⁸ und R⁹ gleich oder verschieden voneinander sind und für Wasserstoff und (C₁-C₄)-Alkyl stehen, oder
für eine Gruppe der Formel 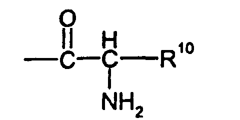 steht, worin R¹⁰ die Seitengruppe einer natürlich vorkommenden α-Aminosäure darstellt, oder
für eine Gruppe der Formel 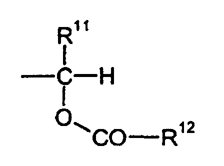 steht, worin R¹¹ für (C₁-C₄)-Alkyl steht, und R¹² für Wasserstoff, (C₁-C₄)-Alkyl oder für eine Gruppe der Formel 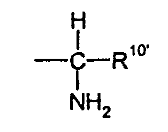 steht, worin R^{10'} die Seitengruppe einer natürlich vorkommenden α-Aminosäure darstellt, oder
R² und R³ gemeinsam mit dem Stickstoffatom einen 5- bis 6-gliedrigen gesättigten Heterocyclus bildet, der gegebenenfalls noch ein Sauerstoffatom aufweisen kann,
R⁴ für Wasserstoff, (C₁-C₆)-Acyl, (C₂-C₆)-Alkenyl, (C₃-C₈)-Cycloalkyl steht, oder
R⁴ für (C₁-C₆)-Alkyl steht, das gegebenfalls substituiert sein kann durch 1 bis 3 Substituenten, die aus der Gruppe ausgewählt werden, die aus Halogen, Hydroxy, (C₁-C₆)Acyl, (C₁-C₆)Alkoxy,
-(OCH₂CH₂)ₙOCH₂CH₃, worin n 0 oder 1 ist, Phenoxy, (C₆-C₁₀)-Aryl und -NR¹³R¹⁴ besteht,
worin R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder
R¹³ und R¹⁴ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR¹⁵ enthalten kann, und durch Oxo substituiert sein kann,
worin R¹⁵ Wasserstoff oder (C₁-C₄)-Alkyl bedeutet, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 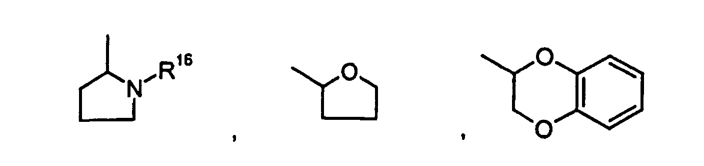 oder 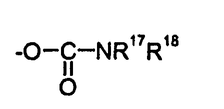 substituiert ist,
worin
R¹⁶ Wasserstoff oder (C₁-C₆)-Alkyl bedeutet,
R¹⁷ und R¹⁸ gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
R⁵ für Wasserstoff, (C₁-C₆)-Alkyl, Halogen, Amino, Mono- oder Di(C₁-C₆)-Alkylamino oder für (C₁-C₆)-Alkanoylamino steht,
R⁶ für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)-Alkoxycarbonylamino, (C₁-C₆)Alkylsulfoxy, (C₁-C₆)Alkylsulfonyl, Tri(C₁-C₆)alkylsilyloxy, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkylthio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formel 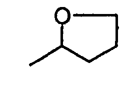 substituiert ist,
- einem gegebenenfalls über ein Stickstoffatom gebundenen 5-bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen(C₁-C₆)alkyl, Halogen(C₁-C₆)-alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)Alkoxycarbonylamino, (C₁-C₆)Alkylsulfoxy, (C₁-C₆)Alkylsulfonyl, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3-bis 8- gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen(C₁-C₆)alkyl und Hydroxy(C₁-C₆)-alkyl substituiert sein kann,
und Gruppen der Formeln
- ―OR¹⁹,
- -NR²⁰R²¹ oder -CO-NR²²R²³
besteht,
worin R¹⁹ Phenyl bedeutet, das seinerseits gegebenenfalls durch eine Gruppe der Formel ―NR²⁴R²⁵ substituiert ist,
worin
R²⁴ und R²⁵ gleich oder verschieden sind und Wasserstoff, (C₁-C₆)-Alkyl oder (C₁-C₆)-Acyl bedeuten,
oder
R¹⁹ (C₁-C₆)-Alkyl bedeutet, das gegebenenfalls ein- bis dreifach durch Hydroxy und/oder Halogen substituiert ist,
R²⁰ und R²¹ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten, wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist, wobei zuvor genanntes Phenyl und zuvor genannter aromatischer Heterocyclus gegebenenfalls ein- bis dreifach gleich oder verschieden durch Halogen und/oder Hydroxy substituiert sind, und
R²² und R²³ gleich oder verschieden sind und Wasserstoff oder (C₁-C₆)-Alkyl bedeuten,
und R⁷ die Bedeutung von R⁵ aufweisen kann und mit dieser gleich oder verschieden sein kann,
und deren Salze.

3. Verbindungen der allgemeinen Formel (I) nach Anspruch 1 oder 2, worin R¹ für Wasserstoff oder (C₁-C₆)-Alkyl steht.

4. Verbindungen der allgemeinen Formel (I) nach Anspruch 1, 2 oder 3, worin R² und R³ jeweils unabhängig für Wasserstoff oder (C₁-C₆)-Alkyl stehen.

5. Verbindungen der allgemeinen Formel (I) nach Anspruch 1, 2, 3 oder 4 worin R⁴ für Wasserstoff oder (C₁-C₆)-Alkyl steht.

6. Verbindungen der allgemeinen Formel (I) nach Anspruch 1, 2, 3, 4 oder 5 worin R⁵ für Wasserstoff steht.

7. Verbindungen der allgemeinen Formel (I) nach Anspruch 1, 2, 3, 4, 5 oder 6 worin
R⁶ für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, Hydroxy, Mono- oder Di(C₁-C₆)alkylamino, Mono- oder Di(C₁-C₆)Alkanoylamino, (C₁-C₆)Alkoxycarbonylamino, und/oder Cyano substituiert sein kann, einem gegebenenfalls über ein Stickstoffatom gebundenen 5- bis 6-gliedrigen aromatischen Heterocyclus mit bis zu 3 Heteroatomen aus der Reihe S, N und/oder O.

8. Verbindungen nach Anspruch 1, die die folgende Formel aufweisen: 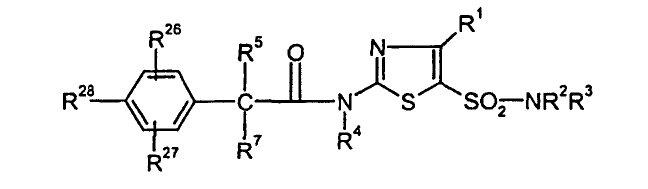 worin R¹, R², R³, R⁴, R⁵ und R⁷ wie im Anspruch 1 definiert sind,
R²⁶ und R²⁷ gleich oder verschieden sind und für Wasserstoff, Halogen, (C₁-C₆)-Alkoxy, (C₁-C₆)-Alkoxycarbonyl, (C₁-C₆)-Alkylthio, Hydroxy, Carboxyl, partiell fluoriertes (C₁-C₆)-Alkoxy mit bis zu 6 Fluoratomen, (C₁-C₆)-Alkyl, eine Gruppe der Formeln -OR¹⁹, -NR²⁰R²¹ oder -CO-NR²²R²³ stehen, worin
R¹⁹ Phenyl bedeutet, das seinerseits gegebenenfalls durch eine Gruppe der Formel ―NR²⁴R²⁵ substituiert ist,
worin R²⁴ und R²⁵ gleich oder verschieden sind und Wasserstoff, (C₁-C₆)-Alkyl oder (C₁-C₆)-Acyl bedeuten, oder
R¹⁹ (C₁-C₆)-Alkyl bedeutet, das gegebenenfalls ein- bis dreifach durch Hydroxy und/oder Halogen substituiert ist,
R²⁰ und R²¹ gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R²² und R²³ gleich oder verschieden sind und Wasserstoff oder (C₁-C₆)-Alkyl bedeuten,
R²⁸ für (C₆-C₁₀)-Aryl steht, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)Alkoxycarbonylamino, (C₁-C₆)-Alkylsulfoxy, (C₁-C₆)Alkylsulfonyl, Tri(C₁-C₆)alkylsilyloxy, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann, oder
R²⁸ für einen gegebenenfalls über ein Stickstoffatom gebundenen 5- bis 6-gliedrigen aromatischen Heterocyclus mit bis zu 3 Heteroatomen aus der Reihe S, N und/oder O steht, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)Alkoxycarbonylamino, (C₁-C₆)Alkylsulfoxy, (C₁-C₆)Alkylsulfonyl, einem gegebenenfalls über ein Stickstoffatom gebundenen 3- bis 8- gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
und deren Salze.

9. Verbindung nach Anspruch 1 der Formel: 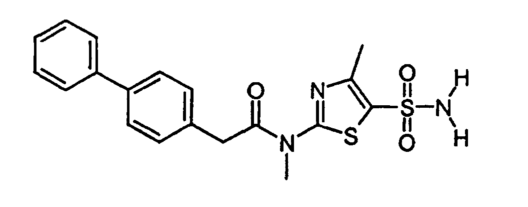 sowie pharmazeutisch verträgliche Salze davon.

10. Verbindung nach Anspruch 1 der Formel: 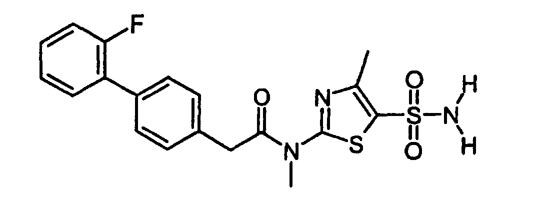 sowie pharmazeutisch verträgliche Salze davon.

11. Verbindung nach Anspruch 1 der Formel: 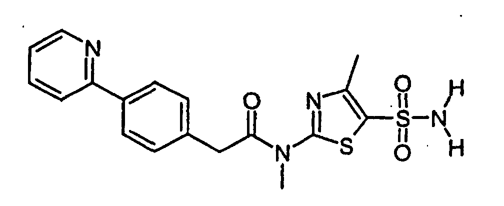 sowie pharmazeutisch verträgliche Salze davon.

12. Verbindung nach Anspruch 1 der Formel:

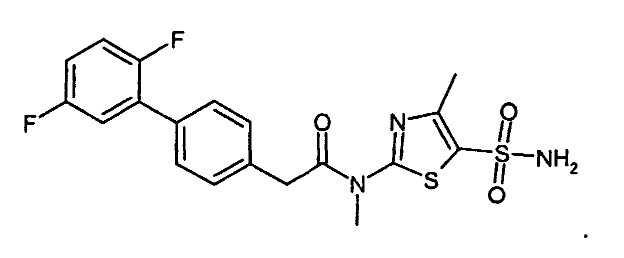

13. Verbindung nach Anspruch 1 der Formel:

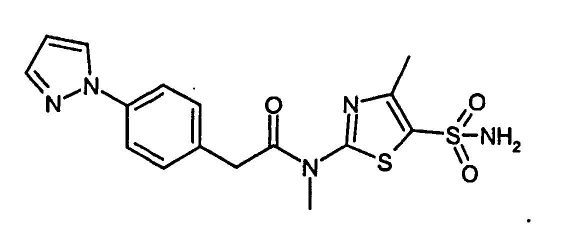

14. Verbindungen der allgemeinen Formel (IV) 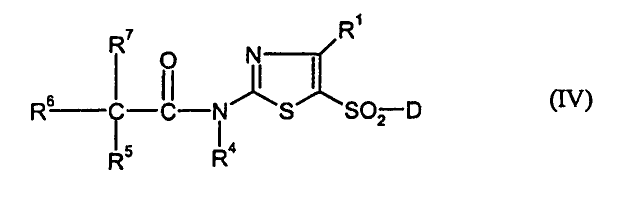 worin R¹, R⁴, R⁵, R⁶ und R⁷ die im Anspruch 1 angegebene Bedeutung haben, und D ein Halogenatom ist.

15. Verfahren zur Herstellung der Verbindungen der allgemeinen Formel (I) nach Anspruch 1, **dadurch gekennzeichnet, daß** man
[A] Verbindungen der allgemeinen Formel (II) 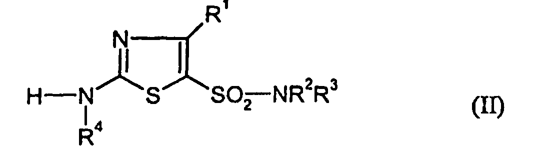 in welcher
R¹, R², R³ und R⁴ die im Anspruch 1 angegebene Bedeutung haben,
mit Verbindungen der allgemeinen Formel (III) 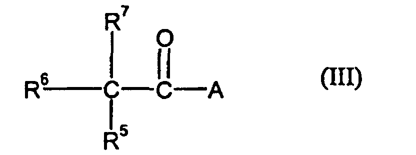 in welcher
A für eine Abgangsgruppe steht, und
R⁵, R⁶ und R⁷ die im Anspruch 1 angegebene Bedeutung haben,
in inerten Lösemitteln, gegebenenfalls in Anwesenheit einer Base und/oder eines Hilfsmittels umsetzt,
oder
[B] Verbindungen der allgemeinen Formel (IV) 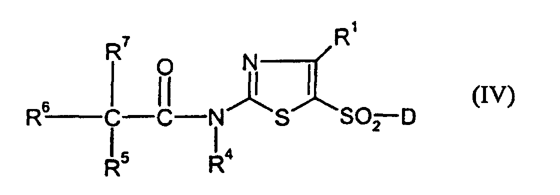 worin
R¹, R⁴, R⁵, R⁶ und R⁷ die im Anspruch 1 angegebene Bedeutung haben, und D ein Halogenatom, vorzugsweise Chlor ist,
mit Aminen der allgemeinen Formel (V):
HNR²R³
worin R² und R³ die im Anspruch 1 angegebene Bedeutung haben,
in inerten Lösemitteln umsetzt,
[C] Verbindungen der allgemeinen Formel (X) 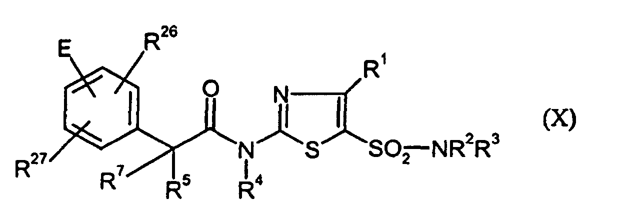 worin R¹, R², R³, R⁴, R⁵, R⁷, R²⁶ und R²⁷ die oben angegebene Bedeutung haben, und E Trifluormethansulfonat oder Halogen ist, mit Boronsäuren oder Stannanen der allgemeinen Formel (XI):
R²⁸M (XI)
worin R²⁸ die oben angegebene Bedeutung hat und M beispielsweise eine Tri(C₁-C₆)alkylstannylgruppe oder eine Boronsäuregruppe sein kann, in inerten Lösemitteln in Gegenwart von Palladiumkatalysatoren, ggf. in Anwesenheit von Base bei Temperaturen von 50 - 140°C zu Verbindungen der Formel (XIV) 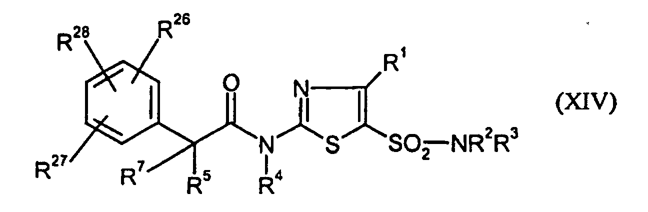 umsetzt, und
[D] Verbindungen der allgemeinen Formel (XII) 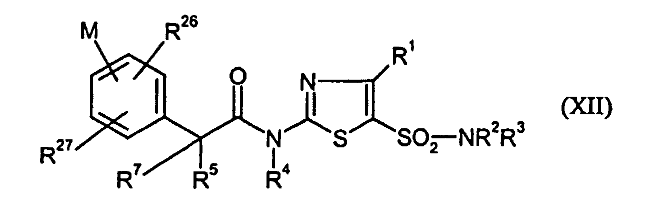
worin R¹, R², R³, R⁴, R⁵, R⁷, R²⁶ und R²⁷ die oben angegebene Bedeutung haben, und M die oben angegebene Bedeutung besitzt, mit Trifluormethansulfonaten oder Halogeniden der allgemeinen Formel (XIII):
R²⁸E (XIII)
worin R²⁸ die oben angegebene Bedeutung hat und E die oben angegebene Bedeutung besitzt, in inerten Lösemitteln in Gegenwart von Palladiumkatalysatoren, ggf. in Anwesenheit von Base, bei Temperaturen von 50-140°C zu Verbindungen der Formel (XIV) umsetzt.

16. Verbindungen nach Anspruch 1 zur Verwendung als Arzneimittel.

17. Pharmazeutische Zusammensetzung, die eine Verbindung der allgemeinen Formel (I) nach Anspruch 1 in Mischung mit einem pharmazeutisch verträglichen Träger oder Exzipienten umfaßt.

18. Verwendung einer Verbindung der allgemeinen Formel (I) nach Anspruch 1 zur Herstellung eines Arzneimittels.

19. Verwendung einer Verbindung der allgemeinen Formel (I) nach Anspruch 1 zur Herstellung eines Arzneimittels zur Behandlung viraler Infektionen.

20. Verwendung einer Verbindung der allgemeinen Formel (I) nach Anspruch 1 zur Herstellung eines Arzneimittels zur Behandlung viraler Infektionen durch Herpes-Viren.

21. Verwendung einer Verbindung der allgemeinen Formel (I) nach Anspruch 1 zur Herstellung eines Arzneimittels zur Behandlung viraler Infektionen durch Herpes Simplex-Viren.

22. Verwendung von N-[5-(Aminosulfonyl)-1,3-thiazol-2-yl]acetamid-Derivaten nach Anspruch 1 zur Herstellung von Arzneimitteln.

23. Verwendung von N-[5-(Aminosulfonyl)-1,3-thiazol-2-yl]-2-phenylacetamid-Derivaten nach Anspruch 1 zur Herstellung von Arzneimitteln.

24. Verwendung von N-[5-(Aminosulfonyl)-1,3-thiazol-2-yl]-2-[1,1'-biphenyl]-4-ylacetamid-Derivaten nach Anspruch 1 zur Herstellung von Arzneimitteln.

25. Verwendung nach Anspruch 22, 23 oder 24 zur Herstellung von Mitteln zur Behandlung und/oder Prävention von viralen Infektionen bei Menschen oder Tieren.

26. Verwendung nach Anspruch 22, 23 oder 24 zur Herstellung von Mitteln zur Behandlung und/oder Prävention von viralen Infektionen bei Menschen oder Tieren durch Herpes-Viren.

27. Verwendung nach Anspruch 22, 23 oder 24 zur Herstellung von Mitteln zur Behandlung und/oder Prävention von viralen Infektionen bei Menschen oder Tieren durch Herpes Simplex-Viren.

## Claims

1. Compounds of the general formula (I): 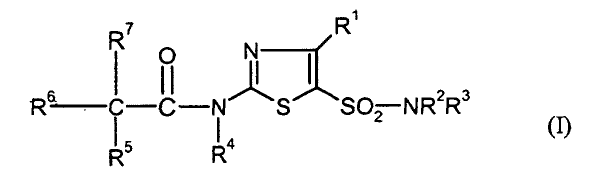 in which
R¹ represents hydrogen, halogen, (C₁-C₆)-alkyl, (C₁-C₆)-alkoxy, amino-(C₁-C₆)-alkyl or halogeno-(C₁-C₆)-alkyl,
R² and R³ are identical or different and represent hydrogen, (C₁-C₆)-alkoxy, (C₃-C₈)-cycloalkyl or biphenylaminocarbonyl, or
represent (C₁-C₆)-alkyl which is optionally substituted by 1 to 3 substituents selected from the group consisting of (C₃-C₆)-cycloalkyl, (C₁-C₆)-alkoxy, halogen, hydroxyl, amino, tri-(C₁-C₆)-alkylsilyloxy, radicals of the formula 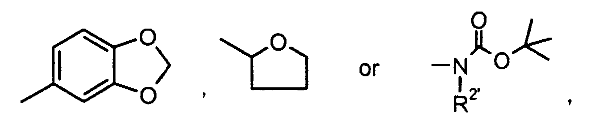 in which R^{2'} represents hydrogen or (C₁-C₄)-alkyl, a 5- or 6-membered aromatic heterocycle having up to 3 heteroatoms from the group consisting of S, N and O, where a nitrogen-containing heterocycle may also be attached via the nitrogen atom,
a 3- to 8-membered saturated or unsaturated nonaromatic heterocycle having up to 3 heteroatoms from the group consisting of S, N and O, which may optionally be attached via a nitrogen atom, and
(C₆-C₁₀)-aryl which for its part may be substituted by hydroxyl or (C₁-C₆)-alkoxy, or
represent a group of the formula 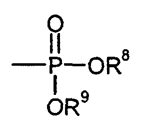
in which R⁸ and R⁹ are identical to or different from one another and represent hydrogen and (C₁-C₄)-alkyl, or
represent a group of the formula 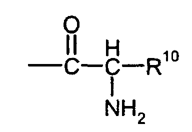 in which R¹⁰ is the side-group of a naturally occurring α-amino acid, or
represent a group of the formula 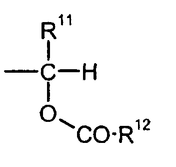 in which R¹¹ represents (C₁-C₄)-alkyl and R¹² represents hydrogen, (C₁-C₄)-alkyl or represents a group of the formula 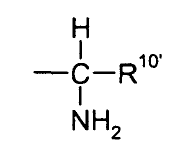 in which R^{10'} is the side-group of a naturally occurring α-amino acid, or
R² and R³ together with the nitrogen atom form a 5- or 6-membered saturated heterocycle which may optionally contain an oxygen atom,
R⁴ represents hydrogen, (C₁-C₆)-acyl, (C₂-C₆)-alkenyl, (C₃-C₈)-cycloalkyl, or
R⁴ represents (C₁-C₆)-alkyl which may optionally be substituted by 1 to 3 substituents selected from the group consisting of halogen, hydroxyl, (C₃-C₈)-cycloalkyl, (C₁-C₆)-acyl, (C₁-C₆)-alkoxy, carboxyl, 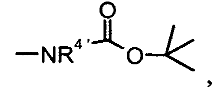 in which R^{4'} represents hydrogen, -(OCH₂CH₂)ₙOCH₂CH₃, in which n is 0 or 1, phenoxy, (C₆-C₁₀)-aryl and -NR¹³R¹⁴,
in which R¹³ and R¹⁴ are identical or different and represent hydrogen, (C₁-C₆)-acyl, (C₁-C₆)-alkyl, carbamoyl, mono- or di-(C₁-C₆)-alkylamino-(C₁-C₆)-alkyl, mono- or di-(C₁-C₆)-alkylaminocarbonyl, (C₆-C₁₀)-aryl or (C₁-C₆)-alkoxycarbonyl, or
R¹³ and R¹⁴ together with the nitrogen atom form a 5- or 6-membered saturated heterocycle which may optionally contain a further heteroatom from the group consisting of S and O or a radical of the formula -NR¹⁵, and which may be substituted by oxo,
in which
R¹⁵ represents hydrogen or (C₁-C₄)-alkyl, or
R⁴ represents (C₁-C₆)-alkyl which is substituted by a 5-or 6-membered aromatic, optionally benzo-fused heterocycle having up to 3 heteroatoms from the group consisting of S, N and O, where a nitrogen-containing heterocycle may also be attached via the nitrogen atom, or which is substituted by
radicals of the formulae 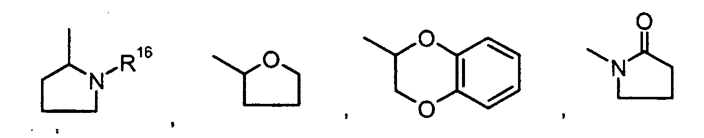 or 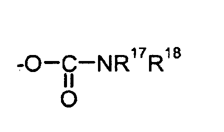 in which
R¹⁶ represents hydrogen or (C₁-C₆)-alkyl,
R¹⁷ and R¹⁸ are identical or different and represent hydrogen, (C₁-C₆)-alkyl or (C₆-C₁₀)-aryl, where abovementioned (C₁-C₆)-alkyl and (C₆-C₁₀)-aryl may optionally be substituted by 1 to 3 substituents selected from the group consisting of hydroxyl, (C₁-C₆)-alkoxy and halogen,
R⁵ represents hydrogen, (C₁-C₆)-alkyl, halogen, amino, mono- or di-(C₁-C₆)-alkylamino or represents (C₁-C₆)-alkanoylamino,
R⁶ represents phenyl which may optionally be substituted by one to three substituents selected from the group consisting of
- halogen,
- (C₆-C₁₀)-aryl which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogeno-(C₁-C₆)-alkyl, halogeno-(C₁-C₆)-alkoxy, amino, (C₁-C₆)-alkylthio, hydroxyl, carboxyl, carbamoyl, mono- or di-(C₁-C₆)-alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsulfonyl, tri-(C₁-C₆)-alkylsilyloxy, a 3- to 8-membered saturated or unsaturated nonaromatic mono- or bicyclic heterocycle having up to 3 heteroatoms from the group consisting of S, N and O, which may optionally be attached via a nitrogen atom, and/or cyano,
- (C₁-C₆)-alkoxy,
- (C₁-C₆)-alkoxycarbonyl,
- (C₁-C₆)-alkylthio,
- hydroxyl,
- carboxyl,
- partially fluorinated (C₁-C₆)-alkoxy having up to 6 fluorine atoms,
- (C₁-C₆)-alkyl which is optionally substituted by a radical of the formula 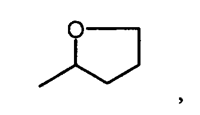
- a 5- or 6-membered aromatic heterocycle having up to 3 heteroatoms from the group consisting of S, N and O which may optionally be attached via a nitrogen atom and which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogeno-(C₁-C₆)-alkyl, halogeno-(C₁-C₆)-alkoxy, amino, (C₁-C₆)-alkylthio, hydroxyl, carboxyl, carbamoyl, aminocarbonyl, mono- or di- (C₁-C₆)-alkylaminocarbonyl, mono- or di- (C₁-C₆)-alkanoylamino, (C₁-C₆)-alkoxycarbonylamino, (C₁-C₆)-alkylsulfoxy, (C₁-C₆)-alkylsuphonyl, a 3- to 8-membered saturated or unsaturated nonaromatic mono- or bicyclic heterocycle having up to 3 heteroatoms from the group consisting of S, N and O which may optionally be attached via a nitrogen atom, and/or cyano,
- a 3- to 8-membered saturated or unsaturated non- aromatic mono- or bicyclic heterocycle having up to 3 heteroatoms from the group consisting of S, N and O, which may optionally be attached via a nitrogen atom and which may optionally be substituted by 1 to 3 substituents selected from the group consisting of oxo, halogen, hydroxyl, (C₁-C₆)-alkoxycarbonyl, (C₁-C₆)-alkoxycarbonylamino, (C₁-C₆)-alkyl, halogeno-(C₁-C₆)-alkyl and hydroxy-(C₁-C₆)-alkyl,
- (C₂-C₆)-alkenyl
and groups of the formulae
- ―OR¹⁹,
- -NR²⁰R²¹ or -CO-NR²²R²³,
- carbazole, dibenzofuran or dibenzothiophene,
- xanthene or 9,10-dihydroacridine,
in which R¹⁹ is phenyl which for its part is optionally substituted by a group of the formula -NR²⁴R²⁵
in which
R²⁴ and R²⁵ are identical or different and represent hydrogen, (C₁-C₆)-alkyl or (C₁-C₆)-acyl,
or
R¹⁹ represents (C₁-C₆)-alkyl which is optionally mono- to trisubstituted by hydroxyl and/or halogen,
R²⁰ and R²¹ are identical or different and represent hydrogen, carbamoyl, mono- or di- (C₁-C₆)-alkylaminocarbonyl, phenyl, (C₁-C₆)-acyl or (C₁-C₆)-alkyl,
where abovementioned (C₁-C₆)-alkyl is optionally substituted by (C₁-C₆)-alkoxy, (C₁-C₆)-acyl, by phenyl or by a 5- or 6-membered aromatic heterocycle having up to 3 heteroatoms from the group consisting of S, N and O,
where abovementioned phenyl and abovementioned aromatic heterocycle are optionally mono- to trisubstituted by identical or different substituents from the group consisting of halogen and hydroxyl, and
R²² and R²³ are identical or different and represent hydrogen or (C₁-C₆)-alkyl,
and R⁷ may have the meaning of R⁵ and may be identical to or different from R⁵,
and their salts.

2. Compounds of the general formula (I) according to Claim 1: 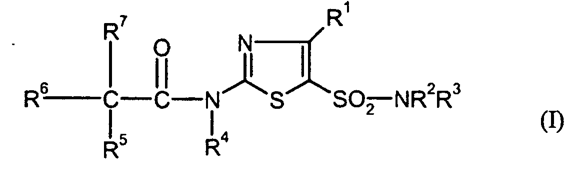 in which
R' represents hydrogen, halogen, (C₁-C₆)-alkyl, (C₁-C₆)-alkoxy, amino-(C₁-C₆)-alkyl or halogeno-(C₁-C₆)-alkyl,
R² and R³ are identical or different and represent hydrogen, (C₁-C₆)-alkoxy, (C₃-C₈)-cycloalkyl or biphenylaminocarbonyl, or
represent (C₁-C₆)-alkyl which is optionally substituted by 1 to 3 substituents selected from the group consisting of (C₃-C₆)-cycloalkyl, (C₁-C₆)-alkoxy, halogen, hydroxyl, amino, radicals of the formula 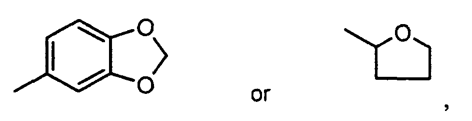 a 5- or 6-membered aromatic heterocycle having up to 3 heteroatoms from the group consisting of S, N and O, where a nitrogen-containing heterocycle may also be attached via the nitrogen atom,
a 3- to 8-membered saturated or unsaturated nonaromatic heterocycle having up to 3 heteroatoms from the group consisting of S, N and O, which may optionally be attached via a nitrogen atom, and
(C₆-C₁₀)-aryl, which for its part may be substituted by hydroxyl or (C₁-C₆)-alkoxy, or
represent a group of the formula 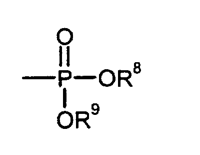 in which R⁸ and R⁹ are identical to or different from one another and represent hydrogen and (C₁-C₄)-alkyl, or
represent a group of the formula 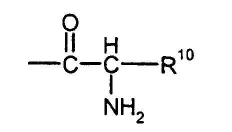 in which R¹⁰ is the side-group of a naturally occurring α-amino acid, or
represent a group of the formula 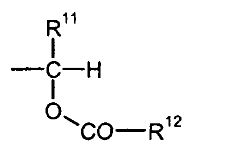 in which R¹¹ represents (C₁-C₄)-alkyl and R¹² represents hydrogen, (C₁-C₄)-alkyl or represents a group of the formula 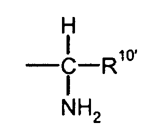 in which R^{10'} is the side-group of a naturally occurring α-amino acid, or
R² and R³ together with the nitrogen atom form a 5- or 6-membered saturated heterocycle which may optionally contain an oxygen atom,
R⁴ represents hydrogen, (C₁-C₆)-acyl, (C₂-C₆)-alkenyl, (C₃-C₈)-cycloalkyl, or
R⁴ represents (C₁-C₆)-alkyl which may optionally be substituted by 1 to 3 substituents selected from the group consisting of halogen, hydroxyl, (C₁-C₆)-acyl, (C₁-C₆)-alkoxy,
-(OCH₂CH₂)ₙOCH₂CH₃, in which n is 0 or 1, phenoxy, (C₆-C₁₀)-aryl and -NR¹³R¹⁴,
in which R¹³ and R¹⁴ are identical or different and represent hydrogen, (C₁-C₆)-acyl, (C₁-C₆)-alkyl, carbamoyl, mono- or di-(C₁-C₆)-alkylamino-(C₁-C₆)-alkyl, mono- or di-(C₁-C₆)-alkylaminocarbonyl, (C₆-C₁₀)-aryl or (C₁-C₆)-alkoxycarbonyl, or
R¹³ and R¹⁴ together with the nitrogen atom form a 5- or 6-membered saturated heterocycle which may optionally contain a further heteroatom from the group consisting of S and O or a radical of the formula -NR¹⁵ and which may be substituted by oxo,
in which R¹⁵ represents hydrogen or (C₁-C₄)-alkyl, or
R⁴ represents (C₁-C₆)-alkyl which is substituted by a 5- or 6-membered aromatic, optionally benzo-fused heterocycle having up to 3 heteroatoms from the group consisting of S, N and O, where a nitrogen-containing heterocycle may also be attached via the nitrogen atom, or which is substituted by radicals of the formulae 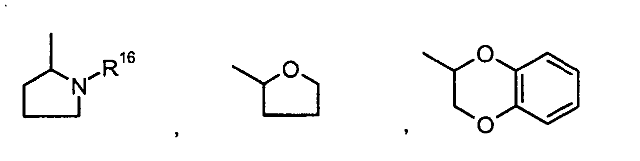 or 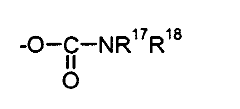 in which
R¹⁶ represents hydrogen or (C₁-C₆)-alkyl,
R¹⁷ and R¹⁸ are identical or different and represent hydrogen, (C₁-C₆)-alkyl or (C₆-C₁₀)-aryl, where abovementioned (C₁-C₆)-alkyl and (C₆-C₁₀)-aryl may optionally be substituted by 1 to 3 substituents selected from the group consisting of hydroxyl, (C₁-C₆)-alkoxy and halogen,
R⁵ represents hydrogen, (C₁-C₆)-alkyl, halogen, amino, mono- or di-(C₁-C₆)-alkylamino or represents (C₁-C₆)-alkanoylamino,
R⁶ represents phenyl which may optionally be substituted by one to three substituents selected from the group consisting of
- halogen,
- (C₆-C₁₀)-aryl which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogeno-(C₁-C₆)-alkyl, halogeno-(C₁-C₆)-alkoxy, amino, (C₁-C₆)-alkylthio, hydroxyl, carboxyl, carbamoyl, mono- or di-(C₁-C₆)-alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsulfonyl, tri-(C₁-C₆)-alkylsilyloxy, a 3- to 8-membered saturated or unsaturated nonaromatic mono- or bicyclic heterocycle having up to 3 heteroatoms from the group consisting of S, N and O, which may optionally be attached via a nitrogen atom, and/or cyano,
- (C₁-C₆)-alkoxy,
- (C₁-C₆)-alkoxycarbonyl,
- (C₁-C₆)-alkylthio,
- hydroxyl,
- carboxyl,
- partially fluorinated (C₁-C₆)-alkoxy having up to 6 fluorine atoms,
- (C₁-C₆)-alkyl which is optionally substituted by a radical of the formula 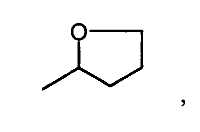
- a 5- or 6-membered aromatic heterocycle having up to 3 heteroatoms from the group consisting of S, N and O which may optionally be attached via a nitrogen atom and which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogeno-(C₁-C₆)-alkyl, halogeno-(C₁-C₆)-alkoxy, amino, (C₁-C₆)-alkylthio, hydroxyl, carboxyl, carbamoyl, mono- or di-(C₁-C₆)-alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsuphonyl, a 3- to 8-membered saturated or unsaturated nonaromatic mono- or bicyclic heterocycle having up to 3 heteroatoms from the group consisting of S, N and O which may optionally be attached via a nitrogen atom, and/or cyano,
- a 3- to 8-membered saturated or unsaturated non- aromatic mono- or bicyclic heterocycle having up to 3 heteroatoms from the group consisting of S, N and O, which may optionally be attached via a nitrogen atom and which may optionally be substituted by 1 to 3 substituents selected from the group consisting of oxo, halogen, hydroxyl, (C₁-C₆)-alkoxycarbonyl, (C₁-C₆)-alkoxycarbonylamino, (C₁-C₆)-alkyl, halogeno-(C₁-C₆)-alkyl and hydroxy-(C₁-C₆)-alkyl,
and groups of the formulae
- ―OR¹⁹,
- -NR²⁰R²¹ or -CO-NR²²R²³,
in which R¹⁹ is phenyl which for its part is optionally substituted by a group of the formula -NR²⁴R²⁵,
in which
R²⁴ and R²⁵ are identical or different and represent hydrogen, (C₁-C₆)-alkyl or (C₁-C₆)-acyl,
or
R¹⁹ represents (C₁-C₆)-alkyl which is optionally mono- to trisubstituted by hydroxyl and/or halogen,
R²⁰ and R²¹ are identical or different and represent hydrogen, carbamoyl, mono- or di- (C₁-C₆)-alkylaminocarbonyl, phenyl, (C₁-C₆)-acyl or (C₁-C₆)-alkyl,
where abovementioned (C₁-C₆)-alkyl is optionally substituted by (C₁-C₆)-alkoxy, (C₁-C₆)-acyl, by phenyl or by a 5- or 6-membered aromatic heterocycle having up to 3 heteroatoms from the group consisting of S, N and O, where abovementioned phenyl and abovementioned aromatic heterocycle are optionally mono- to trisubstituted by identical or different substituents from the group consisting of halogen and hydroxyl, and
R²² and R²³ are identical or different and represent hydrogen or (C₁-C₆)-alkyl, and R⁷ may have the meaning of R⁵ and may be identical to or different from R⁵,
and their salts.

3. Compounds of the general formula (I) according to Claim 1 or 2, in which R¹ represents hydrogen or (C₁-C₆)-alkyl.

4. Compounds of the general formula (I) according to any of Claims 1, 2 and 3, in which R² and R³ each independently represent hydrogen or (C₁-C₆)-alkyl.

5. Compounds of the general formula (I) according to any of Claims 1, 2, 3 and 4, in which R⁴ represents hydrogen or (C₁-C₆)-alkyl.

6. Compounds of the general formula (I) according to any of Claims 1, 2, 3, 4 and 5, in which R⁵ represents hydrogen.

7. Compounds of the general formula (I) according to any of Claims 1, 2, 3, 4, 5 and 6, in which
R⁶ represents phenyl which may optionally be substituted by one to three substituents selected from the group consisting of
- halogen,
- (C₆-C₁₀)-aryl which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogeno-(C₁-C₆)-alkyl, halogeno-(C₁-C₆)-alkoxy, amino, hydroxyl, mono- or di-(C₁-C₆)-alkylamino, mono- or di-(C₁-C₆)-alkanoylamino, (C₁-C₆)-alkoxycarbonylamino, and/or cyano,
- a 5- or 6-membered aromatic heterocycle having up to 3 heteroatoms from the group consisting of S, N and O, which may optionally be attached via a nitrogen atom.

8. Compounds according to Claim 1 having the following formula: 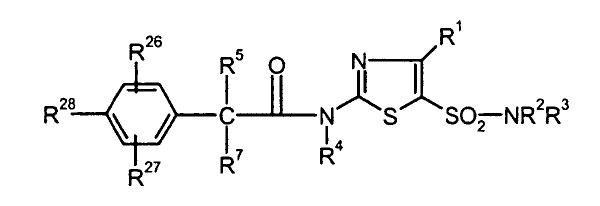 in which R¹, R², R³, R⁴, R⁵ and R⁷ are each as defined in Claim 1,
R²⁶ and R²⁷ are identical or different and represent hydrogen, halogen, (C₁-C₆)-alkoxy, (C₁-C₆)-alkoxycarbonyl, (C₁-C₆)-alkylthio, hydroxyl, carboxyl, partially fluorinated (C₁-C₆)-alkoxy having up to 6 fluorine atoms, (C₁-C₆)-alkyl, a group of the formulae -OR¹⁹,-NR²⁰R²¹ or -CO-NR²²R²³, in which
R¹⁹ represents phenyl which for its part is optionally substituted by a group of the formula -NR²⁴R²⁵,
in which R²⁴ and R²⁵ are identical or different and represent hydrogen, (C₁-C₆)-alkyl or (C₁-C₆)-acyl, or
R¹⁹ represents (C₁-C₆)-alkyl which is optionally mono- to trisubstituted by hydroxyl and/or halogen,
R²⁰ and R²¹ are identical or different and represent hydrogen, carbamoyl, mono- or di-(C₁-C₆)-alkylaminocarbonyl, phenyl, (C₁-C₆)-acyl or (C₁-C₆)-alkyl,
where abovementioned (C₁-C₆)-alkyl is optionally substituted by (C₁-C₆)-alkoxy, (C₁-C₆)-acyl, phenyl or by a 5- or 6-membered aromatic heterocycle having up to 3 heteroatoms from the group consisting of S, N and O,
where abovementioned phenyl and abovementioned aromatic heterocycle are optionally mono- to trisubstituted by identical or different substituents from the group consisting of halogen and hydroxyl, and
R²² and R²³ are identical or different and represent hydrogen or (C₁-C₆)-alkyl,
R²⁸ represents (C₆-C₁₀)-aryl, which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogen-(C₁-C₆)-alkyl, halogen-(C₁-C₆)-alkoxy, amino, (C₁-C₆)-alkylthio, hydroxyl, carboxyl, carbamoyl, mono- or di-(C₁-C₆)-alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsulfonyl, tri-(C₁-C₆)-alkylsilyloxy, a 3- to 8-membered saturated or unsaturated nonaromatic mono- or bicyclic heterocycle having up to 3 heteroatoms from the group consisting of S, N and O, which may optionally be attached via a nitrogen atom, and/or cyano, or
R²⁸ represents a 5- or 6-membered aromatic heterocycle having up to 3 heteroatoms from the group consisting of S, N and O, which may optionally be attached via a nitrogen atom and which may optionally be substituted by 1 to 3 substituents selected from the group consisting of (C₁-C₆)-alkanoyl, (C₁-C₆)-alkoxy, (C₁-C₆)-alkyl, halogen, (C₁-C₆)-alkoxycarbonyl, nitro, halogeno-(C₁-C₆)-alkyl, halogeno-(C₁-C₆)-alkoxy, amino, (C₁-C₆)-alkylthio, hydroxyl, carboxyl, carbamoyl, mono- or di-(C₁-C₆)-alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsulfonyl, a 3- to 8-membered saturated or unsaturated nonaromatic mono- or bicyclic heterocycle having up to 3 heteroatoms from the group consisting of S, N and O, which may optionally be attached via a nitrogen atom, and/or cyano,
and their salts.

9. Compounds according to Claim 1 of the formula: 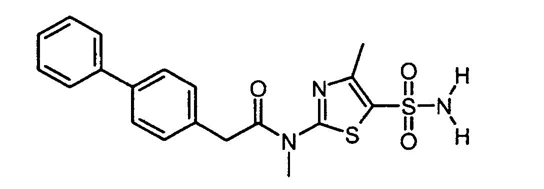 and pharmaceutically acceptable salts thereof.

10. Compounds according to Claim 1 of the formula: 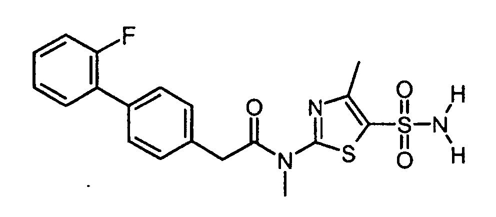 and pharmaceutically acceptable salts thereof.

11. Compounds according to Claim 1 of the formula: 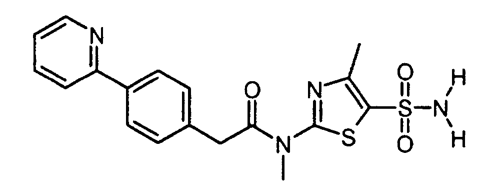 and pharmaceutically acceptable salts thereof.

12. Compounds according to Claim 1 of the formula:

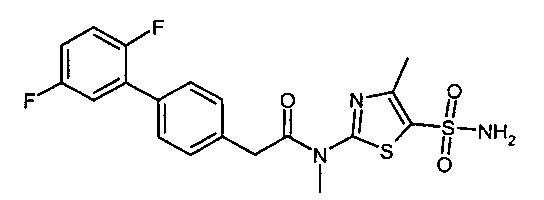

13. Compounds according to Claim 1 of the formula:

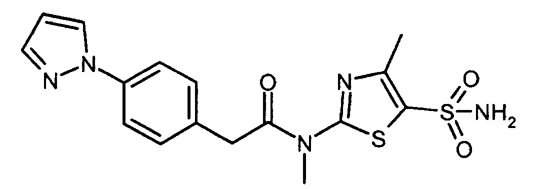

14. Compounds of the general formula (IV) 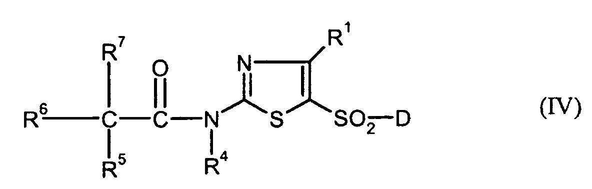 in which R¹, R⁴, R⁵, R⁶ and R⁷ are each as defined in Claim 1 and D is a halogen atom.

15. Process for preparing the compounds of the general formula (I) according to Claim 1, **characterized in that**
[A] compounds of the general formula (II) 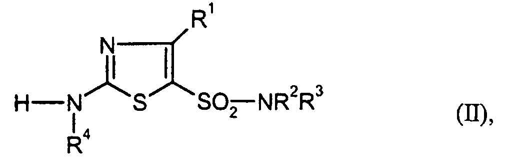 in which
R¹, R², R³ and R⁴ are each as defined in Claim 1,
are reacted with compounds of the general formula (III) 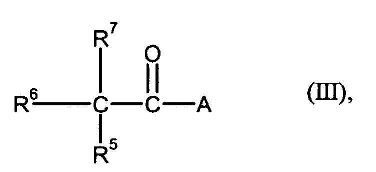 in which
A represents a leaving group and
R⁵, R⁶ and R⁷ are each as defined in Claim 1,
in inert solvents, if appropriate in the presence of a base and/or an auxiliary,
or
[B] compounds of the general formula (IV) 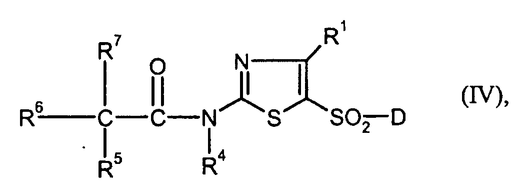 in which
R¹, R⁴, R⁵, R⁶ and R⁷ are each as defined in Claim 1 and
D is a halogen atom, preferably chlorine,
are reacted with amines of the general formula (V),
HNR²R³
in which R² and R³ are each as defined in Claim 1,
in inert solvents,
[C] compounds of the general formula (X) 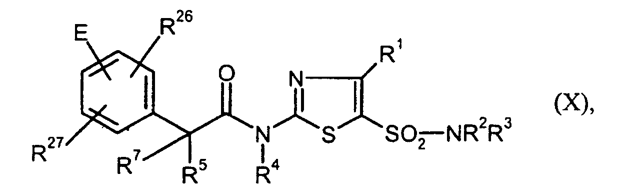 in which R¹, R², R³, R⁴, R⁵, R⁷, R²⁶ and R²⁷ are each as defined above and E is trifluoromethanesulphonate or halogen, are reacted with boronic acids or stannanes of the general formula (XI):
R²⁸M (XI),
in which R²⁸ is as defined above and M may be, for example, a tri-(C₁-C₆)-alkylstannyl group or a boronic acid group, in inert solvents in the presence of palladium catalysts, if appropriate in the presence of base, at temperatures of 50 - 140°C, to give compounds of the formula (XIV). 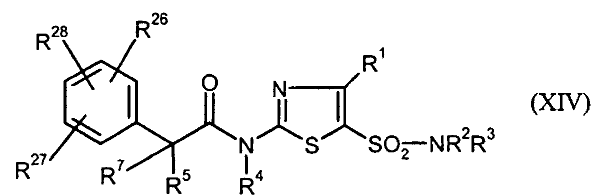 and
[D] compounds of the general formula (XII) 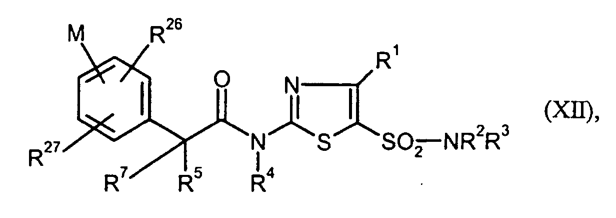
in which R¹, R², R³, R⁴, R⁵, R⁷, R²⁶ and R²⁷ are each as defined above and M is as defined above, are reacted with trifluoromethanesulphonates or halides of the general formula (XIII):
R²⁸E (XIII),
in which R²⁸ is as defined above and E is as defined above, in inert solvents in the presence of palladium catalysts, if appropriate in the presence of a base, at temperatures of 50-140°C, to give compounds of the formula (XIV).

16. Compounds according to Claim 1 for use as a medicament.

17. Pharmaceutical composition, comprising a compound of the general formula (I) according to Claim 1 in a mixture with a pharmaceutically acceptable carrier or excipient.

18. Use of a compound of the general formula (I) according to Claim 1 for preparing a medicament.

19. Use of a compound of the general formula (I) according to Claim 1 for preparing a medicament for treating viral infections.

20. Use of a compound of the general formula (I) according to Claim 1 for preparing a medicament for treating viral infections caused by herpes viruses.

21. Use of a compound of the general formula (I) according to Claim 1 for preparing a medicament for treating viral infections caused by Herpes simplex viruses.

22. Use of N-[5-(aminosulfonyl)-1,3-thiazol-2-yl]acetamide derivatives according to Claim 1 for preparing medicaments.

23. Use of N-[5-(aminosulfonyl)-1,3-thiazol-2-yl]-2-phenylacetamide derivatives according to Claim 1 for preparing medicaments.

24. Use of N-[5-(aminosulfonyl)-1,3-thiazol-2-yl]-2-[1,1'-biphenyl]-4-ylacetamide derivatives according to Claim 1 for preparing medicaments.

25. Use according to any of Claims 22, 23 and 24 for preparing compositions for the treatment and/or prevention of viral infections in humans or animals.

26. Use according to any of Claims 22, 23 and 24 for preparing compositions for the treatment and/or prevention of viral infections caused by herpes viruses in humans or animals.

27. Use according to any of Claims 22, 23 and 24 for preparing compositions for the treatment and/or prevention of viral infections caused by Herpes simplex viruses in humans or animals.

## Revendications

1. Composés de la formule générale (I) : 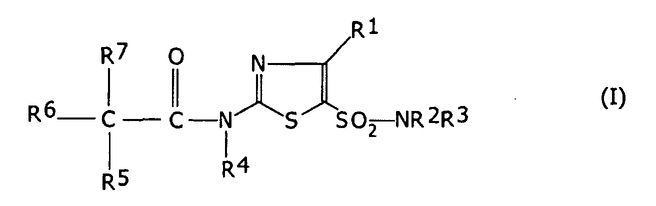 dans laquelle
R¹ représente l'atome d'hydrogène, un atome d'halogène, un radical alcoyle (C₁-C₆), alcoxy (C₁-C₆). aminoalcoyle (C₁-C₆) ou halogénoalcoyle (C₁-C₆),
R² et R³ sont identiques ou différents et représentent l'atome d'hydrogène, un radical alcoxy (C₁-C₆), cycloalcoyle (C₃-C₈) ou biphénylaminocarbonyle,
représentent un radical alcoyle (C₁-C₆), qui est le cas échéant substitué par 1 à 3 substituants, qui sont choisis parmi le groupe qui consiste en un radical cycloalcoyle (C₃-C₈), alcoxy (C₁-C₆), un atome d'halogène, le radical hydroxyle, amino, un radical trialcoyl (C₁-C₆)silyloxy, des restes des formules : 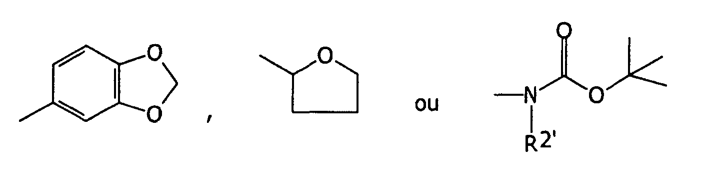 où R^{2'} représente l'atome d'hydrogène ou un radical alcoyle (C₁-C₄),
représentent un hétérocycle aromatique de 5 à 6 membres, avec jusqu'à 3 hétéroatomes de la série S, N et/ou O, où un hétérocycle azoté peut également être relié par l'atome d'azote,
représentent un hétérocycle non aromatique, saturé ou insaturé, de 3 à 8 membres, le cas échéant lié par un atome d'azote, avec jusqu'à 3 hétéroatomes de la série S, N et/ou O, et
représentent un radical aryle (C₆-C₁₀), qui peut être substitué par le radical hydroxyle ou par un radical alcoxy (C₁-C₆), ou
représentent un radical de la formule : 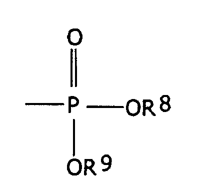 où R⁸ et R⁹ sont identiques ou différents l'un de l'autre et représentent l'atome d'hydrogène ou un radical alcoyle (C₁-C₄), ou
représentent un radical de la formule : 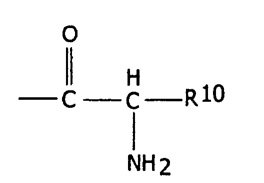 où R¹⁰ représente un groupe latéral d'un acide α-aminé naturel, ou
représentent un radical de la formule : 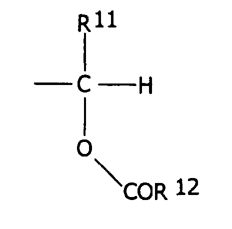 où R¹¹ représente un radical alcoyle (C₁-C₄), et R¹² représente l'atome d'hydrogène, un radical alcoyle (C₁-C₄) ou un radical de la formule : 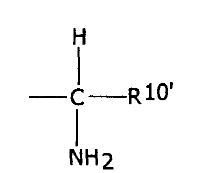 où R^{10'} représente un groupe latéral d'un acide α-aminé naturel, ou
R² et R³ forment avec l'atome d'azote, un hétérocycle saturé de 5 à 6 membres, qui peut présenter le cas échéant, encore un atome d'oxygène,
R⁴ représente l'atome d'hydrogène, un radical acyle (C₁-C₆), alcényle (C₂-C₆), cycloalcoyle (C₃-C₈),
R⁴ représente un radical alcoyle (C₁-C₆), qui peut être le cas échéant, substitué par 1 à 3 substituants, qui sont choisis parmi le groupe qui consiste en un atome d'halogène, le radical hydroxyle, un radical cycloalcoyle (C₃-C₈),acyle (C₁-C₆), alcoxy (C₂-C₆), carboxyle, 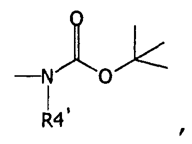 où R^{4'} représente l'atome d'hydrogène, qui consiste en le radical - (OCH₂CH₂)ₙOCH₂CH₃, où n est 0 ou 1, le radical phénoxy, un radical aryle (C₆-C₁₀) et -NR¹³R¹⁴,
où R¹³ et R¹⁴ sont identiques ou différents et représentent l'atome d'hydrogène, un radical acyle (C₁-C₆), alcoyle (C₁-C₆), carbamoyle, mono- ou di (alcoyl (C₁-C₆))aminoalcoyle (C₁-C₆), mono- ou di (alcoyl (C₁-C₆))aminocarbonyle, aryle (C₆-C₁₀) ou alcoxy (C₁-C₆)-carbonyle, ou
R¹³ et R¹⁴ forment avec l'atome d'azote, un hétérocycle saturé de 5 à 6 membres, qui peut contenir le cas échéant, un autre hétéroatome de la série S ou O ou un reste de la formule -NR¹⁵, et qui peut être substitué par oxo,
où
R¹⁵ représente l'atome d'hydrogène ou un radical alcoyle (C₁-C₄),
R⁴ représente un radical alcoyle (C₁-C₆), qui est substitué par un hétérocycle aromatique de 5 à 6 membres, le cas échéant benzocondensé, avec jusqu'à 3 hétéroatomes de la série S, N et/ou O, où un hétérocycle azoté peut également être lié par l'atome d'azote, ou est substitué par un reste des formules : 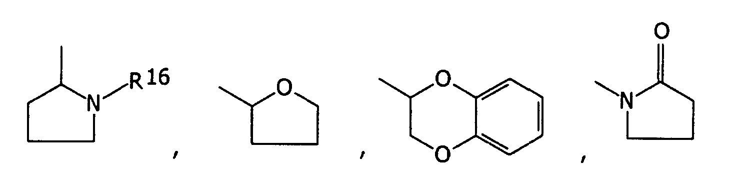 ou 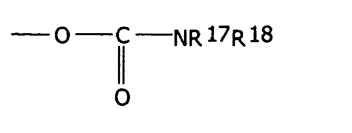 où
R¹⁶ représente l'atome d'hydrogène ou un radical alcoyle (C₁-C₆),
R¹⁷ et R¹⁸ sont identiques ou différents et représentent l'atome d'hydrogène, un radical alcoyle (C₁-C₆) ou aryle (C₆-C₁₀), où les radicaux alcoyle (C₁-C₆) et aryle (C₆-C₁₀) peuvent être le cas échéant substitués par 1 à 3 substituants, qui sont choisis parmi le groupe qui consiste en le radical hydroxyle, un radical alcoxy (C₁-C₆) et un atome d'halogène,
R⁵ représente l'atome d'hydrogène, un radical alcoyle (C₁-C₆), un atome d'halogène, un radical amino, mono- ou di(alcoyl (C₁-C₆))amino ou un radical (alcanoyl (C₁-C₆))amino,
R⁶ représente le radical phényle, qui peut être le cas échéant substitué par 1 à 3 substituants, qui sont choisis parmi le groupe qui consiste e,
- un atome d'halogène,
- un radical aryle (C₆-C₁₀), qui peut être le cas échéant substitué par 1 à 3 substituants, choisis parmi un radical alcanoyle (C₁-C₆), alcoxy (C₁-C₆), alcoyle (C₁-C₆), un atome d'halogène, un radical (alcoxy (C₁-C₆))carbonyle, le radical nitro, un radical halogénoalcoyle (C₁-C₆), un radical halogénoalcoxy(C₁-C₆), amino, alcoylthio (C₁-C₆), hydroxyle, carboxyle, carbamoyle, mono- ou di(alcoyl (C₁-C₆))aminocarbonyle, mono- ou di (alcanoyl (C₁-C₆))amino, (alcoxy (C₁-C₆))carbonylamino, alcoylsulfonyloxy (C₁-C₆), alcoylsulfoxy (C₁-C₆), alcoylsulfonyle (C₁-C₆), tri (alcoyl (C₁-C₆))silyloxy, un hétérocycle non aromatique, saturé ou insaturé de 3 à 8 membres, mono= ou bicyclique, le cas échéant lié par un atome d'azote, avec jusqu'à 3 hétéroatomes de la série S, N et/ou O, et/ou le radical cyano,
- un radical alcoxy (C₁-C₆),
- un radical (alcoxy (C₁-C₆))carbonyle,
- un radical alcoylthio (C₁-C₆),
- le radical hydroxyle,
- le radical carboxyle,
- un radical alcoxy (C₁-C₆) partiellement fluoré, avec jusqu'à 6 atomes de fluor,
- un radical alcoyle (C₁-C₆), qui est le cas échéant substitué par un reste de la formule 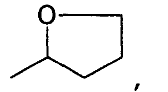
- un hétérocycle aromatique de 5 à 6 membres, le cas échéant lié par un atome d'azote, avec jusqu'à 3 hétéroatomes de la série S, N et/ou O, qui peut être le cas échéant substitué par 1 à 3 substituants, choisis parmi un radical alcanoyle (C₁-C₆), alcoxy (C₁-C₆), alcoyle (C₁-C₆), un atome d'halogène, un radical (alcoxy (C₁-C₆))carbonyle, le radical nitro, un radical halogénoalcoyle (C₁-C₆), un radical halogénoalcoxy(C₁-C₆), amino, alcoylthio (C₁-C₆), hydroxyle, carboxyle, carbamoyle, aminocarbonyle, mono- ou di(alcoyl (C₁-C₆))aminocarbonyle, mono- ou di(alcanoyl (C₁-C₆))amino, (alcoxy (C₁-C₆))carbonylamino, alcoylsulfoxy (C₁-C₆), alcoylsulfonyle (C₁-C₆), tri(alcoyl (C₁-C₆))silyloxy, un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, et/ou le radical cyano,
- un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, qui peut être le cas échéant substitué par 1 à 3 substituants, choisis parmi le radical oxo, un atome d'halogène, le radical hydroxyle, un radical (alcoxy (C₁-C₆))carbonyle, (alcoxy (C₁-C₆))carbonylamino, alcoyle (C₁-C₆), halogénoalcoyle (C₁-C₆) et hydroxyalcoyle (C₁-C₆),
- un radical alcényle (C₂-C₆),
et les groupes des formules
- -OR¹⁹,
- -NR²⁰R²¹ ou -CO-NR²²R²³,
- le radical carbazole, dibenzofuranne ou dibenzothiophène,
- le radical xanthène ou 9,10-dihydroacridine,
où R¹⁹ représente le radical phényle, qui est pour sa part, substitué le cas échéant par un radical de la formule -NR²⁴R²⁵,
où R²⁴ et R²⁵ sont identiques ou différents et représentent l'atome d'hydrogène, un radical alcoyle (C₁-C₆) ou acyle (C₁-C₆),
ou R¹⁹ représente un radical alcoyle (C₁-C₆), qui peut être substitué le cas échéant, une à trois fois, par le radical hydroxyle et/ou un atome d'halogène,
R²⁰ et R²¹ sont identiques ou différents et représentent l'atome d'hydrogène, le radical carbamoyle, un radical mono- ou di(alcoyle (C₁-C₆))aminocarbonyle, phényle, acyle (C₁-C₆) ou alcoyle (C₁-C₆),
où le radical alcoyle (C₁-C₆) indiqué précédemment est le cas échéant substitué par un radical alcoxy (C₁-C₆), acyle (C₁-C₆), par le radical phényle ou par un hétérocycle aromatique, de 5 à 6 membres, avec jusqu'à 3 hétéroatomes de la série S, N et/ou O,
où le radical phényle indiqué précédemment et l'hétérocycle aromatique indiqué précédemment sont le cas échéant substitués une à trois fois, de manière identique ou différente, par un atome d'halogène et/ou le radical hydroxyle,
R²² et R²³ sont identiques ou différents et représentent l'atome d'hydrogène ou un radical alcoyle (C₁-C₆),
et R⁷ peut présenter la signification de R⁵ et peut être identique ou différent de celui-ci,
et leurs sels.

2. Composés de la formule générale (I) selon la revendication 1 : 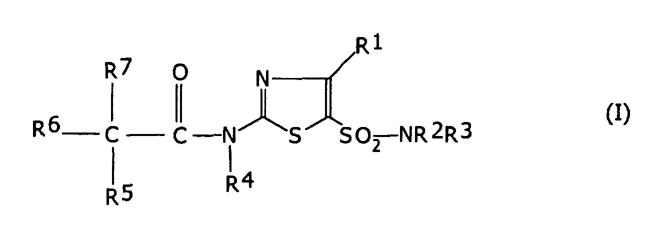 dans laquelle
R¹ représente l'atome d'hydrogène, un atome d'halogène, un radical alcoyle (C₁-C₆), alcoxy (C₁-C₆), aminoalcoyle (C₁-C₆) ou halogénoalcoyle (C₁-C₆),
R² et R³ sont identiques ou différents et représentent l'atome d'hydrogène, un radical alcoxy (C₁-C₆), cycloalcoyle (C₃-C₈) ou biphénylaminocarbonyle, ou
représentent un radical alcoyle (C₁-C₆), qui est le cas échéant substitué par 1 à 3 substituants, qui sont choisis parmi le groupe qui consiste en un radical cycloalcoyle (C₃-C₈), alcoxy (C₁-C₆), un atome d'halogène, le radical hydroxyle, amino, des restes des formules : 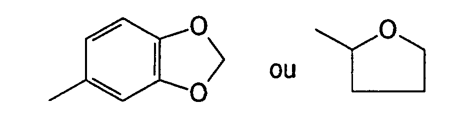 représentent un hétérocycle aromatique de 5 à 6 membres, avec jusqu'à 3 hétéroatomes de la série S, N et/ou O, où un hétérocycle azoté peut également être relié par l'atome d'azote,
représentent un hétérocycle non aromatique, saturé ou insaturé, de 3 à 8 membres, le cas échéant lié par un atome d'azote, avec jusqu'à 3 hétéroatomes de la série S, N et/ou O, et
représentent un radical aryle (C₆-C₁₀), qui peut être substitué par le radical hydroxyle ou par un radical alcoxy (C₁-C₆), ou
représentent un radical de la formule : 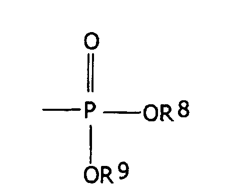 où R⁸ et R⁹ sont identiques ou différents l'un de l'autre et représentent l'atome d'hydrogène ou un radical alcoyle (C₁-C₄), ou
représentent un radical de la formule : 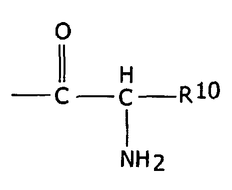 où R¹⁰ représente un groupe latéral d'un acide α-aminé naturel, ou
représentent un radical de la formule : 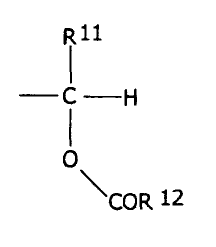 où R¹¹ représente un radical alcoyle (C₁-C₄), et R¹² représente l'atome d'hydrogène, un radical alcoyle (C₁-C₄) ou un radical de la formule : 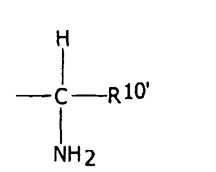 où R^{10'} représente un groupe latéral d'un acide α-aminé naturel, ou
R² et R³ forment avec l'atome d'azote, un hétérocycle saturé de 5 à 6 membres, qui peut présenter le cas échéant, encore un atome d'oxygène,
R⁴ représente l'atome d'hydrogène, un radical acyle (C₁-C₆), alcényle (C₂-C₆), cycloalcoyle (C₃-C₈), ou
R⁴ représente un radical alcoyle (C₁-C₆), qui peut être le cas échéant, substitué par 1 à 3 substituants, qui sont choisis parmi le groupe qui consiste en un atome d'halogène, le radical hydroxyle, un radical acyle (C₁-C₆), alcoxy (C₂-C₆), le radical - (OCH₂CH₂)ₙOCH₂CH₃, où n est 0 ou 1, le radical phénoxy, un radical aryle (C₆-C₁₀) et -NR¹³R¹⁴,
où R¹³ et R¹⁴ sont identiques ou différents et représentent l'atome d'hydrogène, un radical acyle (C₁-C₆), alcoyle (C₁-C₆), carbamoyle, mono- ou di(alcoyl (C₁-C₆))aminoalcoyle (C₁-C₆), mono- ou di(alcoyl (C₁-C₆))aminocarbonyle, aryle (C₆-C₁₀) ou (alcoxy (C₁-C₆))-carbonyle, ou
R¹³ et R¹⁴ forment avec l'atome d'azote, un hétérocycle saturé de 5 à 6 membres, qui peut contenir le cas échéant, un autre hétéroatome de la série S ou O ou un reste de la formule -NR¹⁵, et qui peut être substitué par oxo,
où
R¹⁵ représente l'atome d'hydrogène ou un radical alcoyle (C₁-C₄), ou
R⁴ représente un radical alcoyle (C₁-C₆), qui est substitué par un hétérocycle aromatique de 5 à 6 membres, le cas échéant benzocondensé, avec jusqu'à 3 hétéroatomes de la série S, N et/ou O, où un hétérocycle azoté peut également être lié par l'atome d'azote, ou est substitué par un reste des formules : 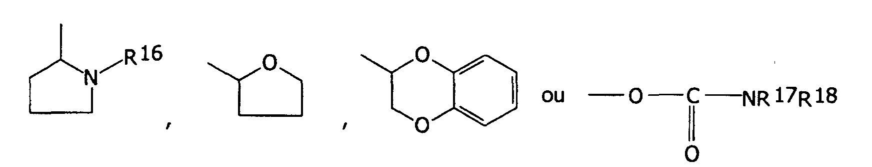 où
R¹⁶ représente l'atome d'hydrogène ou un radical alcoyle (C₁-C₆),
R¹⁷ et R¹⁸ sont identiques ou différents et représentent l'atome d'hydrogène, un radical alcoyle (C₁-C₆) ou aryle (C₆-C₁₀), où les radicaux alcoyle (C₁-C₆) et aryle (C₆-C₁₀) peuvent être le cas échéant substitués par 1 à 3 substituants, qui sont choisis parmi le groupe qui consiste en le radical hydroxyle, un radical alcoxy (C₁-C₆) et un atome d'halogène,
R⁵ représente l'atome d'hydrogène, un radical alcoyle (C₁-C₆), un atome d'halogène, un radical amino, mono- ou di(alcoyl (C₁-C₆))amino ou un radical (alcanoyl (C₁-C₆))amino,
R⁶ représente le radical phényle, qui peut être le cas échéant substitué par 1 à 3 substituants, qui sont choisis parmi le groupe qui consiste en
- un atome d'halogène,
- un radical aryle (C₆-C₁₀), qui peut être le cas échéant substitué par 1 à 3 substituants, choisis parmi un radical alcanoyle (C₁-C₆), alcoxy (C₁-C₆), alcoyle (C₁-C₆), un atome d'halogène, un radical (alcoxy (C₁-C₆))carbonyle, le radical nitro, un radical halogénoalcoyle (C₁-C₆), un radical halogénoalcoxy(C₁-C₆), amino, alcoylthio (C₁-C₆), hydroxyle, carboxyle, carbamoyle, mono- ou di(alcoyl (C₁-C₆))aminocarbonyle, mono- ou di(alcanoyl (C₁-C₆))amino, (alcoxy (C₁-C₆))carbonylamino, alcoylsulfoxy (C₁-C₆), alcoylsulfonyle (C₁-C₆) , tri(alcoyl (C₁-C₆))silyloxy, un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, et/ou le radical cyano,
- un radical alcoxy (C₁-C₆),
- un radical (alcoxy (C₁-C₆))carbonyle,
- un radical alcoylthio (C₁-C₆),
- le radical hydroxyle,
- le radical carboxyle,
- un radical alcoxy (C₁-C₆) partiellement fluoré, avec jusqu'à 6 atomes de fluor,
- un radical alcoyle (C₁-C₆), qui est le cas échéant substitué par un reste de la formule 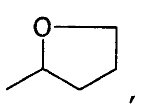
- un hétérocycle aromatique de 5 à 6 membres, le cas échéant lié par un atome d'azote, avec jusqu'à 3 hétéroatomes de la série S, N et/ou O, qui peut être le cas échéant substitué par 1 à 3 substituants, choisis parmi un radical alcanoyle (C₁-C₆), alcoxy (C₁-C₆), alcoyle (C₁-C₆), un atome d'halogène, un radical (alcoxy (C₁-C₆))carbonyle, le radical nitro, un radical halogénoalcoyle (C₁-C₆), un radical halogénoalcoxy(C₁-C₆), amino, alcoylthio (C₁-C₆), hydroxyle, carboxyle, carbamoyle, mono- ou di(alcoyl (C₁-C₆))aminocarbonyle, mono- ou di (alcanoyl (C₁-C₆))amino, (alcoxy (C₁-C₆))carbonylamino, alcoylsulfoxy (C₁-C₆), alcoylsulfonyle (C₁-C₆), un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, et/ou le radical cyano,
- un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, qui peut être le cas échéant substitué par 1 à 3 substituants, choisis parmi le radical oxo, un atome d'halogène, le radical hydroxyle, un radical (alcoxy (C₁-C₆))carbonyle, (alcoxy (C₁-C₆))carbonylamino, alcoyle (C₁-C₆), halogénoalcoyle (C₁-C₆) et hydroxyalcoyle (C₁-C₆),
et les groupes des formules
- -OR¹⁹,
- -NR²⁰R²¹ ou -CO-NR²²R²³,
où R¹⁹ représente le radical phényle, qui est pour sa part, substitué le cas échéant par un radical de la formule -NR²⁴R²⁵,
où R²⁴ et R²⁵ sont identiques ou différents et représentent l'atome d'hydrogène, un radical alcoyle (C₁-C₆) ou acyle (C₁-C₆),
ou R¹⁹ représente un radical alcoyle (C₁-C₆), qui peut être substitué le cas échéant, une à trois fois, par le radical hydroxyle et/ou un atome d'halogène,
R²⁰ et R²¹ sont identiques ou différents et représentent l'atome d'hydrogène, le radical carbamoyle, un radical mono- ou di(alcoyle (C₁-C₆)) aminocarbonyle, phényle, acyle (C₁-C₆) ou alcoyle (C₁-C₆),
où le radical alcoyle (C₁-C₆) indiqué précédemment est le cas échéant substitué par un radical alcoxy (C₁-C₆), acyle (C₁-C₆), par le radical phényle ou par un hétérocycle aromatique, de 5 à 6 membres, avec jusqu'à 3 hétéroatomes de la série S, N et/ou O,
où le radical phényle indiqué précédemment et l'hétérocycle aromatique indiqué précédemment sont le cas échéant substitués une à trois fois, de manière identique ou différente, par un atome d'halogène et/ou le radical hydroxyle,
R²² et R²³ sont identiques ou différents et représentent l'atome d'hydrogène ou un radical alcoyle (C₁-C₆),
et R⁷ peut présenter la signification de R⁵ et peut être identique ou différent de celui-ci,
et leurs sels.

3. Composés de la formule générale (I) selon la revendication 1 ou 2, où R¹ représente l'atome d'hydrogène ou un radical alcoyle (C₁-C₆).

4. Composés de la formule générale (I) selon la revendication 1, 2 ou 3, où R² et R³ représentent chaque fois indépendamment, l'atome d'hydrogène ou un radical alcoyle (C₁-C₆).

5. Composés de la formule générale (I) selon la revendication 1, 2, 3 ou 4, où R⁴ représente l'atome d'hydrogène ou un radical alcoyle (C₁-C₆).

6. Composés de la formule générale (I) selon la revendication 1, 2, 3, 4 ou 5, où R⁵ représente l'atome d'hydrogène.

7. Composés de la formule générale (I) selon la revendication 1, 2, 3, 4, 5 ou 6, où R⁵ représente le radical phényle, qui peut être le cas échéant substitué par 1 à 3 substituants, qui sont choisis parmi le groupe qui consiste en
- un atome d'halogène,
- un radical aryle (C₆-C₁₀), qui peut être le cas échéant substitué par 1 à 3 substituants, choisis parmi un radical alcanoyle (C₁-C₆), alcoxy (C₁-C₆), alcoyle (C₁-C₆), un atome d' halogène, un radical (alcoxy (C₁-C₆))carbonyle, le radical nitro, un radical halogénoalcoyle (C₁-C₆), un radical halogénoalcoxy(C₁-C₆), amino, hydroxyle, mono- ou di(alcanoyl (C₁-C₆))amino, (alcoxy (C₁-C₆))carbonylamino, et/ou le radical cyano,
- un hétérocycle aromatique de 5 à 6 membres, le cas échéant lié par un atome d'azote, avec jusqu'à 3 hétéroatomes de la série S, N et/ou O.

8. Composés selon la revendication 1, qui présentent la formule suivante : 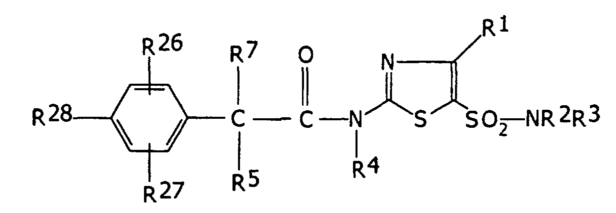 où R¹, R², R³, R⁴, R⁵ et R⁷ sont définis comme à la revendication 1,
R²⁶ et R²⁷ sont identiques ou différents et représentent l'atome d'hydrogène, un atome d'halogène, un radical alcoxy (C₁-C₆), (alcoxy (C₁-C₆))carbonyle, alcoylthio (C₁-C₆), le radical hydroxyle, carboxyle, un radical alcoxy (C₁-C₆) partiellement fluoré, avec jusqu'à 6 atomes de fluor, alcoyle (C₁-C₆), un radical des formules -OR¹⁹, -NR²⁰R²¹ ou -CO-NR²²R²³, où
R¹⁹ représente le radical phényle, qui est pour sa part, substitué le cas échéant par un radical de la formule -NR²⁴R²⁵,
où R²⁴ et R²⁵ sont identiques ou différents et représentent l'atome d'hydrogène, un radical alcoyle (C₁-C₆) ou acyle (C₁-C₆),
ou R¹⁹ représente un radical alcoyle (C₁-C₆), qui peut être substitué le cas échéant, une à trois fois, par le radical hydroxyle et/ou un atome d'halogène,
R²⁰ et R²¹ sont identiques ou différents et représentent l'atome d'hydrogène, le radical carbamoyle, un radical mono- ou di(alcoyle (C₁-C₆))aminocarbonyle, phényle, acyle (C₁-C₆) ou alcoyle (C₁-C₆),
où le radical alcoyle (C₁-C₆) indiqué précédemment est le cas échéant substitué par un radical alcoxy (C₁-C₆), acyle (C₁-C₆), par le radical phényle ou par un hétérocycle aromatique, de 5 à 6 membres, avec jusqu'à 3 hétéroatomes de la série S, N et/ou O,
où le radical phényle indiqué précédemment et l'hétérocycle aromatique indiqué précédemment sont le cas échéant substitués une à trois fois, de manière identique ou différente, par un atome d'halogène et/ou le radical hydroxyle,
R²² et R²³ sont identiques ou différents et représentent l'atome d'hydrogène ou un radical alcoyle (C₁-C₆),
R²⁸ représente un radical aryle (C₆-C₁₀), qui peut être le cas échéant substitué par 1 à 3 substituants, choisis parmi un radical alcanoyle (C₁-C₆), alcoxy (C₁-C₆), alcoyle (C₁-C₆), un atome d'halogène, un radical (alcoxy (C₁-C₆))carbonyle, le radical nitro, un radical halogénoalcoyle (C₁-C₆), un radical halogénoalcoxy(C₁-C₆), amino, alcoylthio (C₁-C₆), hydroxyle, carboxyle, carbamoyle, mono- ou di(alcanoyl (C₁-C₆))aminocarbonyle, mono- ou di (alcanoyl (C₁-C₆))amino, (alcoxy (C₁-C₆)) carbonylamino, alcoylsulfoxy (C₁-C₆), alcoylsulfonyle (C₁-C₆), tri (alcoyl (C₁-C₆))silyloxy, un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, et/ou le radical cyano, ou
R²⁸ représente un hétérocycle aromatique de 5 à 6 membres, le cas échéant lié par un atome d'azote, avec jusqu'à 3 hétéroatomes de la série S, N et/ou O, qui peut être le cas échéant substitué par 1 à 3 substituants, choisis parmi un radical alcanoyle (C₁-C₆), alcoxy (C₁-C₆), alcoyle (C₁-C₆), un atome d'halogène, un radical (alcoxy (C₁-C₆))carbonyle, le radical nitro, un radical halogénoalcoyle (C₁-C₆), un radical halogénoalcoxy(C₁-C₆), amino, alcoylthio (C₁-C₆), hydroxyle, carboxyle, carbamoyle, mono- ou di(alcoyl (C₁-C₆))aminocarbonyle, mono- ou di(alcanoyl (C₁-C₆))amino, (alcoxy (C₁-C₆))carbonylamino, alcoylsulfoxy (C₁-C₆), alcoylsulfonyle (C₁-C₆), tri(alcoyl (C₁-C₆))silyloxy, un hétérocycle non aromatique, saturé ou insaturé de 3 à 8 membres, mono- ou bicyclique, le cas échéant lié par un atome d'azote, avec jusqu'à 3 hétéroatomes de la série S, N et/ou O, et/ou le radical cyano,
et leurs sels.

9. Composé selon la revendication 1, de la formule : 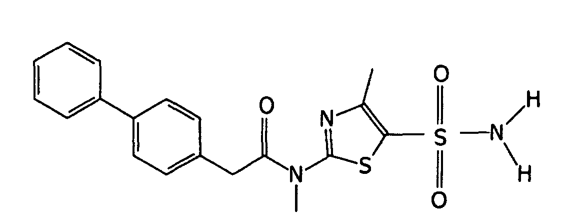 ainsi que ses sels pharmaceutiquement compatibles.

10. Composé selon la revendication 1, de la formule : 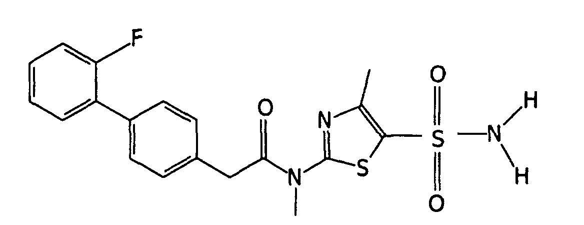 ainsi que ses sels pharmaceutiquement compatibles.

11. Composé selon la revendication 1, de la formule : 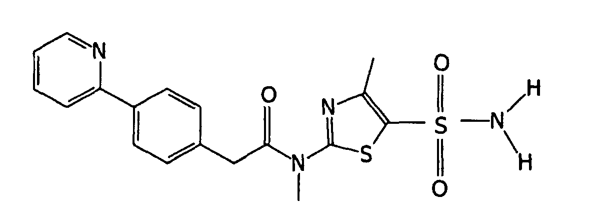 ainsi que ses sels pharmaceutiquement compatibles.

12. Composé selon la revendication 1, de la formule :

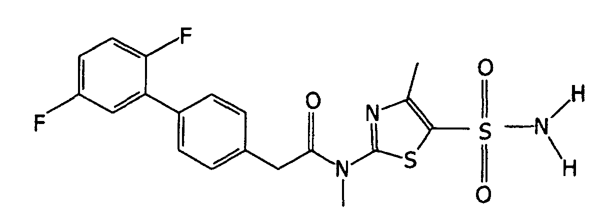

13. Composé selon la revendication 1, de la formule :

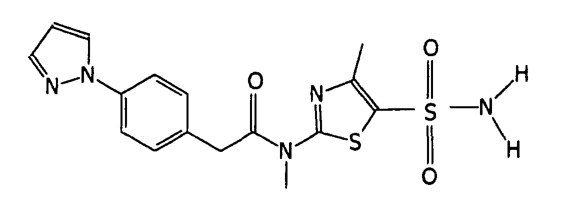

14. Composés de la formule générale (IV) : 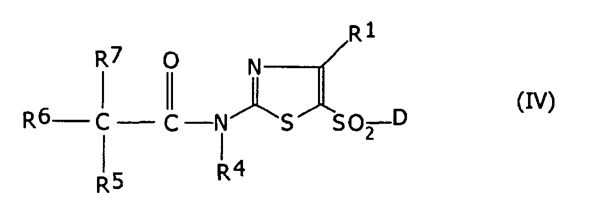 où R¹, R⁴, R⁵ et R⁷ ont la signification donnée à la revendication 1, et DE est un atome d'halogène.

15. Procédé de préparation des composés de la formule générale (I) selon la revendication 1, **caractérisé en ce que**
[A] on fait réagir les composés de la formule générale (II) : 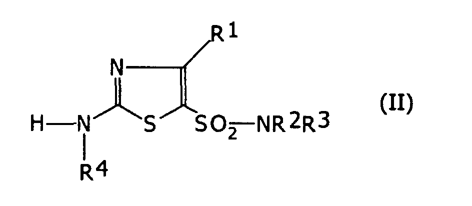 où R¹, R², R³ et R⁴ ont la signification donnée à la revendication 1,
avec des composés de la formule générale (III) : 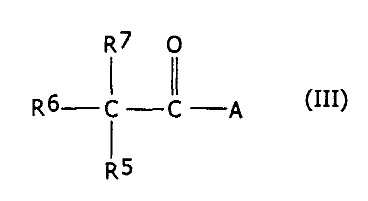 dans laquelle
A représente un groupe partant, et
R⁵, R⁶, et R⁷ ont la signification donnée à la revendication 1,
dans un solvant inerte, le cas échéant en présence d'une base et/ou d'un auxilialire,
ou
[B] on fait réagir les composés de la formule générale (IV) : 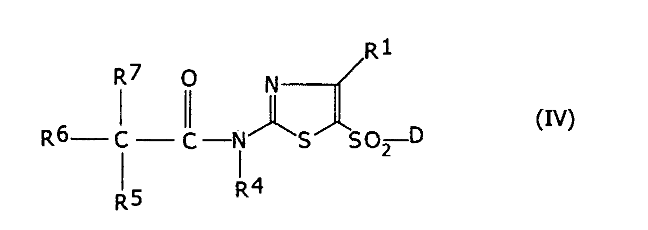 où R¹, R⁴, R⁵, R⁶ et R⁷ ont la signification donnée à la revendication 1, et
D est un atome d'halogène, de préférence l'atome de chlore
avec des amines de la formule générale (V) :
HNR²R³ (V)
où R² et R³ ont la signification donnée à la revendication 1,
dans un solvant inerte,
[C] on fait réagir les composés de la formule générale (X) : 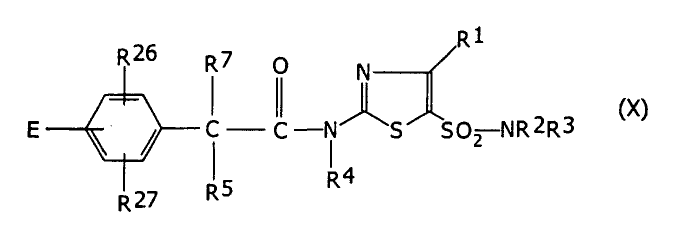 où R¹, R², R³, R⁴, R⁵, R⁷, R²⁶ et R²⁷ ont la signification donnée ci-dessus, et E est le radical trifluorométhanesulfonate ou un atome d'halogène,
avec l'acide boronique ou des stanannes de la formule générale (XI) :
R²⁸M (XI)
où R²⁸ a la signification indiquée ci-dessus et M peut être par exemple, un radical tri (alcoyl (C₁-C₆))stannyle ou un radical acide boronique,
dans un solvant inerte en présence de catalyseurs au palladium, le cas échéant en présence d'une base, à des températures allant de 50 à 140°C, en les composés de la formule (XIV) : 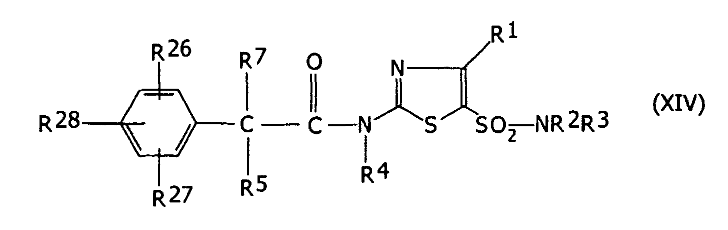 et
[D] on fait réagir les composés de la formule générale (XII) : 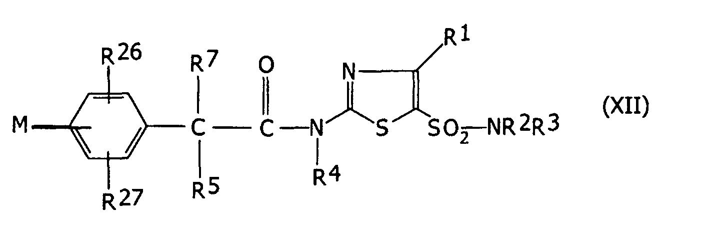
où R¹, R², R³, R⁴, R⁵, R⁷, R²⁶ et R²⁷ ont la signification donnée ci-dessus, et M possède la signification donnée ci-dessus, avec des trifluorométhanesulfonates ou des halogénures de la formule générale (XIII) :
R²⁸E (XIII)
où R²⁸ a la signification indiquée ci-dessus et E possède la signification donnée ci-dessus dans un solvant inerte en présence de catalyseurs au palladium, le cas échéant en présence d'une base, à des températures allant de 50 à 140°C, en les composés de la formule (XIV).

16. Composés selon la revendication 1, à utiliser comme agent pharmaceutique.

17. Composition pharmaceutique, qui comprend un composé de la formule générale (I) selon la revendication 1 en mélange avec un support ou excipient pharmaceutiquement compatible.

18. Utilisation d'un composé de la formule générale (I) selon la revendication 1, pour la préparation d'un agent pharmaceutique.

19. Utilisation d'un composé de la formule générale (I) selon la revendication 1, pour la préparation d'un agent pharmaceutique pour le traitement d'infections virales.

20. Utilisation d'un composé de la formule générale (I) selon la revendication 1, pour la préparation d'un agent pharmaceutique pour le traitement d'infections virales par des virus de l'herpès.

21. Utilisation d'un composé de la formule générale (I) selon la revendication 1, pour la préparation d'un agent pharmaceutique pour le traitement d'infections virales par des virus Herpès Simplex.

22. Utilisation de dérivés N-[5-(aminosulfonyl)-1,3-thiazol-2-yl]acétamide selon la revendication 1, pour la préparation d'un agent pharmaceutique.

23. Utilisation de dérivés N-[5-(aminosulfonyl)-1,3-thiazol-2-yl]-2-phénylacétamide selon la revendication 1, pour la préparation d'un agent pharmaceutique.

24. Utilisation de dérivés N-[5-(aminosulfonyl)-1,3-thiazol-2-yl]-2-(1,1'-biphényl)-4-ylacétamide selon la revendication 1, pour la préparation d'un agent pharmaceutique.

25. Utilisation selon la revendication 22, 23 ou 24 pour la préparation d'agents pour le traitement et/ou la prévention d'infections virales chez l'homme ou les animaux.

26. Utilisation selon la revendication 22, 23 ou 24 pour la préparation d'agents pour le traitement et/ou la prévention d'infections virales chez l'homme ou les animaux par des virus de l'herpès.

27. Utilisation selon la revendication 22, 23 ou 24 pour la préparation d'agents pour le traitement et/ou la prévention d'infections virales chez l'homme ou les animaux par des virus Herpès Simplex.
